# EUROPEAN PATENT SPECIFICATION

(11) **EP 4 514 846 B1**
(45) Date of publication and mention of the grant of the patent: **16.09.2026**
(21) Application number: 23724937.0
(22) Date of filing: 25.04.2023
(51) Int. Cl.: C07K 16/24, C07K 16/46, A61P 17/00, A61P 37/00, A61K 39/395, A61K 39/00

(54) **MULTISPECIFIC ANTIBODIES TARGETING IL-13 AND IL-18**
GEGEN IL-13 UND IL-18 GERICHTETE MULTISPEZIFISCHE ANTIKÖRPER
ANTICORPS MULTISPÉCIFIQUES CIBLANT IL-13 ET IL-18

(30) Priority: 26.04.2022 US 202263334964 P
(43) Date of publication of application: 05.03.2025
(60) Divisional application: 26195593.4
(73) Proprietor: Novartis AG, 4056 Basel (CH)
(72) Inventor: BARDROFF, Michael Otto, 4002 Basel (CH); CEBE, Regis, 4002 Basel (CH); KOVARIK, Jiri, 4002 Basel (CH); KOLBINGER, Frank, 4002 Basel (CH); KIFFE, Michael, 4002 Basel (CH); RITTER, Anett, 4002 Basel (CH); ROTH, Lukas, 4002 Basel (CH)
(74) Representative: Papapetridis, Ioannis
(86) International application number: PCT/IB2023/054248
(87) International publication number: WO 2023/209568

(56) References cited:
- WO-A1-2018/229612
- WO-A2-2005/062967
- WO-A2-2012/088290
- WO-A2-2014/037899
- WO-A2-2015/127405
- SONJA HÄNZELMANN ET AL: "GSVA: gene set variation analysis for microarray and RNA-Seq data", BMC BIOINFORMATICS, BIOMED CENTRAL , LONDON, GB, vol. 14, no. 1, 16 January 2013 (2013-01-16), pages 7, XP021146329, ISSN: 1471-2105, DOI: 10.1186/1471-2105-14-7
- YASUDA KOUBUN ET AL: "Interleukin-18 in Health and Disease", INTERNATIONAL JOURNAL OF MOLECULAR SCIENCES, vol. 20, no. 3, 2 February 2019 (2019-02-02), pages 649, XP093042734, DOI: 10.3390/ijms20030649

## Description

### Field of the Invention

The present invention relates to the field of immunology. Specifically, the invention relates to bispecific antibodies targeting Interleukin 13 (IL-13) and Interleukin 18 (IL-18), methods of making and in vitro use of the antibodies, the antibodies for medical use, as well as pharmaceutical formulations or kits comprising them, isolated nucleic acids or vectors encoding them, and host cells comprising the nucleic acids or vectors.

### Background of the Invention

Atopic dermatitis (AD) is a chronic/relapsing inflammatory skin disease characterized by symptoms including intense pruritus (e.g., severe itch) and by scaly and dry eczematous lesions. Severe disease can be extremely disabling due to major psychological problems, significant sleep loss, and impaired quality of life, leading to high socioeconomic costs. The pathophysiology of AD is influenced by a complex interplay between Immunoglobulin E (IgE)-mediated sensitization, the immune system, and environmental factors. The primary skin defect may be an immunological disturbance that causes IgE-mediated sensitization, with epithelial-barrier dysfunction that is the consequence of both genetic mutations and local inflammation. AD often begins in childhood before age 5 and may persist into adulthood.

Typical treatments for AD include topical lotions and moisturizers, topical corticosteroid ointments, creams or injections. Most treatment options, however, offer only temporary, incomplete, symptom relief. Moreover, many patients with moderate-to-severe AD become resistant to treatment by topical corticosteroids or by calcineurin inhibitors. Thus, a need exists in the art for novel targeted therapies for the treatment and/or prevention of AD.

The pathogenesis of AD is multifactorial, and the immunomediated mechanisms are characterized by an inappropriate activation of type 2 T helper cells (Th2) and type 2 innate lymphoid cells (ILC2), with an increased expression of inflammatory cytokines, particularly interleukins IL-4 and IL-13 (Moyle et al. (2019) Exp Dermatol. 28(7):756-768; Roediger et al. (2013) Nat Immunol.14(6):564-573). IL-13 stands out as one of the main cytokines in the pathophysiology of AD (Tsoi et al. (2019) J Invest Dermatol. 139(7):1480-1489), through its prominent role in the production and maintenance of the inflammatory process as well as epidermal barrier dysfunction.

Currently, dupilumab which is an anti-IL4Ra antibody has been approved by the US Food and Drug Administration and by the European Medicines Agency for the treatment of moderate-to severe forms of AD. Antibodies specifically targeting IL-13 are also being developed, such as lebrikizumab and tralokinumab.

IL-18 is considered to be involved in pathogenesis of AD, because IL-18 induces the super Th1 cells producing and secreting IFN-γ and IL-13 (Terada et al. (2006) Proc Natl Acad Sci U S A. 103:8816-8821). IL-18 is released by keratinocytes and inflammatory dendritic cell, and the serum IL-18 levels in patients with AD has shown to be significantly correlated with skin scores of AD lesions (Ikezawa et al. (2010) Allergy, Asthma & Immunology Research 2(4): 235-246). *In vivo* administration of IL-18 also causes Th2 differentiation and increases IgE production in a CD4+ T cell-, IL-4- and STAT6-dependent fashion in mice (Yoshimoto et al. (2000) Nat Immunol 1: 132-137; Hoshino et al. (2000) Eur J Immunol 30: 1998-2006).

Since IL-13 and IL-18 are pro-inflammatory cytokines affecting a number of different cell types relevant in AD, there remains a need for an effective treatment achieving co-blockade of IL-13 and IL-18 signalling. Multispecific antibodies (e.g., bispecific antibodies) targeting both IL-13 and IL-18 may address the unmet medical need in this chronic inflammatory disease.

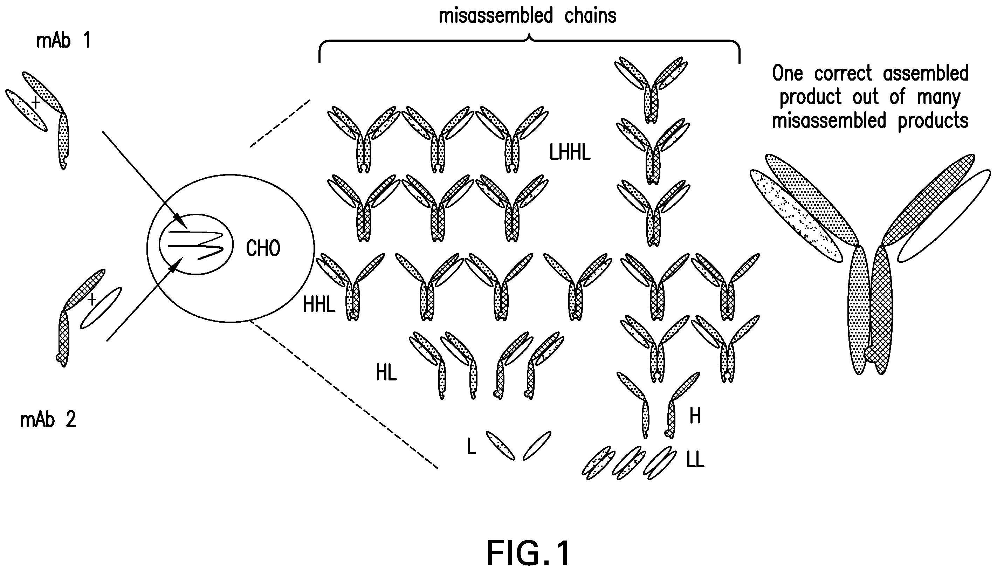

One of the most common problems in the production of bispecific IgG (BsIgG) by coexpressing two different antibodies is the unwanted homodimerization of the component heavy chains and the unwanted pairings of the component light chains with the incorrect heavy chain. Figure 1 shows possible misassembled products. To overcome this problem of heavy chain homodimerization, heavy chains can be remodeled for heterodimerization using engineered disulfide bonds in combination with previously identified "knobs-into-holes" mutations. One of the variants, S354C:T366W/Y349'C:T366'S:L368'A:Y407V, can provide near quantitative (-95%) heterodimerization (Merchant et al, 1998). However, this near quantitative heterodimerization does not solve the light chain pairing problem. And thus, assuming random light chain pairing, only 25% of the produced antibodies are the desired bispecific. The need still remains to further improve both heavy chain heterodimerization and light chain pairing, and to thereby improve the purity, yield, and quality of the bispecific antibodies.
WO2012/088290 A2 discloses non-IgG chimeric triple variable domain immunoglobulins. WO 2014/037899 A2 and WO 2005/062967 A2 describe, respectively, monospecific antibodies against IL-18 and IL-13. WO 2015/127405 A2 and WO 2018/229612 A1 describe, respectively, an anti-IL13/IL-17 and an anti-IL-18/IL-1β bispecific antibody.

### Summary of the Invention

The claimed invention is set out in the appended set of claims.

References to methods of treatment or of treating as such in the subsequent paragraphs of this description are to be interpreted as references to the bispecific antibodies, pharmaceutical compositions or medicaments of the present invention for use in those methods.

Without wishing to be bound by theory, the present inventors hypothesize that co-blockade of IL13 and IL18 provides unexpectedly superior efficacy in the treatment of autoimmune or inflammatory disorders as compared to blockade of IL-13 or IL-18 alone. The provided co-blockade comprises administering an antagonist that inhibits both IL-13 and IL-18, that is a bispecific antibody that binds both IL-18 and IL-13.

The present invention provides bispecific antibodies targeting both IL-13 and IL-18 for the treatment of AD, as defined in the claims, ensuring a sufficient overall yield, purity and product quality to proceed with clinical development and commercial manufacturing, at a reasonable cost.

The antibody is a bispecific IgG antibody, comprising a) a first part comprising a first light chain variable domain (VL1) and a first heavy chain variable domain (VH1), that binds specifically to Interleukin-18 (IL-18), wherein the VL1 domain comprises SEQ ID NO: 13, and wherein the VH1 domain comprises SEQ ID NO: 41, and b) a second part comprising a second light chain variable domain (VL2) and a second heavy chain variable domain (VH2), that binds specifically to Interleukin-13 (IL-13), wherein the VL2 domain comprises SEQ ID NO: 27, and wherein the VH2 domain comprises SEQ ID NO: 55.

In some embodiments, the engineered bispecific antibody is a human or humanized (e.g., CDR-grafted) IgG1, IgG2, IgG3 or IgG4 antibody. In some cases, the engineered bispecific antibody is a human or humanized (e.g., CDR-grafted) IgG1 antibody.

Undesirable Fc interactions with Fcγ receptors and the complement receptor C1q can be decoupled from binding to the Neonatal Fc Receptor (FcRn) which can increase serum persistence. In vivo serum persistence conferred by FcRn is shown to be a tunable property that can be modulated by mutations in the IgG Fc. Increase the Fc affinity to FcRn in endosomal condition (acidic pH) by Fc engineering is an effective approach to prolong the pharmacokinetics of monoclonal antibodies (Maeda, 2017). YTE mutation set (M252Y, S254T, T256E according to EU numbering) or LS mutation set (M428L, N434S according to EU numbering) are examples of such developed mutation sets in Fc CH2 domains.

In one embodiment, the engineered bispecific antibody comprises, according to EU numbering, M252Y/S254T/T256E (YTE). In one embodiment, the engineered bispecific antibody comprises, according to EU numbering, M428L, N434S (LS).

Chain pairing mutations have been demonstrated to be efficient at driving heavy-chain heterodimerization by introducing complementarity at the CH3-CH3 interface of bispecific or multispecific antibodies. A number of chain pairing mutation sets are used in the production of multispecific antibodies: increasing/decreasing side-chain volume (T366W/S354C-T366S/L368A/Y407V/Y349C, knob-into-hole) (Ridgway, 1996), charge inversions (K409D/K392D-D399K/E356K, electrostatic steering) (Gunasekaran, 2010), or multiple IgA substitutions (SEEDbody) (Davis, 2010).

In one embodiment, the engineered bispecific antibody comprises, chain pairing amino acid substitutions, e.g., in combination with silencing and/or half-life extension mutations. In some cases, the chain pairing amino acid substitutions are knob-into-hole (KiH) mutations, e.g., wherein the engineered bispecific antibody comprises a first constant heavy chain with amino acid substitution of T366W and a second constant heavy chain with amino acid substitutions of T366S, L368A and Y407V, and the amino acid residues are numbered according to the EU numbering.

In another embodiment, the chain paring amino acid substitutions are knob-into-hole (KiH) mutations, wherein comprises a first constant heavy chain with amino acid substitutions of S354C and T366W and a second constant heavy chain with amino acid substitutions of Y349C, T366S, L368A and Y407V, and the amino acid residues are numbered according to the EU numbering.

In a further embodiment, the engineered bispecific antibody comprises both T366W/S354C-T366S/L368A/Y407V/Y349C (KiH) and M252Y/S254T/T256E (YTE), and the amino acid residues are numbered according to the EU numbering.

Without being bound by theory, in some embodiments the bispecific antibody comprises one or mutations to silence, e.g., ADCC and/or CDC effector function within hFc. Various mutation sets are described in the art like LALA (L234A, L235A according to EU numbering) (Wines et al, 2000) or DAPA (D265A, P329A according to EU numbering) (Genentech, US 6,737,056) for instance. Several investigators have employed a cross-subclass approach to reduce effector functions. In a further refinement of the cross-subclass approach, IgG2 variant was generated with point mutations from IgG4 (i.e., H268Q, V309L, A330S, P331S according to EU numbering) (An et al., 2009). Another silent IgG1 antibody comprises the N297A mutation, which results in aglycosylated/non-glycosylated antibodies (Strohl et al, 2009). Some used mutation sets combine previously described technologies, achieving higher levels of silencing up to completely abolishing some or all effector functions. DANAPA is one example (D265A, N297A, P329A) (WO2019068632 Janssen). Other alternate approaches to engineer or mutate critical residues in the Fc region that are responsible for effector functions have been reported. For examples see PCT publications WO 2009/100309 (Medimmune), WO 2006/076594 (Xencor), US 2006/0134709 (Macrogenics), US 6,737,056 (Genentech), US 2010/0166740 (Roche).

In one embodiment, the engineered bispecific antibody comprises D265A/P329A (DAPA), and wherein the amino acid residues are numbered according to the EU numbering.

In one embodiment, the engineered bispecific antibody comprises L234A/L235A(LALA), and wherein the amino acid residues are numbered according to the EU numbering.

In another embodiment, the engineered bispecific antibody comprises one or more cysteine substitutions selected from the group consisting of positions: 234, 235, 236, 297 and 299, and wherein the amino acid residues are numbered according to the EU numbering.

In a further embodiment, the one or more cysteine substitutions of the engineered bispecific antibody are selected from positions 234, 235 and 236. In one embodiment, the engineered bispecific antibody comprises a cysteine substitution at position 234. In another embodiment, the engineered bispecific antibody comprises a cysteine substitution at position 235. In another embodiment, the engineered bispecific antibody comprises a cysteine substitution at position 236.

In some embodiments, the engineered bispecific antibody comprises one or more amino acid substitutions which reduce Fc effector functions, and one or more amino acid substitutions which enhance the half-life of the engineered bispecific antibody via enhanced FcRn binding and/or one or more amino acid substitutions that facilitate correct chain pairing.

Thus, for example, in some embodiments, the engineered bispecific antibody comprises, according to EU numbering, T366W/S354C-T366S/L368A/Y407V/Y349C (KiH), and half-life extension mutations selected from the group consisting of YTE (M252Y, S254T, T256E) and LS (M428L, N434S). In some embodiments, the engineered bispecific antibody comprises, according to EU numbering, T366W/S354C-T366S/L368A/Y407V/Y349C (KiH), and Fc silencing mutations selected from the group consisting of LALA (L234A, L235A), DAPA (D265A, P329A), and N297. In some embodiments, the engineered bispecific antibody comprises, according to EU numbering, T366W/S354C-T366S/L368A/Y407V/Y349C (KiH), half-life extension mutations selected from the group consisting of YTE (M252Y, S254T, T256E) and LS (M428L, N434S), and Fc silencing mutations selected from the group consisting of LALA (L234A, L235A), DAPA (D265A, P329A), and N297.

In one embodiment, the engineered bispecific antibody comprises L234A/L235A(LALA) and M252Y/S254T/T256E (YTE), and wherein the amino acid residues are numbered according to the EU numbering.

In one embodiment, the engineered bispecific antibody comprises L234C and M252Y/S254T/T256E (YTE), and wherein the amino acid residues are numbered according to the EU numbering. In one embodiment, the engineered bispecific antibody comprises L235C and M252Y/S254T/T256E (YTE), and wherein the amino acid residues are numbered according to the EU numbering. In another embodiment, the engineered bispecific antibody comprises G236C and M252Y/S254T/T256E (YTE), and wherein the amino acid residues are numbered according to the EU numbering.

In one embodiment, the engineered bispecific antibody thereof comprises L234A/L235A(LALA), M252Y/S254T/T256E (YTE) and T366W/S354C-T366S/L368A/Y407V/Y349C (KiH), and wherein the amino acid residues are numbered according to the EU numbering.

In one embodiment, the engineered bispecific antibody comprises L234C, M252Y/S254T/T256E (YTE) and T366W/S354C-T366S/L368A/Y407V/Y349C (KiH), and wherein the amino acid residues are numbered according to the EU numbering. In one embodiment, the engineered bispecific antibody comprises L235C, M252Y/S254T/T256E (YTE) and T366W/S354C-T366S/L368A/Y407V/Y349C (KiH), and wherein the amino acid residues are numbered according to the EU numbering. In another embodiment, the engineered bispecific antibody comprises G236C, M252Y/S254T/T256E (YTE) and T366W/S354C-T366S/L368A/Y407V/Y349C (KiH), and wherein the amino acid residues are numbered according to the EU numbering.

In some embodiments, the VH1 and VH2 domain of the bispecific antibody comprise Complementarity Determining Regions (CDR) HCDR1, HCDR2, HCDR3; and the VL1 and VL2 comprise LCDR1, LCDR2, LCDR3, wherein:
a. the VH1 domain comprises (e.g. in sequence):
   i. said HCDR1 having the amino acid sequence SEQ ID NO:32, said HCDR2 having the amino acid sequence SEQ ID NO:33, and said HCDR3 having the amino acid sequence SEQ ID NO:34; or
   ii. said HCDR1 having the amino acid sequence SEQ ID NO:35, said HCDR2 having the amino acid sequence SEQ ID NO:36, and said HCDR3 having the amino acid sequence SEQ ID NO:37; or
   iii. said HCDR1 having the amino acid sequence SEQ ID NO:38, said HCDR2 having the amino acid sequence SEQ ID NO:39, and said HCDR3 having the amino acid sequence SEQ ID NO:40; and
b. the VL1 domain comprises (e.g. in sequence):
   i. said LCDR1 having the amino acid sequence SEQ ID NO:4, said LCDR2 having the amino acid sequence SEQ ID NO:5, and said LCDR3 having the amino acid sequence SEQ ID NO:6;or
   ii. said LCDR1 having the amino acid sequence SEQ ID NO:7, said LCDR2 having the amino acid sequence SEQ ID NO:8, and said LCDR3 having the amino acid sequence SEQ ID NO:9;or
   iii. said LCDR1 having the amino acid sequence SEQ ID NO:10, said LCDR2 having the amino acid sequence SEQ ID NO:11, and said LCDR3 having the amino acid sequence SEQ ID NO:12; and
c. the VH2 domain comprises (e.g. in sequence):
   i. said HCDR1 having the amino acid sequence SEQ ID NO:46, said HCDR2 having the amino acid sequence SEQ ID NO:47, and said HCDR3 having the amino acid sequence SEQ ID NO:48; or
   ii. said HCDR1 having the amino acid sequence SEQ ID NO:49, said HCDR2 having the amino acid sequence SEQ ID NO:50, and said HCDR3 having the amino acid sequence SEQ ID NO:51; or
   iii. said HCDR1 having the amino acid sequence SEQ ID NO:52, said HCDR2 having the amino acid sequence SEQ ID NO:53, and said HCDR3 having the amino acid sequence SEQ ID NO:54; and
d. the VL2 domain comprises (e.g. in sequence):
   i. said LCDR1 having the amino acid sequence SEQ ID NO:18, said LCDR2 having the amino acid sequence SEQ ID NO:19, and said LCDR3 having the amino acid sequence SEQ ID NO:20; or
   ii. said LCDR1 having the amino acid sequence SEQ ID NO:21, said LCDR2 having the amino acid sequence SEQ ID NO:22, and said LCDR3 having the amino acid sequence SEQ ID NO:23; or
   iii. said LCDR1 having the amino acid sequence SEQ ID NO:24, said LCDR2 having the amino acid sequence SEQ ID NO:25, and said LCDR3 having the amino acid sequence SEQ ID NO:26.

In some embodiments, the first light chain is of lambda type, and the second light chain is of kappa type.

In some embodiments, the first light chain is of lambda 1 type, and the second light chain is of kappa 4 type.

The bispecific antibody of the invention comprises: a VH1 domain comprising the amino acid sequence SEQ ID NO:41, a VL1 domain comprising the amino acid sequence SEQ ID NO:13, a VH2 domain comprising the amino acid sequence SEQ ID NO:55, and a VL2 domain comprising the amino acid sequence SEQ ID NO:27.

In some embodiments, the bispecific antibody comprises a first light chain comprising an amino acid sequence as set forth in SEQ ID NO:14 and a second light chain comprising an amino acid sequence as set forth in SEQ ID NO:28.

In some embodiments, the bispecific antibody comprises a first heavy chain comprising a hetero-dimerization modification, and a second heavy chain comprising a hetero-dimerization modification which is complementary to the hetero-dimerization modification of the first heavy chain.

In some embodiments, the first and second constant heavy chains are human IgG1 comprising hetero-dimerization modification(s) and:
a) the hetero-dimerization modification of the first immunoglobulin heavy chain comprises a serine at position 366, an alanine at position 368, and a valine at position 407, and the hetero-dimerization modification of the second immunoglobulin heavy chain comprises a tryptophan at position 366; or
b) the hetero-dimerization modification of the second immunoglobulin heavy chain comprises a serine at position 366, an alanine at position 368, and a valine at position 407, and the hetero-dimerization modification of the first immunoglobulin heavy chain comprises a tryptophan at position 366,
and wherein the amino acid residues are numbered according to the EU numbering.

In some embodiments, the bispecific antibody comprises mutations which enhance the half-life of the bispecific antibody via enhanced FcRn binding.

In some embodiments, the mutations which enhance the half-life of the bispecific antibody are M252Y/S254T/T256E (YTE), and wherein the amino acid residues are numbered according to the EU numbering.

In some embodiments, the first heavy chain comprises an amino acid sequence as set forth in SEQ ID NO:42, and the second heavy chain comprises an amino acid sequence as set forth in SEQ ID NO:56.

In some embodiments, the first heavy chain comprises an amino acid sequence as set forth in SEQ ID NO:57, and the second heavy chain comprises an amino acid sequence as set forth in SEQ ID NO:58.

In some embodiments, the first heavy chain comprises an amino acid sequence as set forth in SEQ ID NO:42, and the first light chain comprises an amino acid sequence as set forth in SEQ ID NO:14, and the second heavy chain comprises an amino acid sequence as set forth in SEQ ID NO:56, and the second light chain comprises an amino acid sequence as set forth in SEQ ID NO:28.

In some embodiments, the first heavy chain comprises an amino acid sequence as set forth in SEQ ID NO:57, and the first light chain comprises an amino acid sequence as set forth in SEQ ID NO:14, and the second heavy chain comprises an amino acid sequence as set forth in SEQ ID NO:58, and the second light chain comprises an amino acid sequence as set forth in SEQ ID NO:28.

Also provided herein is a pharmaceutical composition comprising the bispecific antibody of the present disclosure, in combination with one or more pharmaceutically acceptable excipients, diluents or carriers.

In some embodiments, the pharmaceutical composition further comprises one or more additional active agents.

Also provided herein is an isolated nucleic acid molecule encoding the bispecific antibody of the present disclosure.

Also provided herein is a cloning or expression vector comprising one or more nucleic acid sequences as outlined above, wherein the vector is suitable for the recombinant production of the bispecific antibody of the present disclosure. In some embodiments, provided herein is a set of two cloning or expression vectors the first vector encoding a full-length heavy chain comprising constant domains, and a variable domain as defined in the claims; and a full-length light chain comprising a constant, and a variable domain as defined in the claims, wherein the heavy and light chains encoded by the first vector are capable of combining to form an anti-IL-18 arm of a bispecific IgG antibody, the second vector encoding a full-length heavy chain comprising constant domains and a variable domain, and a full-length light chain comprising a constant and a variable domain, wherein the heavy and light chains encoded by the second vector are capable of combining to form an anti-IL-13 arm of a bispecific IgG antibody as described herein. In some embodiments, the first and second vectors are expression vectors, and wherein co-expression of the first and second vectors in a common host cell provides an anti-IL-18/IL-13 bispecific IgG-like antibody with high yield, purity, and activity.

Also provided herein is a host cell comprising one or more cloning or expression vectors as outlined above.

Also provided herein is a process for the production of the bispecific antibody of the present disclosure, comprising culturing a host cell as outlined above under conditions sufficient to express the bispecific antibody, and thereafter purifying and recovering the bispecific antibody from the host cell culture.

Also provided herein is a kit comprising one or more cloning and/or expression vectors of the present disclosure, wherein the kit additionally comprises instructions for production of a bispecific antibody provided herein.

Also provided herein is a kit comprising a bispecific antibody of the present disclosure or the pharmaceutical composition of the present disclosure, wherein the kit additionally comprises instructions for use and means for administering the bispecific antibody or the pharmaceutical composition to a subject in need thereof.

In some embodiments, the means for administering comprises a syringe, an autoinjector, an injection pen, a vial and syringe, an infusion pump, a patch, or an infusion bag and needle.

Also disclosed herein is an *in vitro* method of simultaneously binding IL-13 and IL-18, comprising contacting the IL-13 and IL-18 with an effective amount of a bispecific antibody of the present disclosure.

Also provided herein is a method of simultaneously inhibiting the activities of IL-13 and IL-18, comprising contacting a plurality of mammalian cells with an effective amount of a bispecific antibody of the present disclosure, wherein the contacting is performed in an *in vitro* or *ex vivo* culture. In some cases, the IL-13 and IL-18 activities are reduced by at least 10%, at least 25%, at least 50%, at least 75%, or at least 90%. In some cases, a reduction in IL-13 activity is measured by a reduction in STAT-6 signalling. In some cases, a reduction in IL-18 activity is measured by a reduction in IFNγ production, such as a reduction in LPS/IL-12 induced IFNγ production. In some cases, a reduction in IL-18 and/or IL-13 activity is measured by an increase in the level of IL-18 or IL-13 respectively that is bound to a bispecific antibody described herein or by a decrease in the level of free IL-18 or IL-13 respectively.

Also disclosed herein, for reference purposes, is a method of simultaneously inhibiting the activities of IL-13 and IL-18 in a subject, comprising administering to the subject a therapeutically effective amount of a bispecific antibody of the present disclosure. Without wishing to be bound by theory, the present inventors hypothesize that co-blockade of IL-13 and II-18 can have complementary (e.g., synergistic) effects as compared to blockade of IL-18, or as compared to blockade of IL-13. In some embodiments, that co-blockade of IL-13 and II-18 can have complementary (e.g., synergistic) effects as compared to blockade of IL-18.

Also disclosed herein, for reference purposes, is a method of treating an IL-13 and/or IL-18 mediated disorder in a subject, comprising administering to the subject a therapeutically effective amount of a bispecific antibody of the present disclosure. In some embodiments, the method is an improved treatment as compared to treatment with a mono-specific anti-IL-13 antagonist. In some embodiments, the method is an improved treatment as compared to treatment with a mono-specific anti-IL-18 antagonist. In some embodiments, the method is an improved treatment as compared to treatment with an anti-IL-13 or anti-IL-18 antagonist. In some embodiments, the improvement is indicated by a better Eczema Area and Severity Index (EASI) score, a better Investigator Global Assessment (IGA) score, a better Pruritus Numeric Rating Scale score, and/or a better Dermatology Life Quality Index score after 16, 24, 36, or 52 weeks of treatment. In some embodiments, the method is an improved treatment as compared to treatment with an anti-IL-13 or anti-IL-18 antagonist, wherein the improvement is indicated by a lower Severity Scoring of Atopic Dermatitis (SCORAD) score after 16, 24, 36, or 52 weeks of treatment.

In some embodiments, the methods of present disclosure decrease the expression level of one or more AD-associated biomarker, in particular one or more AD-associated biomarker selected from the list consisting of CCL17/TARC, IgE (e.g., serum IgE), CCL26/eotaxin-3, CCL22/MDC, hsCRP, CD40, IL-13, IL-24, IL-22, IL-18 (e.g., serum IL-18, serum free IL-18 (bioactive)), and IL-18BP (e.g., serum IL-18BP), as compared to the level before co-blockade of IL13 and IL18 (e.g., treatment with the bispecific antibody.

Also disclosed herein, for reference purposes, is a method of treating and/or preventing an inflammatory or immune condition comprising simultaneously or sequentially inhibiting IL13 and IL18 (e.g., by administering to a subject in need thereof a therapeutically effective amount of a bispecific antibody of the present disclosure). In a preferred embodiment, the inflammatory or immune condition is a skin condition. In a preferred embodiment, the skin condition is atopic dermatitis. In some embodiments, the atopic dermatitis is moderate to severe atopic dermatitis. In some cases, the atopic dermatitis is moderate to severe atopic dermatitis. In some cases, the atopic dermatitis is moderate to severe as determined by Rajka/Langeland criteria score and wherein the Rajka/Langeland criteria score is determined to be between 4.5 and 9. In some embodiments, the method further comprises administration of one or more topical corticosteroids. In some embodiments, the atopic dermatitis is inadequately controlled by administration of the one or more topical corticosteroids.

In a further aspect, provided herein is a bispecific antibody of the present disclosure, for use as a medicament, especially for use in the treatment and/or prevention of atopic dermatitis (AD).

In a further aspect, disclosed herein are uses of a bispecific antibody of the present disclosure, for the manufacture of a medicament for treatment and/or prevention of AD.

Additional details and embodiments are provided in the following sections.

### Brief Description of the Figures

**Figure 1** illustrates the possible products when expressing two KiH modified mAbs in the same host cell line. LHHL: light-heavy-heavy-light chain, and this fraction includes the final bispecific. HHL: heavy-heavy-light chain. HL: heavy-light chain = half mAb. H: heavy chain. L light chain. LL: light chain hetero- and homodimer.
**Figure 2** is a schematic representation of plasmids A-D for expressing IL-13 and IL-18. Plasmids A and C are encoding for expression of anti-IL13 kappa LC and anti-L13 knob HC; plasmid B and D are encoding for expression of anti-IL18 lambda LC and anti-IL18 hole HC.
**Figure 3** is a schematic representation of Furin-2A peptide (F2A) plasmids E and F. F2A technology enables combined expression of more than one protein chain from one promoter. On plasmids E and F, the first expression cassette is encoding for anti-IL18 lambda LC and anti-IL18 hole HC and the second expression cassette is encoding for anti-IL13 kappa LC and anti-L13 knob HC.
**Figure 4** is a schematic representation of adapted plasmid G and H. Plasmid G is encoding for expression of anti-IL13 kappa LC and anti-L13 knob HC; plasmid H is encoding for expression of anti-IL18 lambda LC and anti-IL18 hole HC.
**Figure 5** is a schematic representation of a Furin-2A peptide (F2A) plasmid I with a different combination of protein chains in the expression cassettes compared to plasmids E and F. On plasmid I the first expression cassette is encoding for anti-IL18 lambda LC, anti-IL18 hole HC and anti-IL13 knob HC and the second expression cassette is encoding for anti-IL13 kappa LC.
**Figure 6** illustrates the melting curves of the IL-13/IL-18 bispecific antibodies. **Figure 6A** is the melting curve of bbmAb1. **Figure 6B** is the melting curve of bbmAb2. **Figure 6C** is the melting curve of bbmAb5. **Figure 6D** is the melting curve of bbmAb4. **Figure 6E** is the melting curve of bbmAb3.
**Figure 7** shows the comparison of pharmacokinetic profiles in Tg276 mice following administration of bbmAb1 and bbmAb2 as well as the Fc-silenced variants bbmAb6, bbmAb7, bbmAb8 and bbmAb9 (pooled serum samples were plotted per sampling time).

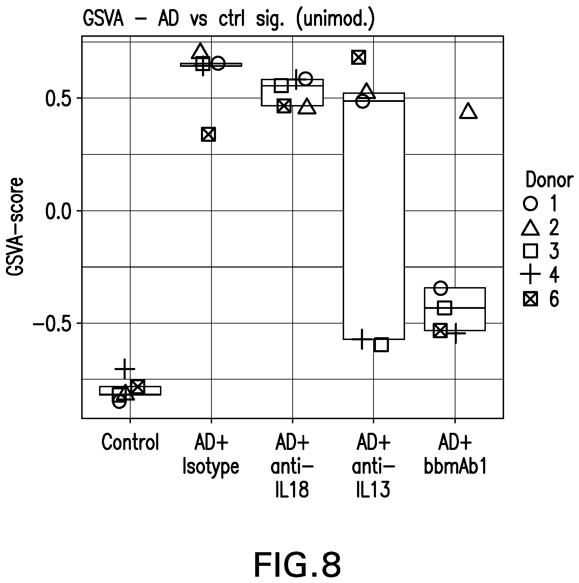

**Figure 8** illustrates results of a gene set variation analysis (GSVA) on differentially expressed genes between control samples (skin biopsies) that are not treated with a cocktail of cytokines that induces an atopic dermatitis (AD)-like transcriptome (Control), samples that are treated with the cocktail of cytokines that induces an atopic dermatitis (AD)-like transcriptome (induced samples) and co-incubated with an isotype control antibody (AD+isotype). Induced samples co-incubated with an anti-IL-18 antibody (AD+anti-IL18); induced samples co-incubated with an anti-IL-13 antibody (AD+anti-IL13); and induced samples co-incubated with a bispecific antibody that simultaneously inhibits the activities of IL-13 and IL-18 (AD+bbmAb1) show various degrees of inhibition. Data are shown for cells from five different donor samples.
**Figure 9** illustrates results of a t-distributed stochastic neighbour embedding (t-SNE) analysis (with perplexity 5) based on the in vitro disease transcriptome (AD+isotype versus control) of the 507 upregulated genes as described in Example 8.
**Figure 10** illustrates results of t-SNE analysis (with perplexity 5) based on the in vitro disease transcriptome (AD+isotype versus control) of the 1485 differentially expressed genes as described in Example 8.

### Detailed Description

The technical disclosure set out below may in some respects go beyond the scope of the claims. Elements of the disclosure which do not fall within the scope of the claims are provided for information.

In order that the present disclosure may be more readily understood, certain terms are specifically defined throughout the detailed description. Unless defined otherwise, all technical and scientific terms used herein have the same meaning as commonly understood by those of ordinary skill in the art to which this disclosure pertains.

### Definitions

Interleukin (IL)-18 (referred to simply as "IL-18" herein), is primarily produced by macrophages and T cells as a precursor protein (pro-IL-18) and secreted as an active protein following cleavage by caspase-1 (Dinarello CA et al (1999) J Allergy Clin Immunol; 103:11-24). In normal physiology IL-18, in synergy with IL-12, is associated with induction of cell-mediated immunity following infection with microbial products such as lipopolysaccharide (LPS) (Sareneva T et al (2000) J Immunol; 165(4):1933-8). After stimulation with IL-18, natural killer (NK) cells and T cells release the cytokine interferon gamma (INF-γ) which plays an important role in activating macrophages and other cells. IL-18 has also various functions in addition to an ability to induce interferon gamma. These biological properties include activation of NF-κB, Fas ligand expression, the induction of both CC and CXC chemokines, and increased production of competent human immuno-deficiency virus.

The term "IL-18" refers to IL-18 polypeptide, also known as Interleukin-18 polypeptide, IFN-gamma-inducing factor, Interferon-gamma-inducing-factor, or INF-γ inducing factor. Throughout this specification, the term IL-18 encompasses both pro-IL-18 (precursor of mature IL-18 prior protease cleavage) and mature IL-18 (post protease cleavage) interchangeably unless it is specified that the pro- or mature form is meant.

Interleukin (IL)-13 (referred to simply as "IL-13" herein), is a pleiotropic cytokine predominantly produced by Th2 cells and ILC2 but also, to a lesser extent, by mast cells, basophils, eosinophils, natural killer cells, macrophages, dendritic cells and monocytes. Free IL-13 binds to the a1 subunit of the IL-13 receptor (IL-13Ra1) in all cells of the human body, but particularly to monocytes and B cells. In a cascade reaction, this binding favours the recruitment of IL-4Ra, inducing, by dimerization, the formation of a signal transducer that activates Janus kinase 1 (JAK1) and tyrosine kinase 2 (TYK2), leading to the phosphorylation of signal transducer and activator of transcription 6 (STAT6), a transcription factor that promotes TH2 differentiation, and to class-switching to IgE (Silverberg et al (2017) Dermatol Clin. 35(3):327-334; Goenka et al (2011) Immunol Res. 50(1):87-96). The term "IL-13" is synonym to IL-13 polypeptide or Interleukin-13 polypeptide.

In all cases where the term "comprise", "comprises", "comprising" or the like are used in reference to a sequence (e.g., an amino acid sequence), it shall be understood that said sequence may also be limited by the term "consist", "consists", "consisting" or the like. As used herein, the phrase "consisting essentially of" refers to the genera or species of active pharmaceutical agents included in a method or composition, as well as any excipients inactive for the intended purpose of the methods or compositions. In some aspects, the phrase "consisting essentially of" expressly excludes the inclusion of one or more additional active agents other than a bispecific antibody of the present disclosure. In some aspects, the phrase "consisting essentially of" expressly excludes the inclusion of one or more additional active agents other than a bispecific antibody of the present disclosure and a second co-administered agent.

The term "antibody" as used herein refers to a polypeptide (or set of polypeptides) of the immunoglobulin family that is capable of binding an antigen non-covalently, reversibly, and specifically. For example, a naturally occurring "antibody" of the IgG type is a tetramer comprising at least two heavy (H) chains and two light (L) chains inter-connected by disulfide bonds. Each heavy chain is comprised of a heavy chain variable domain (abbreviated herein as VH) and a heavy chain constant domain. The heavy chain constant domain is comprised of three domains, CH1, CH2 and CH3. Each light chain is comprised of a light chain variable domain (abbreviated herein as VL) and a light chain constant domain (abbreviated herein as CL). The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen, which is sometimes referred to herein as the antigen binding domain. The constant regions of the antibodies may mediate the binding of the immunoglobulin to host tissues or factors, including various cells of the immune system (e.g., effector cells) and the first component (Clq) of the classical complement system.

The term "antibody" includes, but is not limited to, monoclonal antibodies, human antibodies, humanized antibodies, camelised antibodies, chimeric antibodies, bispecific antibodies and anti-idiotypic (anti-Id) antibodies. The antibodies can be of any subclass (e.g., IgG₁, IgG₂, IgG₃, IgG₄).

Both the light and heavy chains are divided into regions of structural and functional homology. The terms "constant" and "variable" are used functionally. In this regard, it will be appreciated that the variable domains of both the light (VL) and heavy (VH) chain portions determine antigen recognition and specificity. Conversely, the constant domains of the light chain (CL) and the heavy chain (CH1, CH2 or CH3) confer important biological properties such as secretion, transplacental mobility, Fc receptor binding, complement binding, and the like. By convention the numbering of the constant region domains increases as they become more distal from the antigen binding site or amino-terminus of the antibody. The N-terminus of the molecule contains the variable region and the C-terminus contains the constant region; the CH3 and CL domains comprise the carboxy-terminus of the heavy and light chain, respectively.

The phrase "antibody fragment" as used herein, for reference purposes, refers to one or more portions of an antibody. These portions can be part of the constant domain(s) of an antibody, e.g., fragment crystallizable (Fc), constant (C) domains, etc. In other cases, these portion(s) can be antigen-binding fragments that retain the ability of binding an antigen non-covalently, reversibly and specifically, sometimes referred to herein as the antigen binding domain. The phrase "antigen-binding fragment," as used herein, refers to one or more portions of an antibody that retain the ability to specifically interact with (e.g., by binding, steric hindrance, stabilizing/destabilizing, spatial distribution) an epitope of an antigen. Examples of binding fragments include, but are not limited to, single-chain Fvs (scFv) (with or without an internal cysteine bridge), disulfide-linked Fvs (sdFv), F(ab) ₂ fragment, Fab fragment, F(ab')₂, fragment F(ab') fragments, a monovalent fragment consisting of the VL, VH, CL and CH1 domains; a bivalent fragment comprising two Fab fragments linked by a disulfide bridge at the hinge region; an Fv fragment consisting of the VL and VH domains of a single arm of an antibody; a dAb fragment (Ward et al., (1989) Nature, 341:544-546), which consists of a V_{H} domain; and an isolated complementarity determining region (CDR), or other epitope-binding fragments of an antibody.

As used herein, an "Fc" or "Fc region" comprises a CH2 and a CH3, and, optionally, any portion of an antibody hinge region. An Fc region is comprised of two polypeptide chains that dimerize to form an Fc region. Each half antibody of the disclosure contains one Fc polypeptide chain. This half antibody may be paired with another half antibody, such that the two Fc polypeptide chains dimerize into an Fc region of a bispecific antibody of the disclosure. As with all polypeptide chains, an Fc polypeptide chain contains an N-terminus and a C-terminus, each of which is capable of being linked to an antigen-binding domain (e.g., an IL-18 binding domain or an IL-13 binding domain).

Antibody fragments, for reference purposes, can also be incorporated into single domain antibodies, maxibodies, minibodies, intrabodies, diabodies, triabodies, tetrabodies, v-NAR and bis-scFv (see, *e.g*., Hollinger and Hudson, (2005) Nature Biotechnology 23: 1126-1136).

Antibody fragments, for reference purposes, can be incorporated into single chain molecules comprising a pair of tandem Fv segments (for example, VH-CH1- VH -CH1) which, together with complementary light chain polypeptides (for example, VL-CL-VL-CL), form a pair of antigen binding regions (Zapata et al., (1995) Protein Eng., 8: 1057-1062; and US 5,641,870).

The term "half antibody" refers to a portion of an antibody molecule, or bispecific binding molecule that comprises a single antigen binding domain. In an embodiment, a half antibody refers to a heavy and light chain pair of, for example, an IgG antibody. In one embodiment, a half antibody refers to a polypeptide comprising a VL domain and a CL domain, and a second polypeptide comprising a VH domain, a CH1 domain, a hinge domain, a CH2 domain, and a CH3 domain (i.e., an Fd and an Fc), wherein said VL and VH domains comprise an antigen-binding domain. In some bispecific binding molecule embodiments, a first half antibody will associate, e.g., heterodimerize, with a second half antibody. In some bispecific binding molecules a first half antibody will be covalently linked to a second half antibody.

The term "monospecific molecule," as used herein, for reference purposes, refers to an Fc containing molecule that binds to one epitope on a target antigen. In some embodiments, a mono-specific molecule of the present disclosure can be a monospecific antibody-like molecule. A monospecific molecule of the present disclosure can be a monospecific antibody. The term "bispecific molecule" refers to a multispecific Fc containing binding molecule that binds to two different antigens as defined in the claims. The term "trispecific molecule", for reference purposes, refers to an Fc containing multispecific binding molecule that binds to three different antigens via three different binding moieties.

The term "multispecific antibody", for reference purposes, refers to antibody capable of recognizing two or more epitopes of an antigen or two or more antigens. Recognition of each antigen is generally accomplished via an "antigen-binding domain". In particular, bispecific antibodies recognize two different epitopes either on the same or on different antigens. All bispecific IgG molecules, i.e., bispecific antibodies indistinguishable in their composition from natural immunoglobulins, are bivalent and possess an asymmetric architecture due to the presence of, at least, different Fv regions. Depending on the method of preparation and origin of heavy and light chains, they may furthermore differ in the constant regions of the heavy or light chain (Brinkmann and Kontermann, 2017).

The bispecific antibodies are "heterodimers", which means that one part comes from first antibody, specific for a first target, and another part comes from a second antibody, specific for a second target. A "heterodimerization modification" is a modification to one or both parts of the antibodies forming the heterodimeric bispecific antibody, intended to facilitate such formation. An example of heterodimerization modifications of the Fc domains of two IgG1 parts of antibodies intended to form a bispecific is a "knob" with a bulky amino acid (aa) side chain (S354C, T366W) in the first heavy chain and a "hole" with small aa side chains (Y349C, T366S, L368A, Y407V) were introduced in the second heavy chain as well as an additional disulfide bridge in the CH3 region connecting both heavy chains (Merchant et al., Nat. Biotechnol., 16:677-681 (1998), page 678, table 1).

The term "mismatched" or "mispaired" or "misassembled" means that different parts of an intended protein complex, such as a bispecific antibody, do not complex bind as intended, which means that the protein complex does not look or behave as intended. Examples of mismatching in the context of a bispecific antibody are shown in Figure 1.

The terms "recognize" or "bind" as used herein refers to a binding molecule, an antibody or antigen-binding fragment thereof that finds and interacts (e.g., binds or recognizes) its epitope, whether that epitope is linear, discontinuous or conformational. The term "epitope" refers to a site on an antigen to which an antibody or antigen-binding fragment of the disclosure specifically binds. Epitopes can be formed both from contiguous amino acids or noncontiguous amino acids juxtaposed by tertiary folding of a protein. Epitopes formed from contiguous amino acids are typically retained on exposure to denaturing solvents, whereas epitopes formed by tertiary folding are typically lost on treatment with denaturing solvents. An epitope typically includes at least 3, 4, 5, 6, 7, 8, 9, 10, 11, 12, 13, 14 or 15 amino acids in a unique spatial conformation. Methods of determining spatial conformation of epitopes include techniques in the art, for example, x-ray crystallography and 2-dimensional nuclear magnetic resonance (see, e.g., Epitope Mapping Protocols in Methods in Molecular Biology, Vol. 66, G. E. Morris, Ed. (1996)), or electron microscopy. A "paratope" is the part of the antibody which recognizes the epitope of the antigen.

The phrase "specifically binds" or "selectively binds," when used in the context of describing the interaction between an antigen (e.g., a protein) and an antibody, antibody fragment, or antibody-derived binding agent, refers to a binding reaction that is determinative of the presence of the antigen in a heterogeneous population of proteins and other biologics, e.g., in a biological sample, e.g., a blood, serum, plasma or tissue sample. Thus, under certain designated immunoassay conditions, the antibodies or binding agents with a particular binding specificity bind to a particular antigen at least two times the background and do not substantially bind in a significant amount to other antigens present in the sample. In one aspect, under designated immunoassay conditions, the antibody or binding agent with a particular binding specificity binds to a particular antigen at least ten (10) times the background and does not substantially bind in a significant amount to other antigens present in the sample. Specific binding to an antibody or binding agent under such conditions may require the antibody or agent to have been selected for its specificity for a particular protein. As desired or appropriate, this selection may be achieved by subtracting out antibodies that cross-react with molecules from other species (e.g., mouse or rat) or other subtypes. Alternatively, in some aspects, antibodies or antibody fragments are selected that cross-react with certain desired molecules.

The term "antigen-binding site" refers to the part of an antibody that comprises determinants that form an interface that binds to the antigen, or an epitope thereof. The term "antigen binding site" may be used interchangeably with the term "antigen binding domain" or antigen binding moiety. With respect to proteins (or protein mimetics), the antigen-binding site typically includes one or more loops (of at least four amino acids or amino acid mimics) that form an interface that binds to the antigen polypeptide. Typically, the antigen-binding site of an antibody molecule includes at least one or two CDRs and/or hypervariable loops, or more typically at least three, four, five or six CDRs and/or hypervariable loops.

The terms "complementarity determining region" or "CDR," as used herein, refer to the sequences of amino acids within antibody variable regions which confer antigen specificity and binding affinity. For example, in general, there are three CDRs in each heavy chain variable region (e.g., HCDR1, HCDR2, and HCDR3) and three CDRs in each light chain variable region (LCDR1, LCDR2, and LCDR3). The positions of the CDRs and framework regions can be determined using various known definitions in the art, e.g., Kabat, Chothia, IMGT, AbM, and combined definitions (see, e.g., Kabat et al., (1991) "Sequences of Proteins of Immunological Interest," 5th Ed. Public Health Service, National Institutes of Health, Bethesda, MD; Johnson et al., (2001) Nucleic Acids Res., 29: 205-206; Chothia & Lesk, (1987) J. Mol. Biol., 196: 901-917; Chothia et al., (1989) Nature, 342: 877-883; Chothia et al., (1992) J. Mol. Biol., 227: 799-817; Lefranc MP (2001) Nucleic Acids Res., 29: 207-209; Al-Lazikani et al., (1997) J.Mol.Biol., 273:927-748). Definitions of antigen combining sites are also described in the following: Ruiz et al., (2000) Nucleic Acids Res., 28:219-221; MacCallum et al., (1996) J. Mol. Biol., 262:732-745; and Martin et al., (1989) PNAS. USA, 86:9268-9272; Martin et al., (1991) Methods Enzymol., 203:121-153; and Rees et al., In Sternberg M.J.E. (ed.), Protein Structure Prediction, Oxford University Press, Oxford, 141-172 (1996). Under the Kabat numbering scheme, in some embodiments, the CDR amino acid residues in the heavy chain variable domain (VH ) are numbered 31-35 (HCDR1), 50-65 (HCDR2), and 95-102 (HCDR3); and the CDR amino acid residues in the light chain variable domain (VL) are numbered 24-34 (LCDR1), 50-56 (LCDR2), and 89-97 (LCDR3). Under the Chothia numbering scheme, in some embodiments, the CDR amino acids in the VH are numbered 26-32 (HCDR1), 52-56 (HCDR2), and 95-102 (HCDR3); and the CDR amino acid residues in the VL are numbered 26-32 (LCDR1), 50-52 (LCDR2), and 91-96 (LCDR3). In a combined Kabat and Chothia numbering scheme, in some embodiments, the CDRs correspond to the amino acid residues that are part of a Kabat CDR, a Chothia CDR, or both. For instance, in some embodiments, the CDRs correspond to amino acid residues 26-35 (HCDR1), 50-65 (HCDR2), and 95-102 (HCDR3) in a human VH, e.g., a mammalian VH , e.g., a human VH; and amino acid residues 24-34 (LCDR1), 50-56 (LCDR2), and 89-97 (LCDR3) in human VL, e.g., a mammalian VL , e.g., a human VL. Under IMGT the CDR amino acid residues in the VH are numbered approximately 26-35 (CDR1), 51-57 (CDR2) and 93-102 (CDR3), and the CDR amino acid residues in the VL are numbered approximately 27-32 (CDR1), 50-52 (CDR2), and 89-97 (CDR3) (numbering according to "Kabat"). Under IMGT, the CDR regions of an antibody can be determined using the program IMGT/DomainGap Align.

The term "humanized" forms of non-human (e.g., murine) antibodies relates to chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin lo sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma & Immunol. 1: 105-115 (1998); Harris, Biochem. Soc. Transactions 23:1035-1038 (1995); Hurle and Gross, Curr. Op. Biotech. 5:428-433 (1994).

The term "human antibody" as used herein includes antibodies having variable regions in which both the framework and CDR regions are derived from sequences of human origin. Furthermore, if the antibody contains a constant region, the constant region also is derived from such human sequences, e.g., human germline sequences, or mutated versions of human germline sequences or antibody containing consensus framework sequences derived from human framework sequences analysis, for example, as described in Knappik, et al. (2000. J Mol Biol 296, 57-86).

The human antibodies of the invention may include amino acid residues not encoded by human sequences (e.g., mutations introduced by random or site-specific mutagenesis in vitro or by somatic mutation in vivo, or a conservative substitution to promote stability or manufacturing). However, the term "human antibody", as used herein, is not intended to include antibodies in which CDR sequences derived from the germline of another mammalian species, such as a mouse, have been grafted onto human framework sequences.

A "modification" or "mutation" of an amino acid residue/position, as used herein, refers to a change of a primary amino acid sequence as compared to a starting amino acid sequence, wherein the change results from a sequence alteration involving said amino acid residue/positions. For example, typical modifications include substitution of the residue (or at said position) with another amino acid (e.g., a conservative or non-conservative substitution), insertion of one or more amino acids adjacent to said residue/position, and deletion of said residue/position. An "amino acid substitution," or variation thereof, refers to the replacement of an existing amino acid residue in a predetermined (starting) amino acid sequence with a different amino acid residue. Generally and preferably, the modification results in alteration in at least one physicobiochemical activity of the variant polypeptide compared to a polypeptide comprising the starting (or "wild type") amino acid sequence. For example, in the case of an antibody, a physicobiochemical activity that is altered can be binding affinity, binding capability and/or binding effect upon a target molecule.

The term "conservatively modified variant" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein that encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid that encodes a polypeptide is implicit in each described sequence.

For polypeptide sequences, "conservatively modified variants" include individual substitutions, deletions or additions to a polypeptide sequence which result in the substitution of an amino acid with a chemically similar amino acid. Conservative substitution tables providing functionally similar amino acids are known in the art. Such conservatively modified variants are in addition to and do not exclude polymorphic variants, interspecies homologs, and alleles of the invention. The following eight groups contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton, Proteins (1984)). In some embodiments, the phrase "conservative sequence modifications" are used to refer to amino acid modifications that do not significantly affect or alter the binding characteristics of the antibody or the antibody-like molecule containing the amino acid sequence.

The terms "percent identical" or "percent identity," in the context of two or more nucleic acids or polypeptide sequences, refers to two or more sequences or subsequences that are the same. Two sequences are "substantially identical" if two sequences have a specified percentage of amino acid residues or nucleotides that are the same (i.e., 60% identity, optionally 65%, 70%, 75%, 80%, 85%, 90%, 95%, or 99% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity exists over a region that is at least about 50 nucleotides (or 10 amino acids) in length, or more preferably over a region that is 100 to 500 or 1000 or more nucleotides (or 20, 50, 200 or more amino acids) in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

The term "comparison window" as used herein includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are known in the art. Optimal alignment of sequences for comparison can be conducted, e.g., by the local homology algorithm of Smith and Waterman (1970) Adv. Appl. Math. 2:482c, by the homology alignment algorithm of Needleman and Wunsch (1970) J. Mol. Biol. 48:443, by the search for similarity method of Pearson and Lipman, (1988) Proc. Nat'l. Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, e.g., Brent et al., (2003) Current Protocols in Molecular Biology).

Two examples of algorithms that are suitable for determining percent sequence identity and sequence similarity are the BLAST and BLAST 2.0 algorithms, which are described in Altschul et al., (1977) Nuc. Acids Res. 25:3389-3402; and Altschul et al., (1990) J. Mol. Biol. 215:403-410, respectively. Software for performing BLAST analyses is publicly available through the National Center for Biotechnology Information. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al., supra). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a word length (W) of 11, an expectation (E) or 10, M=5, N=-4 and a comparison of both strands. For amino acid sequences, the BLASTP program uses as defaults a word length of 3, and expectation (E) of 10, and the BLOSUM62 scoring matrix (see Henikoff and Henikoff, (1989) Proc. Natl. Acad. Sci. USA 89:10915) alignments (B) of 50, expectation (E) of 10, M=5, N=-4, and a comparison of both strands.

The BLAST algorithm also performs a statistical analysis of the similarity between two sequences (see, e.g., Karlin and Altschul (1993) Proc. Natl. Acad. Sci. USA 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

The percent identity between two amino acid sequences can also be determined using the algorithm of E. Meyers and W. Miller (Comput. Appl. Biosci. 4:11-17 (1988)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol, Biol. 48:444-453 (1970)) algorithm which has been incorporated into the GAP program in the GCG software package (available at www.gcg.com), using either a Blossom 62 matrix or a PAM250 matrix, and a gap weight of 16, 14, 12, 10, 8, 6, or 4 and a length weight of 1, 2, 3, 4, 5, or 6.

Other than percentage of sequence identity noted above, another indication that two nucleic acid sequences or polypeptides are substantially identical is that the polypeptide encoded by the first nucleic acid is immunologically cross reactive with the antibodies raised against the polypeptide encoded by the second nucleic acid, as described below. Thus, a polypeptide is typically substantially identical to a second polypeptide, for example, where the two peptides differ only by conservative substitutions. Another indication that two nucleic acid sequences are substantially identical is that the two molecules or their complements hybridize to each other under stringent conditions, as described below. Yet another indication that two nucleic acid sequences are substantially identical is that the same primers can be used to amplify the sequence.

The term "nucleic acid" or "polynucleotide" refers to deoxyribonucleic acid (DNA) or ribonucleic acid (RNA) and polymers thereof in either single- or double-stranded form. Unless specifically limited, the term encompasses nucleic acids containing known analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g. degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994))

The nucleotide in the "polynucleotide" or "nucleic acid" may comprise modifications including base modifications such as bromouridine and inosine derivatives, ribose modification such as phosphorothioate, phosphorodithioate, phosphoroselenoate, phosphorodiselenoate, phosphoroanilothioate, phosphoraniladate and phosphoroamidate.

The term "vector" means any molecule or entity (e.g. nucleic acid, plasmid, bacteriophage or virus) that is suitable for transformation or transfection of a host cell and contains nucleic acid sequences that direct and/or control (in conjunction with the host cell) expression of one or more heterologous coding regions operatively linked thereto.

The term "operably linked" or functionally linked, as used herein, refers to a functional relationship between two or more polynucleotide (e.g., DNA) segments. Typically, it refers to the functional relationship of a transcriptional regulatory sequence to a transcribed sequence. For example, a promoter or enhancer sequence is operably linked to a coding sequence if it stimulates or modulates the transcription of the coding sequence in an appropriate host cell or other expression system. Generally, promoter transcriptional regulatory sequences that are operably linked to a transcribed sequence are physically contiguous to the transcribed sequence, i.e., they are cis-acting. However, some transcriptional regulatory sequences, such as enhancers, need not be physically contiguous or located in close proximity to the coding sequences whose transcription they enhance.

The term "co-expression" means that different polypeptides are expressed together in a single host cell, common for all the polypeptides. Co-expression of a bispecific antibody means that the different parts forming the functional bispecific antibody are expressed in a single, common, host cell. Co-expression may be achieved by incorporating several expression vectors in the expression host cell, such as one for each of the halves of a bispecific antibody, or by incorporating one expression vector encoding all parts of the bispecific antibody. As used herein, "C-terminus" refers to the carboxyl terminal amino acid of a polypeptide chain having a free carboxyl group (-COOH). As used herein, "N-terminus" refers to the amino terminal amino acid of a polypeptide chain having a free amine group (-NH2).

The terms "polypeptide" and "protein" are used interchangeably herein to refer to a polymer of amino acid residues. The phrases also apply to amino acid polymers in which one or more amino acid residue is an artificial chemical mimetic of a corresponding naturally occurring amino acid, as well as to naturally occurring amino acid polymers and non-naturally occurring amino acid polymer. Unless otherwise indicated, a particular polypeptide sequence also implicitly encompasses conservatively modified variants thereof.

The term "in vivo half life", as used herein, refers to the half-life of the molecule of interest or variants thereof circulating in the blood of a given mammal.

Human antibodies may be produced by a number of methods known to those of skill in the art. Human antibodies can be made by the hybridoma method using human myeloma or mouse-human heteromyeloma cells lines (Kozbor, J Immunol; (1984) 133:3001; Brodeur, Monoclonal Isolated Antibody Production Techniques and Applications, pp51-63, Marcel Dekker Inc, 1987). Alternative methods include the use of phage libraries or transgenic mice both of which utilize human variable region repertories (Winter G; (1994) Annu Rev Immunol 12:433-455, Green LL, (1999) J Immunol Methods 231:11-23).

Several strains of transgenic mice are now available wherein their mouse immunoglobulin loci has been replaced with human immunoglobulin gene segments (Tomizuka K, (2000) Proc Natl Acad Sci , 97:722-727; Fishwild DM (1996) Nature Biotechnol 14:845-851; Mendez MJ, (1997) Nature Genetics 15:146-156). Upon antigen challenge such mice are capable of producing a repertoire of human antibodies from which antibodies of interest can be selected. Of particular note is the TrimeraTM system (Eren R et al, (1988) Immunology 93:154-161) where human lymphocytes are transplanted into irradiated mice, the Selected Lymphocyte Isolated antibody System (SLAM, Babcook et al, Proc Natl Acad Sci (1996) 93:7843-7848) where human (or other species) lymphocytes are effectively put through a massive pooled in vitro isolated antibody generation procedure followed by deconvoluted, limiting dilution and selection procedure and the XenomouseTM (Abgenix Inc). An alternative approach is available from Morphotek Inc using the MorphodomaTM technology.

Phage display technology can be used to produce human antibodies and fragments thereof, (McCafferty; (1990) Nature, 348:552-553 and Griffiths AD et al (1994) EMBO 13:3245-3260). According to this technique, isolated antibody variable domain genes are cloned in frame into either a major or minor coat of protein gene of a filamentous bacteriophage such as M13 or fd and displayed (usually with the aid of a helper phage) as function isolated antibody fragments on the surface of the phage particle. Selections based on the function properties of the isolated antibody result in selection of the gene encoding the isolated antibody exhibiting these properties. The phage display technique can be used to select antigen specific antibodies from libraries made from human B cells taken from individuals afflicted with a disease or disorder or alternatively from unimmunized human donors (Marks; J Mol Bio (1991) 222:581-591,). Where an intact human isolated antibody is desired comprising an Fc domain it is necessary reclone the phage displayed derived fragment into a mammalian expression vectors comprising the desired constant regions and establishing stable expressing cell lines.

The technique of affinity maturation (Marks; Biotechnol (1992) 10:779-783) may be used to provide binding affinity wherein the affinity of the primary human isolated antibody is improved by sequentially replacing the H and L chain variable regions with naturally occurring variants and selecting on the basis of improved binding affinities. Variants of this technique such as 'epitope imprinting' are now also available (WO 93/06213; Waterhouse; Nucl Acids Res (1993) 21:2265-2266).

The term "pure" when used in the context of purified bispecific antibody relates to purity and identity of different bispecific antibody combinations and constructs after co-expression in selected cells under conditions wherein the cells express the bispecific antibody and after protein-A purification using an intact UPLC-MS mass screening approach. Pure or purity refers to the relative quantify of the formed hetero-and homodimer bbmAbs. Using the method of the invention correctly formed heterodimeric bispecific antibodies could be observed with a relative purity of over 85% based on intact mass signal intensity.

The term "therapeutically acceptable amount" or "therapeutically effective amount" or "therapeutically effective dose" interchangeably refer to an amount sufficient to effect the desired result (i.e., a reduction disease activity, reduction in disease progression, reduction in disease signs and/or symptoms, etc.). In some aspects, a therapeutically acceptable amount does not induce or cause undesirable side effects. A therapeutically acceptable amount can be determined by first administering a low dose, and then incrementally increasing that dose until the desired effect is achieved. A "prophylactically effective dosage," and a "therapeutically effective dosage," of the molecules of the present disclosure can prevent the onset of, or result in a decrease in severity of, respectively, disease symptoms, including symptoms associated with IL-13 activity and IL-18 activity.

The term "subject" includes human and non-human animals. Non-human animals include all vertebrates, e.g., mammals and non-mammals, such as non-human primates, sheep, dog, cow, chickens, amphibians, and reptiles. Except when noted, the terms "patient" or "subject" are used herein interchangeably.

As used herein, phrases such as "a patient in need of treatment" or "a subject in need of treatment" includes subjects, such as mammalian subjects, that would benefit from administration of molecule or pharmaceutical composition of the present disclosure used, e.g., for detection, for a diagnostic procedure and/or for treatment.

The term "treat", "treating", "treatment", "prevent", "preventing" or "prevention" includes therapeutic treatments, prophylactic treatments and applications in which one reduces the risk that a subject will develop a disorder or other risk factor. Treatment does not require the complete curing of a disorder and encompasses the reduction of the symptoms or underlying risk factors. As used herein, a human antibody or a fragment thereof comprises heavy or light chain variable regions or full length heavy or light chains that are "the product of" or "derived from" a particular germline sequence if the variable regions or full length chains of the antibody are obtained from a system that uses human germline immunoglobulin genes. Such systems include immunizing a transgenic mouse carrying human immunoglobulin genes with the antigen of interest or screening a human immunoglobulin gene library displayed on phage with the antigen of interest. A human antibody or fragment thereof that is "the product of" or "derived from" a human germline immunoglobulin sequence can be identified as such by comparing the amino acid sequence of the human antibody to the amino acid sequences of human germline immunoglobulins and selecting the human germline immunoglobulin sequence that is closest in sequence (i.e., greatest % identity) to the sequence of the human antibody. A human antibody that is "the product of" or "derived from" a particular human germline immunoglobulin sequence may contain amino acid differences as compared to the germline sequence, due to, for example, naturally occurring somatic mutations or intentional introduction of site-directed mutation. However, a selected human antibody typically is at least 90% identical in amino acids sequence to an amino acid sequence encoded by a human germline immunoglobulin gene and contains amino acid residues that identify the human antibody as being human when compared to the germline immunoglobulin amino acid sequences of other species (e.g. murine germline sequences). In certain cases, a human antibody may be at least 60%, 70%, 80%, 90%, or at least 95%, or even at least 96%, 97%, 98%, or 99% identical in amino acid sequence to the amino acid sequence encoded by the germline immunoglobulin gene. Typically, a human antibody derived from a particular human germline sequence will display no more than 10 amino acid differences from the amino acid sequence encoded by the human germline immunoglobulin gene. In certain cases, the human antibody may display no more than 5, or even no more than 4, 3, 2, or 1 amino acid difference from the amino acid sequence encoded by the germline immunoglobulin gene.

Various aspects of the invention are described in further detail in the following sections and subsections.

### Bispecific antibodies that bind IL-13 and IL-18

### I. IL-18 binding domains

The disclosure provides bispecific antibodies engineered to bind human IL-18. Recognition of IL-18 by the bispecific antibodies of the disclosure occurs via an "IL-18 antigen-binding domain" , which is referred to interchangeably as an "IL-18 binding domain."

In one preferred aspect, the bispecific antibody comprises one IL-18 binding domain, such that the bi-specific binding molecule is monovalent with respect to binding IL-18.

In some aspects, the IL-18 binding domain of the bispecific antibody comprises an IL-18 Fab.

In some embodiments, the bispecific antibody comprises an IL-18 binding domain having a binding affinity K_{D} of 10⁻⁴ M to 10⁻⁸ M, e.g., 10⁻⁵ M to 10⁻⁷ M, e.g., 10⁻⁶ M or 10⁻⁷ M, for IL-18.

In a preferred embodiment, the bispecific antibody comprises one IL-18 Fab.

In some instances, Fabs can be prepared according to methods known in the art. The enzyme papain can be used to cleave an immunoglobulin monomer into two Fab fragments and an Fc fragment. The enzyme pepsin cleaves below the hinge region, so a F(ab')2 fragment and a pFc' fragment is formed. The F(ab')2 fragment can be split into two Fab' fragments by mild reduction. Fab fragments are highly stable due to non-covalent interactions that occur over a large interface between heavy and light chain polypeptides and the presence of a stabilizing disulphide bond between the CH1 and CL domains (see Glover & Humphreys, Chapter 2, Antibodies, Vol 1: Production and Purification, Kluwer Academic/Plenum Publishers, New York 2004, edited by: G Subramanian). For examples of linker orientation and size see, e.g., Hollinger et al. (1993) PNAS U.S.A. 90: 6444-6448, US 2005/0100543, US 2005/0175606, US 2007/0014794, and WO2006/020258 and WO2007/024715.

The phrases "a Fab that binds human IL-18" and "IL-18 Fab" refer to a Fab that binds to human IL-18. In one aspect the IL-18 Fab retains equivalent binding affinity, e.g., binds IL-18 with comparable efficacy to full-length antibodies. In other embodiments, the IL-18 Fab has a lower binding affinity, e.g., it binds IL-18 with a lower binding affinity than full-length antibodies, but it nevertheless provides a biological response described herein.

The IL-18 Fab for use in the provided bispecific antibody comprises a VH comprising an amino acid sequence as set forth in SEQ ID NO:41 and a VL comprising an amino acid sequence as set forth in SEQ ID NO:13.

In one aspect, the present disclosure provides polynucleotides encoding a bispecific antibody comprising an IL-18 binding domain, e.g. IL-18 Fab. The present disclosure also provides isolated nucleic acid molecules that encode these Fabs.

Provided herein are isolated nucleic acids molecules that encode a bispecific antibody comprising an IL-18 binding domain comprising a VH having the amino acid sequence as set forth in SEQ ID NO:41, and comprising a VL having the amino acid sequence as set forth in SEQ ID NO:13.

In one aspect, the IL-18 binding domain (e.g., IL-18 Fab) of a bispecific antibody thereof is encoded by a transgene whose sequence has been codon optimized for expression in a mammalian cell. In one aspect, the entire construct of the bispecific antibody of the disclosure is encoded by a transgene whose entire sequence has been codon optimized for expression in a mammalian cell. Codon optimization refers to the discovery that the frequency of occurrence of synonymous codons (i.e., codons that code for the same amino acid) in coding DNA is biased in different species. Such codon degeneracy allows an identical polypeptide to be encoded by a variety of nucleotide sequences. A variety of codon optimization methods is known in the art, and include, e.g., methods disclosed in at least US 5,786,464 and US 6,114,148.

### II. IL-13 binding domains

The disclosure provides bispecific antibodies engineered to bind human IL-13. Recognition of IL-13 by the bispecific antibodies of the disclosure occurs via an "IL-13 antigen-binding domain" , which is referred to interchangeably as an "IL-13 binding domain."

In one preferred aspect, the bispecific antibody comprises one IL-13 binding domain, such that the bi-specific binding molecule is monovalent with respect to binding IL-13.

In some aspects, the IL-13 binding domain of the bispecific antibody comprises an IL-13 Fab.

In some embodiments, the bispecific antibody comprises an IL-13 binding domain having a binding affinity K_{D} of 10⁻⁴ M to 10⁻⁸ M, e.g., 10⁻⁵ M to 10⁻⁷ M, e.g., 10⁻⁶ M or 10⁻⁷ M, for IL-13.

In a preferred embodiment, the bispecific antibody comprises one IL-13 Fab.

The phrases "a Fab that binds human IL-13" and "IL-13 Fab" refer to a Fab that binds to human IL-13. In one aspect the IL-13 Fab retains equivalent binding affinity, e.g., binds IL-13 with comparable efficacy to full-length antibodies. In other embodiments, the Fab has a lower binding affinity, e.g., it binds IL-13 with a lower binding affinity than full-length antibodies, but it nevertheless provides a biological response described herein.

The IL-13 Fab for use in the provided bispecific antibody comprises a VH comprising an amino acid sequence as set forth in SEQ ID NO:55 and a VL comprising an amino acid sequence as set forth in SEQ ID NO:27.

Another IL-13 Fab disclosed herein, for reference purposes, comprises a VH comprising an amino acid sequence as set forth in SEQ ID NO:85 and a VL comprising an amino acid sequence as set forth in SEQ ID NO:71.

In one aspect, the present disclosure provides polynucleotides encoding a bispecific antibody comprising an IL-13 binding domain, e.g. IL-13 Fab. The present disclosure also provides isolated nucleic acid molecules that encode these Fabs.

Provided herein are isolated nucleic acids molecules that encode a bispecific antibody comprising an IL-13 binding domain comprising a VH having the amino acid sequence as set forth in SEQ ID NO:55, and comprising a VL having the amino acid sequence as set forth in SEQ ID NO:27.

Also disclosed herein, for reference purposes, are isolated nucleic acids molecules that encode a bispecific antibody comprising an IL-13 binding domain comprising a VH having the amino acid sequence as set forth in SEQ ID NO:85, and comprising a VL having the amino acid sequence as set forth in SEQ ID NO:71.

Also included in the disclosure are constructs (e.g., cloning or expression vectors) comprising one or more of the aforesaid isolated nucleic acid molecules and polynucleotides. Also included in the disclosure are host cells comprising one or more of the aforesaid constructs (e.g., cloning or expression vectors).

In one aspect, the present disclosure encompasses a recombinant nucleic acid construct comprising a polynucleotide encoding a bispecific antibody as disclosed herein, wherein the polynucleotide comprises a nucleic acid sequence encoding an IL-13 binding domain.

### III. Linkage and Orientation of Domains and Regions of the bispecific antibodies

As used herein, the term "linked" or "linking" refers to one portion of a bispecific antibody being attached, directly or indirectly, to another portion of the molecule. Direct attachment is a form of linkage, and is referred to herein as "fused" or "fusion". Using a molecule having the form A -B-C as an example: portion A is linked to directly to portion B and indirectly to portion C (portion A may also be described as being fused to portion B).

In some embodiments, the IL-18 binding domain and the IL-13 binding domain of the bispecific antibody are linked. In some embodiments, that linkage is a direct linkage and the regions are thus fused to each other. In some embodiments, an IL-18 binding domain and/or an IL-13 binding domain are fused to a Fc polypeptide chain.

### IV. Formats of the bispecific antibodies

An exemplary bispecific antibody is characterized by a first antigen binding domain (e.g., comprising a first VL and a first VH) which has binding specificity for IL-18 and a second antigen binding domain that has binding specificity for IL-13, as defined in the claims, respectively. In embodiments of the present disclosure, a bispecific antibody comprises a heavy chain variable domain sequence and a light chain variable domain sequence that have binding specificity for IL-18, and a further binding domain which has binding specificity for IL-13.

For reference purposes, protocols for generating bispecific or heterodimeric antibody are known in the art; including but not limited to, for example, the "knob in a hole" approach described in, e.g., US 5,731,168; the electrostatic steering Fc pairing as described in, e.g., WO 2009/089004, WO 2006/106905 and WO 2010/129304; Strand Exchange Engineered Domains (SEED) heterodimer formation as described in, e.g., WO 2007/110205; Fab arm exchange as described in, e.g., WO2008/119353, WO 2011/131746, and WO 2013/060867; double antibody conjugate, e.g., by antibody cross-linking to generate a bi-specific structure using a heterobifunctional reagent having an amine-reactive group and a sulfhydryl reactive group as described in, e.g., US 4,433,059; bispecific antibody or antibody-like molecule determinants generated by recombining half antibodies (heavy-light chain pairs or Fabs) from different antibodies or antibody-like molecules through cycle of reduction and oxidation of disulfide bonds between the two heavy chains, as described in, e.g., US 4,444,878; trifunctional antibodies, e.g., three Fab' fragments cross-linked through sulfhydryl reactive groups, as described in, e.g., US 5,273,743; biosynthetic binding proteins, e.g., pair of scFvs cross-linked through C-terminal tails preferably through disulfide or amine-reactive chemical cross-linking, as described in, e.g., US 5,534,254; bifunctional antibodies, e.g., Fab fragments with different binding specificities dimerized through leucine zippers that have replaced the constant domain, as described in, e.g., US 5,582,996; bispecific and oligospecific mono-and oligovalent receptors, e.g., VH-CH1 regions of two antibodies (two Fab fragments) linked through a polypeptide spacer between the CH1 region of one antibody and the VH region of the other antibody typically with associated light chains, as described in, e.g., US 5,591,828; bispecific DNA-antibody conjugates, e.g., crosslinking of antibodies or Fab fragments through a double stranded piece of DNA, as described in, e.g., US 5,635,602; bispecific fusion proteins, e.g., an expression construct containing two scFvs with a hydrophilic helical peptide linker between them and a full constant region, as described in, e.g., US 5,637,481; multivalent and multi-specific binding proteins, e.g., dimer of polypeptides having first domain with binding region of Ig heavy chain variable region, and second domain with binding region of Ig light chain variable region, generally termed diabodies (higher order structures are also encompassed creating for bispecific, trispecific, or tetraspecific molecules, as described in, e.g., US 5,837,242; minibody constructs with linked VL and VH chains further connected with peptide spacers to an antibody hinge region and CH3 region, which can be dimerized to form bispecific/multivalent molecules, as described in, e.g., US 5,837,821; VL and VH domains linked with a short peptide linker (e.g., 5 or 10 amino acids) or no linker at all in either orientation, which can form dimers to form bispecific diabodies; trimers and tetramers, as described in, e.g., US 5,844,094; string of VH domains (or VL domains in family members) connected by peptide linkages with crosslinkable groups at the C-terminus further associated with VL domains to form a series of Fvs (or scFvs), as described in, e.g., US 5,864,019; VL and VH domains, scFvs, or Fabs wherein one of the antigens is bound monovalently and one of the antigens is bound bivalently, optionally comprising heterodimeric Fc regions, as described in, e.g., WO2011/028952; and single chain binding polypeptides with both a VL and VH domain linked through a peptide linker are combined into multivalent structures through non-covalent or chemical crosslinking to form, e.g., homobivalent, heterobivalent, trivalent, and tetravalent structures using both scFv or diabody type format, as described in, e.g., US 5,869,620. Additional exemplary multispecific and bispecific molecules and methods of making the same are found, for example, in US 5,910,573, US 5,932,448, US 5,959,083, US 5,989,830, US 6,005,079, US 6,239,259, US 6,294,353, US 6,333,396, US 6,476,198, US 6,511,663, US 6,670,453, US 6,743,896, US 6,809,185, US 6,833,441, US 7,129,330, US 7,183,076, US 7,521,056, US 7,527,787, US 7,534,866, US 7,612,181, US 2002004587A1, US 2002076406A1, US 2002103345A1, US 2003207346A1, US 2003211078A1, US 2004219643A1, US 2004220388A1, US 2004242847A1, US 2005003403A1, US 2005004352A1, US 2005069552A1, US 2005079170A1, US 2005100543A1, US 2005136049A1, US 2005136051A1, US 2005163782A1, US 2005266425A1, US 2006083747A1, US 2006120960A1, US 2006204493A1, US 2006263367A1, US 2007004909A1, US 2007087381A1, US 2007128150A1, US 2007141049A1, US 2007154901A1, US 2007274985A1, US 2008050370A1, US 2008069820A1, US 2008152645A1, US 2008171855A1, US 2008241884A1, US 2008254512A1, US 2008260738A1, US 2009130106A1, US 2009148905A1, US 2009155275A1, US 2009162359A1, US 2009162360A1, US 2009175851A1, US 2009175867A1, US 2009232811A1, US 2009234105A1, US 2009263392A1, US 2009274649A1, EP 346087A2, WO 2000/06605A2, WO 2007/2635A2, WO 2004/081051A1, WO 2006/020258A2, WO 2007/044887A2, WO 2007/095338A2, WO 2007/137760A2, WO 2008/119353A1, WO 2009/021754A2, WO 2009/068630A1, WO 1991/03493A1, WO 1993/23537A1, WO 1994/09131A1, WO 1994/12625A2, WO 1995/09917A1, WO 1996/37621A2, WO 1999/64460A1.

### V. Exemplary anti-IL-13/IL-18 bispecific antibodies

The amino acid sequences in **Table 1** are examples of IL-13/IL-18 bispecific antibody sequences, as well as portions thereof.

**Table 1. Exemplary IL-13/IL-18 bispecific antibody Sequences.**

| bbmAb1 | | |
|---|---|---|
| SEQ ID NO:1(Combined) | LCDR1 | SGSSSNIGNHYVN |
| SEQ ID NO:2 (Combined) | LCDR2 | RNNHRPS |
| SEQ ID NO:3 (Combined) | LCDR3 | QSWDYSGFSTV |
| SEQ ID NO:4 (Kabat) | LCDR1 | SGSSSNIGNHYVN |
| SEQ ID NO:5 (Kabat) | LCDR2 | RNNHRPS |
| SEQ ID NO:6 (Kabat) | LCDR3 | QSWDYSGFSTV |
| SEQ ID NO:7 (Chothia) | LCDR1 | SSSNIGNHY |
| SEQ ID NO:8 (Chothia) | LCDR2 | RNN |
| SEQ ID NO:9 (Chothia) | LCDR3 | WDYSGFST |
| SEQ ID NO:10 (IMGT) | LCDR1 | SSNIGNHY |
| SEQ ID NO:11 (IMGT) | LCDR2 | RNN |
| SEQ ID NO:12 (IMGT) | LCDR3 | QSWDYSGFSTV |
| SEQ ID NO:13 | VL | DIVLTQPPSVSGAPGQRVTISCSGSSSNIGNHYVNWY QQLPGTAPKLLIYRNNHRPSGVPDRFSGSKSGTSASL AITGLQSEDEADYYCQSWDYSGFSTVFGGGTKLTVL |
| SEQ ID NO:14 | Light Chain mAb1 | DIVLTQPPSVSGAPGQRVTISCSGSSSNIGNHYVNWY QQLPGTAPKLLIYRNNHRPSGVPDRFSGSKSGTSASL AITGLQSEDEADYYCQSWDYSGFSTVFGGGTKLTVLG QPKAAPSVTLFPPSSEELQANKATLVCLISDFYPGAVT VAWKADSSPVKAGVETTTPSKQSNNKYAASSYLSLTP EQWKSHRSYSCQVTHEGSTVEKTVAPTECS |
| SEQ ID NO:15 (Combined) | LCDR1 | RASKSVDSYGNSFMH |
| SEQ ID NO:16 (Combined) | LCDR2 | LASNLES |
| SEQ ID NO:17 (Combined) | LCDR3 | QQNNEDPRT |
| SEQ ID NO:18 (Kabat) | LCDR1 | RASKSVDSYGNSFMH |
| SEQ ID NO:19 (Kabat) | LCDR2 | LASNLES |
| SEQ ID NO:20 (Kabat) | LCDR3 | QQNNEDPRT |
| SEQ ID NO:21 (Chothia) | LCDR1 | SKSVDSYGNSF |
| SEQ ID NO:22 (Chothia) | LCDR2 | LAS |
| SEQ ID NO:23 (Chothia) | LCDR3 | NNEDPR |
| SEQ ID NO:24 (IMGT) | LCDR1 | KSVDSYGNSF |
| SEQ ID NO:25 (IMGT) | LCDR2 | LAS |
| SEQ ID NO:26 (IMGT) | LCDR3 | QQNNEDPRT |
| SEQ ID NO:27 | VL | |
| SEQ ID NO:28 | Light Chain mAb2 | DIVMTQSPDSLSVSLGERATINCRASKSVDSYGNSFM HWYQQKPGQPPKLLIYLASNLESGVPDRFSGSGSGTD FTLTISSLQAEDVAVYYCQQNNEDPRTFGGGTKVEIKR TVAAPSVFIFPPSDEQLKSGTASVVCLLNNFYPREAKV QWKVDNALQSGNSQESVTEQDSKDSTYSLSSTLTLSK ADYEKHKVYACEVTHQGLSSPVTKSFNRGEC |
| SEQ ID NO:29 (Combined) | HCDR1 | GGTFKSYAIS |
| SEQ ID NO:30 (Combined) | HCDR2 | NIIPMTGQTYYAQKFQG |
| SEQ ID NO:31 (Combined) | HCDR3 | AAYHPLVFDN |
| SEQ ID NO:32 (Kabat) | HCDR1 | SYAIS |
| SEQ ID NO:33 (Kabat) | HCDR2 | NIIPMTGQTYYAQKFQG |
| SEQ ID NO:34 (Kabat) | HCDR3 | AAYHPLVFDN |
| SEQ ID NO:35 (Chothia) | HCDR1 | GGTFKSY |
| SEQ ID NO:36 (Chothia) | HCDR2 | IPMTGQ |
| SEQ ID NO:37 (Chothia) | HCDR3 | AAYHPLVFDN |
| SEQ ID NO:38 (IMGT) | HCDR1 | GGTFKSYA |
| SEQ ID NO:39 (IMGT) | HCDR2 | IIPMTGQT |
| SEQ ID NO:40 (IMGT) | HCDR3 | ARAAYHPLVFDN |
| SEQ ID NO:41 | VH | EVQLVQSGAEVKKPGSSVKVSCKASGGTFKSYAISWV RQAPGQGLEWMGNIIPMTGQTYYAQKFQGRVTITADE STSTAYMELSSLRSEDTAVYYCARAAYHPLVFDNWGQ GTLVTVSS |
| SEQ ID NO:42 | Heavy Chain mAb1 | EVQLVQSGAEVKKPGSSVKVSCKASGGTFKSYAISWV RQAPGQGLEWMGNIIPMTGQTYYAQKFQGRVTITADE STSTAYMELSSLRSEDTAVYYCARAAYHPLVFDNWGQ GTLVTVSSASTKGPSVFPLAPSSKSTSGGTAALGCLVK DYFPEPVTVSWNSGALTSGVHTFPAVLQSSGLYSLSS VVTVPSSSLGTQTYICNVNHKPSNTKVDKRVEPKSCD KTHTCPPCPAPELLGGPSVFLFPPKPKDTLYITREPEV TCVVVDVSHEDPEVKFNWYVDGVEVHNAKTKPREEQ YNSTYRVVSVLTVLHQDWLNGKEYKCKVSNKALPAPI EKTISKAKGQPREPQVCTLPPSREEMTKNQVSLSCAV KGFYPSDIAVEWESNGQPENNYKTTPPVLDSDGSFFL VSKLTVDKSRWQQGNVFSCSVMHEALHNHYTQKSLS LSPGK |
| SEQ ID NO:43 (Combined) | HCDR1 | GFSLSAYSVN |
| SEQ ID NO:44 (Combined) | HCDR2 | MIWGDGKIVYNSALKS |
| SEQ ID NO:45 (Combined) | HCDR3 | DGYYPYAMDN |
| SEQ ID NO:46 (Kabat) | HCDR1 | AYSVN |
| SEQ ID NO:47 (Kabat) | HCDR2 | MIWGDGKIVYNSALKS |
| SEQ ID NO:48 (Kabat) | HCDR3 | DGYYPYAMDN |
| SEQ ID NO:49 (Chothia) | HCDR1 | GFSLSAY |
| SEQ ID NO:50 (Chothia) | HCDR2 | WGDGK |
| SEQ ID NO:51 (Chothia) | HCDR3 | DGYYPYAMDN |
| SEQ ID NO:52 (IMGT) | HCDR1 | GFSLSAYS |
| SEQ ID NO:53 (IMGT) | HCDR2 | IWGDGKI |
| SEQ ID NO:54 (IMGT) | HCDR3 | AGDGYYPYAMDN |
| SEQ ID NO:55 | VH | |
| SEQ ID NO:56 | Heavy Chain mAb2 | QVTLRESGPALVKPTQTLTLTCTVSGFSLSAYSVNWIR QPPGKALEWLAMIWGDGKIVYNSALKSRLTISKDTSKN QVVLTMTNMDPVDTATYYCAGDGYYPYAMDNWGQG SLVTVSSASTKGPSVFPLAPSSKSTSGGTAALGCLVKD YFPEPVTVSWNSGALTSGVHTFPAVLQSSGLYSLSSV VTVPSSSLGTQTYICNVNHKPSNTKVDKRVEPKSCDK THTCPPCPAPELLGGPSVFLFPPKPKDTLYITREPEVT CVVVDVSHEDPEVKFNWYVDGVEVHNAKTKPREEQY NSTYRVVSVLTVLHQDWLNGKEYKCKVSNKALPAPIE KTISKAKGQPREPQVYTLPPCREEMTKNQVSLWCLVK GFYPSDIAVEWESNGQPENNYKTTPPVLDSDGSFFLY SKLTVDKSRWQQGNVFSCSVMHEALHNHYTQKSLSL SPGK |
| | | |

| bbmAb2 | | |
|---|---|---|
| >anti-IL18(mAb1)/ anti-IL13(mAb2)hlgG1_YTE_(KiH 1:1)_NEG1966 | | |
| SEQ ID NO:1 (Combined) | LCDR1 | SGSSSNIGNHYVN |
| SEQ ID NO:2 (Combined) | LCDR2 | RNNHRPS |
| SEQ ID NO:3 (Combined) | LCDR3 | QSWDYSGFSTV |
| SEQ ID NO:4 (Kabat) | LCDR1 | SGSSSNIGNHYVN |
| SEQ ID NO:5 (Kabat) | LCDR2 | RNNHRPS |
| SEQ ID NO:6 (Kabat) | LCDR3 | QSWDYSGFSTV |
| SEQ ID NO:7 (Chothia) | LCDR1 | SSSNIGNHY |
| SEQ ID NO:8 (Chothia) | LCDR2 | RNN |
| SEQ ID NO:9 (Chothia) | LCDR3 | WDYSGFST |
| SEQ ID NO:10 (IMGT) | LCDR1 | SSNIGNHY |
| SEQ ID NO:11 (IMGT) | LCDR2 | RNN |
| SEQ ID NO:12 (IMGT) | LCDR3 | QSWDYSGFSTV |
| SEQ ID NO:13 | VL | DIVLTQPPSVSGAPGQRVTISCSGSSSNIGNHYVNWY QQLPGTAPKLLIYRNNHRPSGVPDRFSGSKSGTSASL AITGLQSEDEADYYCQSWDYSGFSTVFGGGTKLTVL |
| SEQ ID NO:14 | Light Chain mAb1 | DIVLTQPPSVSGAPGQRVTISCSGSSSNIGNHYVNWY QQLPGTAPKLLIYRNNHRPSGVPDRFSGSKSGTSASL AITGLQSEDEADYYCQSWDYSGFSTVFGGGTKLTVLG QPKAAPSVTLFPPSSEELQANKATLVCLISDFYPGAVT VAWKADSSPVKAGVETTTPSKQSNNKYAASSYLSLTP EQWKSHRSYSCQVTHEGSTVEKTVAPTECS |
| SEQ ID NO:15 (Combined) | LCDR1 | RASKSVDSYGNSFMH |
| SEQ ID NO:16 (Combined) | LCDR2 | LASNLES |
| SEQ ID NO:17 (Combined) | LCDR3 | QQNNEDPRT |
| SEQ ID NO:18 (Kabat) | LCDR1 | RASKSVDSYGNSFMH |
| SEQ ID NO:19 (Kabat) | LCDR2 | LASNLES |
| SEQ ID NO:20 (Kabat) | LCDR3 | QQNNEDPRT |
| SEQ ID NO:21 (Chothia) | LCDR1 | SKSVDSYGNSF |
| SEQ ID NO:22 (Chothia) | LCDR2 | LAS |
| SEQ ID NO:23 (Chothia) | LCDR3 | NNEDPR |
| SEQ ID NO:24 (IMGT) | LCDR1 | KSVDSYGNSF |
| SEQ ID NO:25 (IMGT) | LCDR2 | LAS |
| SEQ ID NO:26 (IMGT) | LCDR3 | QQNNEDPRT |
| SEQ ID NO:27 | VL | |
| SEQ ID NO:28 | Light Chain mAb2 | DIVMTQSPDSLSVSLGERATINCRASKSVDSYGNSFM HWYQQKPGQPPKLLIYLASNLESGVPDRFSGSGSGTD FTLTISSLQAEDVAVYYCQQNNEDPRTFGGGTKVEIKR TVAAPSVFIFPPSDEQLKSGTASVVCLLNNFYPREAKV QWKVDNALQSGNSQESVTEQDSKDSTYSLSSTLTLSK ADYEKHKVYACEVTHQGLSSPVTKSFNRGEC |
| SEQ ID NO:29 (Combined) | HCDR1 | GGTFKSYAIS |
| SEQ ID NO:30 (Combined) | HCDR2 | NIIPMTGQTYYAQKFQG |
| SEQ ID NO:31 (Combined) | HCDR3 | AAYHPLVFDN |
| SEQ ID NO:32 (Kabat) | HCDR1 | SYAIS |
| SEQ ID NO:33 (Kabat) | HCDR2 | NIIPMTGQTYYAQKFQG |
| SEQ ID NO:34 (Kabat) | HCDR3 | AAYHPLVFDN |
| SEQ ID NO:35 (Chothia) | HCDR1 | GGTFKSY |
| SEQ ID NO:36 (Chothia) | HCDR2 | IPMTGQ |
| SEQ ID NO:37 (Chothia) | HCDR3 | AAYHPLVFDN |
| SEQ ID NO:38 (IMGT) | HCDR1 | GGTFKSYA |
| SEQ ID NO:39 (IMGT) | HCDR2 | IIPMTGQT |
| SEQ ID NO:40 (IMGT) | HCDR3 | ARAAYHPLVFDN |
| SEQ ID NO:41 | VH | EVQLVQSGAEVKKPGSSVKVSCKASGGTFKSYAISWV RQAPGQGLEWMGNIIPMTGQTYYAQKFQGRVTITADE STSTAYMELSSLRSEDTAVYYCARAAYHPLVFDNWGQ GTLVTVSS |
| SEQ ID NO:57 | Heavy Chain mAb1 | EVQLVQSGAEVKKPGSSVKVSCKASGGTFKSYAISWV RQAPGQGLEWMGNIIPMTGQTYYAQKFQGRVTITADE STSTAYMELSSLRSEDTAVYYCARAAYHPLVFDNWGQ GTLVTVSSASTKGPSVFPLAPSSKSTSGGTAALGCLVK DYFPEPVTVSWNSGALTSGVHTFPAVLQSSGLYSLSS VVTVPSSSLGTQTYICNVNHKPSNTKVDKRVEPKSCD KTHTCPPCPAPELLGGPSVFLFPPKPKDTLYITREPEV TCVVVDVSHEDPEVKFNWYVDGVEVHNAKTKPREEQ YNSTYRVVSVLTVLHQDWLNGKEYKCKVSNKALPAPI EKTISKAKGQPREPQVYTLPPCREEMTKNQVSLWCLV KGFYPSDIAVEWESNGQPENNYKTTPPVLDSDGSFFL YSKLTVDKSRWQQGNVFSCSVMHEALHNHYTQKSLS LSPGK |
| SEQ ID NO:43 (Combined) | HCDR1 | GFSLSAYSVN |
| SEQ ID NO:44 (Combined) | HCDR2 | MIWGDGKIVYNSALKS |
| SEQ ID NO:45 (Combined) | HCDR3 | DGYYPYAMDN |
| SEQ ID NO:46 (Kabat) | HCDR1 | AYSVN |
| SEQ ID NO:47 (Kabat) | HCDR2 | MIWGDGKIVYNSALKS |
| SEQ ID NO:48 (Kabat) | HCDR3 | DGYYPYAMDN |
| SEQ ID NO:49 (Chothia) | HCDR1 | GFSLSAY |
| SEQ ID NO:50 (Chothia) | HCDR2 | WGDGK |
| SEQ ID NO:51 (Chothia) | HCDR3 | DGYYPYAMDN |
| SEQ ID NO:52 (IMGT) | HCDR1 | GFSLSAYS |
| SEQ ID NO:53 (IMGT) | HCDR2 | IWGDGKI |
| SEQ ID NO:54 (IMGT) | HCDR3 | AGDGYYPYAMDN |
| SEQ ID NO:55 | VH | |
| SEQ ID NO:58 | Heavy Chain mAb2 | QVTLRESGPALVKPTQTLTLTCTVSGFSLSAYSVNWIR QPPGKALEWLAMIWGDGKIVYNSALKSRLTISKDTSKN QVVLTMTNMDPVDTATYYCAGDGYYPYAMDNWGQG SLVTVSSASTKGPSVFPLAPSSKSTSGGTAALGCLVKD YFPEPVTVSWNSGALTSGVHTFPAVLQSSGLYSLSSV VTVPSSSLGTQTYICNVNHKPSNTKVDKRVEPKSCDK THTCPPCPAPELLGGPSVFLFPPKPKDTLYITREPEVT CVVVDVSHEDPEVKFNWYVDGVEVHNAKTKPREEQY NSTYRVVSVLTVLHQDWLNGKEYKCKVSNKALPAPIE KTISKAKGQPREPQVCTLPPSREEMTKNQVSLSCAVK GFYPSDIAVEWESNGQPENNYKTTPPVLDSDGSFFLV SKLTVDKSRWQQGNVFSCSVMHEALHNHYTQKSLSL SPGK |
| | | |

| NVP-bbmAb3 | | |
|---|---|---|
| >anti-IL18(mAb1)/ anti-IL13(mAb3)hIgG1_YTE_(KiH 1:1) | | |
| SEQ ID NO:1 (Combined) | LCDR1 | SGSSSNIGNHYVN |
| SEQ ID NO:2 (Combined) | LCDR2 | RNNHRPS |
| SEQ ID NO:3 (Combined) | LCDR3 | QSWDYSGFSTV |
| SEQ ID NO:4 (Kabat) | LCDR1 | SGSSSNIGNHYVN |
| SEQ ID NO:5 (Kabat) | LCDR2 | RNNHRPS |
| SEQ ID NO:6 (Kabat) | LCDR3 | QSWDYSGFSTV |
| SEQ ID NO:7 (Chothia) | LCDR1 | SSSNIGNHY |
| SEQ ID NO:8 (Chothia) | LCDR2 | RNN |
| SEQ ID NO:9 (Chothia) | LCDR3 | WDYSGFST |
| SEQ ID NO:10 (IMGT) | LCDR1 | SSNIGNHY |
| SEQ ID NO:11 (IMGT) | LCDR2 | RNN |
| SEQ ID NO:12 (IMGT) | LCDR3 | QSWDYSGFSTV |
| SEQ ID NO:13 | VL | DIVLTQPPSVSGAPGQRVTISCSGSSSNIGNHYVNWY QQLPGTAPKLLIYRNNHRPSGVPDRFSGSKSGTSASL AITGLQSEDEADYYCQSWDYSGFSTVFGGGTKLTVL |
| SEQ ID NO:14 | Light Chain mAb1 | DIVLTQPPSVSGAPGQRVTISCSGSSSNIGNHYVNWY QQLPGTAPKLLIYRNNHRPSGVPDRFSGSKSGTSASL AITGLQSEDEADYYCQSWDYSGFSTVFGGGTKLTVLG QPKAAPSVTLFPPSSEELQANKATLVCLISDFYPGAVT VAWKADSSPVKAGVETTTPSKQSNNKYAASSYLSLTP EQWKSHRSYSCQVTHEGSTVEKTVAPTECS |
| SEQ ID NO:59 (Combined) | LCDR1 | RAGQSVSSYLV |
| SEQ ID NO:60 (Combined) | LCDR2 | DASNRAT |
| SEQ ID NO:61 (Combined) | LCDR3 | QQRSSWPPVYT |
| SEQ ID NO:62 (Kabat) | LCDR1 | RAGQSVSSYLV |
| SEQ ID NO:63 (Kabat) | LCDR2 | DASNRAT |
| SEQ ID NO:64 (Kabat) | LCDR3 | QQRSSWPPVYT |
| SEQ ID NO:65 (Chothia) | LCDR1 | GQSVSSY |
| SEQ ID NO:66 (Chothia) | LCDR2 | DAS |
| SEQ ID NO:67 (Chothia) | LCDR3 | RSSWPPVY |
| SEQ ID NO:68 (IMGT) | LCDR1 | QSVSSY |
| SEQ ID NO:69 (IMGT) | LCDR2 | DAS |
| SEQ ID NO:70 (IMGT) | LCDR3 | QQRSSWPPVYT |
| SEQ ID NO:71 | VL | EIVLTQSPATLSLSPGERAILSCRAGQSVSSYLVWYQQ KPGQAPRLLIYDASNRATGIPARFSGSGSGTDFTLTISS LEPEDFAVYYCQQRSSWPPVYTFGQGTKLEIK |
| SEQ ID NO:72 | Light Chain mAb3 | EIVLTQSPATLSLSPGERAILSCRAGQSVSSYLVWYQQ KPGQAPRLLIYDASNRATGIPARFSGSGSGTDFTLTISS LEPEDFAVYYCQQRSSWPPVYTFGQGTKLEIKRTVAA PSVFIFPPSDEQLKSGTASVVCLLNNFYPREAKVQWK VDNALQSGNSQESVTEQDSKDSTYSLSSTLTLSKADY EKHKVYACEVTHQGLSSPVTKSFNRGEC |
| SEQ ID NO:73 (Combined) | HCDR1 | GFTFSSYGMH |
| SEQ ID NO:74 (Combined) | HCDR2 | IIWYDGSNKYYADSVKG |
| SEQ ID NO:75 (Combined) | HCDR3 | LWFGDLDAFDI |
| SEQ ID NO:76 (Kabat) | HCDR1 | SYGMH |
| SEQ ID NO:77 (Kabat) | HCDR2 | IIWYDGSNKYYADSVKG |
| SEQ ID NO:78 (Kabat) | HCDR3 | LWFGDLDAFDI |
| SEQ ID NO:79 (Chothia) | HCDR1 | GFTFSSY |
| SEQ ID NO:80 (Chothia) | HCDR2 | WYDGSN |
| SEQ ID NO:81 (Chothia) | HCDR3 | LWFGDLDAFDI |
| SEQ ID NO:82 (IMGT) | HCDR1 | GFTFSSYG |
| SEQ ID NO:83 (IMGT) | HCDR2 | IWYDGSNK |
| SEQ ID NO:84 (IMGT) | HCDR3 | ARLWFGDLDAFDI |
| SEQ ID NO:85 | VH | EVQLVESGGGVVQPGRSLRLSCAASGFTFSSYGMHW VRQAPGKGLEWVAIIWYDGSNKYYADSVKGRFTISRD NSKNTLYLQMNSLRAEDTAVYYCARLWFGDLDAFDIW GQGTMVTVSS |
| SEQ ID NO:86 | Heavy Chain mAb3 | EVQLVESGGGVVQPGRSLRLSCAASGFTFSSYGMHW VRQAPGKGLEWVAIIWYDGSNKYYADSVKGRFTISRD NSKNTLYLQMNSLRAEDTAVYYCARLWFGDLDAFDIW GQGTMVTVSSASTKGPSVFPLAPSSKSTSGGTAALGC LVKDYFPEPVTVSWNSGALTSGVHTFPAVLQSSGLYS LSSVVTVPSSSLGTQTYICNVNHKPSNTKVDKRVEPKS CDKTHTCPPCPAPELLGGPSVFLFPPKPKDTLYITREP EVTCVVVDVSHEDPEVKFNWYVDGVEVHNAKTKPRE EQYNSTYRVVSVLTVLHQDWLNGKEYKCKVSNKALPA PIEKTISKAKGQPREPQVCTLPPSREEMTKNQVSLSCA VKGFYPSDIAVEWESNGQPENNYKTTPPVLDSDGSFF LVSKLTVDKSRWQQGNVFSCSVMHEALHNHYTQKSL SLSPGK |
| SEQ ID NO:29 (Combined) | HCDR1 | GGTFKSYAIS |
| SEQ ID NO:30 (Combined) | HCDR2 | NIIPMTGQTYYAQKFQG |
| SEQ ID NO:31 (Combined) | HCDR3 | AAYHPLVFDN |
| SEQ ID NO:32 (Kabat) | HCDR1 | SYAIS |
| SEQ ID NO:33 (Kabat) | HCDR2 | NIIPMTGQTYYAQKFQG |
| SEQ ID NO:34 (Kabat) | HCDR3 | AAYHPLVFDN |
| SEQ ID NO:35 (Chothia) | HCDR1 | GGTFKSY |
| SEQ ID NO:36 (Chothia) | HCDR2 | IPMTGQ |
| SEQ ID NO:37 (Chothia) | HCDR3 | AAYHPLVFDN |
| SEQ ID NO:38 (IMGT) | HCDR1 | GGTFKSYA |
| SEQ ID NO:39 (IMGT) | HCDR2 | IIPMTGQT |
| SEQ ID NO:40 (IMGT) | HCDR3 | ARAAYHPLVFDN |
| SEQ ID NO:41 | VH | EVQLVQSGAEVKKPGSSVKVSCKASGGTFKSYAISWV RQAPGQGLEWMGNIIPMTGQTYYAQKFQGRVTITADE STSTAYMELSSLRSEDTAVYYCARAAYHPLVFDNWGQ GTLVTVSS |
| SEQ ID NO:87 | Heavy Chain mAb1 | EVQLVQSGAEVKKPGSSVKVSCKASGGTFKSYAISWV RQAPGQGLEWMGNIIPMTGQTYYAQKFQGRVTITADE STSTAYMELSSLRSEDTAVYYCARAAYHPLVFDNWGQ GTLVTVSSASTKGPSVFPLAPSSKSTSGGTAALGCLVK DYFPEPVTVSWNSGALTSGVHTFPAVLQSSGLYSLSS VVTVPSSSLGTQTYICNVNHKPSNTKVDKRVEPKSCD KTHTCPPCPAPELLGGPSVFLFPPKPKDTLYITREPEV TCVVVDVSHEDPEVKFNWYVDGVEVHNAKTKPREEQ YNSTYRVVSVLTVLHQDWLNGKEYKCKVSNKALPAPI EKTISKAKGQPREPQVYTLPPCREEMTKNQVSLWCLV KGFYPSDIAVEWESNGQPENNYKTTPPVLDSDGSFFL YSKLTVDKSRWQQGNVFSCSVMHEALHNHYTQKSLS LSPGK |
| | | |

| NVP-bbmAb4 | | |
|---|---|---|
| >anti-IL18(mAb1)/ anti-IL13(mAb2)hIgG1_LALA_YTE_(KiH 1:1) | | |
| SEQ ID NO:1 (Combined) | LCDR1 | SGSSSNIGNHYVN |
| SEQ ID NO:2 (Combined) | LCDR2 | RNNHRPS |
| SEQ ID NO:3 (Combined) | LCDR3 | QSWDYSGFSTV |
| SEQ ID NO:4 (Kabat) | LCDR1 | SGSSSNIGNHYVN |
| SEQ ID NO:5 (Kabat) | LCDR2 | RNNHRPS |
| SEQ ID NO:6 (Kabat) | LCDR3 | QSWDYSGFSTV |
| SEQ ID NO:7 (Chothia) | LCDR1 | SSSNIGNHY |
| SEQ ID NO:8 (Chothia) | LCDR2 | RNN |
| SEQ ID NO:9 (Chothia) | LCDR3 | WDYSGFST |
| SEQ ID NO:10 (IMGT) | LCDR1 | SSNIGNHY |
| SEQ ID NO:11 (IMGT) | LCDR2 | RNN |
| SEQ ID NO:12 (IMGT) | LCDR3 | QSWDYSGFSTV |
| SEQ ID NO:13 | VL | DIVLTQPPSVSGAPGQRVTISCSGSSSNIGNHYVNWY QQLPGTAPKLLIYRNNHRPSGVPDRFSGSKSGTSASL AITGLQSEDEADYYCQSWDYSGFSTVFGGGTKLTVL |
| SEQ ID NO:14 | Light Chain mAb1 | DIVLTQPPSVSGAPGQRVTISCSGSSSNIGNHYVNWY QQLPGTAPKLLIYRNNHRPSGVPDRFSGSKSGTSASL AITGLQSEDEADYYCQSWDYSGFSTVFGGGTKLTVLG QPKAAPSVTLFPPSSEELQANKATLVCLISDFYPGAVT VAWKADSSPVKAGVETTTPSKQSNNKYAASSYLSLTP EQWKSHRSYSCQVTHEGSTVEKTVAPTECS |
| SEQ ID NO:15 (Combined) | LCDR1 | RASKSVDSYGNSFMH |
| SEQ ID NO:16 (Combined) | LCDR2 | LASNLES |
| SEQ ID NO:17 (Combined) | LCDR3 | QQNNEDPRT |
| SEQ ID NO:18 (Kabat) | LCDR1 | RASKSVDSYGNSFMH |
| SEQ ID NO:19 (Kabat) | LCDR2 | LASNLES |
| SEQ ID NO:20 (Kabat) | LCDR3 | QQNNEDPRT |
| SEQ ID NO:21 (Chothia) | LCDR1 | SKSVDSYGNSF |
| SEQ ID NO:22 (Chothia) | LCDR2 | LAS |
| SEQ ID NO:23 (Chothia) | LCDR3 | NNEDPR |
| SEQ ID NO:24 (IMGT) | LCDR1 | KSVDSYGNSF |
| SEQ ID NO:25 (IMGT) | LCDR2 | LAS |
| SEQ ID NO:26 (IMGT) | LCDR3 | QQNNEDPRT |
| SEQ ID NO:27 | VL | |
| SEQ ID NO:28 | Light Chain MAb2 | DIVMTQSPDSLSVSLGERATINCRASKSVDSYGNSFM HWYQQKPGQPPKLLIYLASNLESGVPDRFSGSGSGTD FTLTISSLQAEDVAVYYCQQNNEDPRTFGGGTKVEIKR TVAAPSVFIFPPSDEQLKSGTASVVCLLNNFYPREAKV QWKVDNALQSGNSQESVTEQDSKDSTYSLSSTLTLSK ADYEKHKVYACEVTHQGLSSPVTKSFNRGEC |
| SEQ ID NO:29 (Combined) | HCDR1 | GGTFKSYAIS |
| SEQ ID NO:30 (Combined) | HCDR2 | NIIPMTGQTYYAQKFQG |
| SEQ ID NO:31 (Combined) | HCDR3 | AAYHPLVFDN |
| SEQ ID NO:32 (Kabat) | HCDR1 | SYAIS |
| SEQ ID NO:33 (Kabat) | HCDR2 | NIIPMTGQTYYAQKFQG |
| SEQ ID NO:34 (Kabat) | HCDR3 | AAYHPLVFDN |
| SEQ ID NO:35 (Chothia) | HCDR1 | GGTFKSY |
| SEQ ID NO:36 (Chothia) | HCDR2 | IPMTGQ |
| SEQ ID NO:37 (Chothia) | HCDR3 | AAYHPLVFDN |
| SEQ ID NO:38 (IMGT) | HCDR1 | GGTFKSYA |
| SEQ ID NO:39 (IMGT) | HCDR2 | IIPMTGQT |
| SEQ ID NO:40 (IMGT) | HCDR3 | ARAAYHPLVFDN |
| SEQ ID NO:41 | VH | EVQLVQSGAEVKKPGSSVKVSCKASGGTFKSYAISWV RQAPGQGLEWMGNIIPMTGQTYYAQKFQGRVTITADE STSTAYMELSSLRSEDTAVYYCARAAYHPLVFDNWGQ GTLVTVSS |
| SEQ ID NO:88 | Heavy Chain mAb1 | EVQLVQSGAEVKKPGSSVKVSCKASGGTFKSYAISWV RQAPGQGLEWMGNIIPMTGQTYYAQKFQGRVTITADE STSTAYMELSSLRSEDTAVYYCARAAYHPLVFDNWGQ GTLVTVSSASTKGPSVFPLAPSSKSTSGGTAALGCLVK DYFPEPVTVSWNSGALTSGVHTFPAVLQSSGLYSLSS VVTVPSSSLGTQTYICNVNHKPSNTKVDKRVEPKSCD KTHTCPPCPAPEAAGGPSVFLFPPKPKDTLYITREPEV TCVVVDVSHEDPEVKFNWYVDGVEVHNAKTKPREEQ YNSTYRVVSVLTVLHQDWLNGKEYKCKVSNKALPAPI EKTISKAKGQPREPQVYTLPPCREEMTKNQVSLWCLV KGFYPSDIAVEWESNGQPENNYKTTPPVLDSDGSFFL YSKLTVDKSRWQQGNVFSCSVMHEALHNHYTQKSLS LSPGK |
| SEQ ID NO:43 (Combined) | HCDR1 | GFSLSAYSVN |
| SEQ ID NO:44 (Combined) | HCDR2 | MIWGDGKIVYNSALKS |
| SEQ ID NO:45 (Combined) | HCDR3 | DGYYPYAMDN |
| SEQ ID NO:46 (Kabat) | HCDR1 | AYSVN |
| SEQ ID NO:47 (Kabat) | HCDR2 | MIWGDGKIVYNSALKS |
| SEQ ID NO:48 (Kabat) | HCDR3 | DGYYPYAMDN |
| SEQ ID NO:49 (Chothia) | HCDR1 | GFSLSAY |
| SEQ ID NO:50 (Chothia) | HCDR2 | WGDGK |
| SEQ ID NO:51 (Chothia) | HCDR3 | DGYYPYAMDN |
| SEQ ID NO:52 (IMGT) | HCDR1 | GFSLSAYS |
| SEQ ID NO:53 (IMGT) | HCDR2 | IWGDGKI |
| SEQ ID NO:54 (IMGT) | HCDR3 | AGDGYYPYAMDN |
| SEQ ID NO:55 | VH | |
| SEQ ID NO:89 | Heavy Chain MAb2 | QVTLRESGPALVKPTQTLTLTCTVSGFSLSAYSVNWIR QPPGKALEWLAMIWGDGKIVYNSALKSRLTISKDTSKN QVVLTMTNMDPVDTATYYCAGDGYYPYAMDNWGQG SLVTVSSASTKGPSVFPLAPSSKSTSGGTAALGCLVKD YFPEPVTVSWNSGALTSGVHTFPAVLQSSGLYSLSSV VTVPSSSLGTQTYICNVNHKPSNTKVDKRVEPKSCDK THTCPPCPAPEAAGGPSVFLFPPKPKDTLYITREPEVT CVVVDVSHEDPEVKFNWYVDGVEVHNAKTKPREEQY NSTYRVVSVLTVLHQDWLNGKEYKCKVSNKALPAPIE KTISKAKGQPREPQVCTLPPSREEMTKNQVSLSCAVK GFYPSDIAVEWESNGQPENNYKTTPPVLDSDGSFFLV SKLTVDKSRWQQGNVFSCSVMHEALHNHYTQKSLSL SPGK |
| | | |

| NVP-bbmAb5 | | |
|---|---|---|
| >anti-IL13(MAb2) / anti-IL18(mAb1)hIgG1_YTE_LALA_(KiH 1:1) | | |
| SEQ ID NO:1 (Combined) | LCDR1 | SGSSSNIGNHYVN |
| SEQ ID NO:2 (Combined) | LCDR2 | RNNHRPS |
| SEQ ID NO:3 (Combined) | LCDR3 | QSWDYSGFSTV |
| SEQ ID NO:4 (Kabat) | LCDR1 | SGSSSNIGNHYVN |
| SEQ ID NO:5 (Kabat) | LCDR2 | RNNHRPS |
| SEQ ID NO:6 (Kabat) | LCDR3 | QSWDYSGFSTV |
| SEQ ID NO:7 (Chothia) | LCDR1 | SSSNIGNHY |
| SEQ ID NO:8 (Chothia) | LCDR2 | RNN |
| SEQ ID NO:9 (Chothia) | LCDR3 | WDYSGFST |
| SEQ ID NO:10 (IMGT) | LCDR1 | SSNIGNHY |
| SEQ ID NO:11 (IMGT) | LCDR2 | RNN |
| SEQ ID NO:12 (IMGT) | LCDR3 | QSWDYSGFSTV |
| SEQ ID NO:13 | VL | DIVLTQPPSVSGAPGQRVTISCSGSSSNIGNHYVNWY QQLPGTAPKLLIYRNNHRPSGVPDRFSGSKSGTSASL AITGLQSEDEADYYCQSWDYSGFSTVFGGGTKLTVL |
| SEQ ID NO:14 | Light Chain mAb1 | DIVLTQPPSVSGAPGQRVTISCSGSSSNIGNHYVNWY QQLPGTAPKLLIYRNNHRPSGVPDRFSGSKSGTSASL AITGLQSEDEADYYCQSWDYSGFSTVFGGGTKLTVLG QPKAAPSVTLFPPSSEELQANKATLVCLISDFYPGAVT VAWKADSSPVKAGVETTTPSKQSNNKYAASSYLSLTP EQWKSHRSYSCQVTHEGSTVEKTVAPTECS |
| SEQ ID NO:15 (Combined) | LCDR1 | RASKSVDSYGNSFMH |
| SEQ ID NO:16 (Combined) | LCDR2 | LASNLES |
| SEQ ID NO:17 (Combined) | LCDR3 | QQNNEDPRT |
| SEQ ID NO:18 (Kabat) | LCDR1 | RASKSVDSYGNSFMH |
| SEQ ID NO:19 (Kabat) | LCDR2 | LASNLES |
| SEQ ID NO:20 (Kabat) | LCDR3 | QQNNEDPRT |
| SEQ ID NO:21 (Chothia) | LCDR1 | SKSVDSYGNSF |
| SEQ ID NO:22 (Chothia) | LCDR2 | LAS |
| SEQ ID NO:23 (Chothia) | LCDR3 | NNEDPR |
| SEQ ID NO:24 (IMGT) | LCDR1 | KSVDSYGNSF |
| SEQ ID NO:25 (IMGT) | LCDR2 | LAS |
| SEQ ID NO:26 (IMGT) | LCDR3 | QQNNEDPRT |
| SEQ ID NO:27 | VL | |
| SEQ ID NO:28 | Light Chain MAb2 | DIVMTQSPDSLSVSLGERATINCRASKSVDSYGNSFM HWYQQKPGQPPKLLIYLASNLESGVPDRFSGSGSGTD FTLTISSLQAEDVAVYYCQQNNEDPRTFGGGTKVEIKR TVAAPSVFIFPPSDEQLKSGTASVVCLLNNFYPREAKV QWKVDNALQSGNSQESVTEQDSKDSTYSLSSTLTLSK ADYEKHKVYACEVTHQGLSSPVTKSFNRGEC |
| SEQ ID NO:29 (Combined) | HCDR1 | GGTFKSYAIS |
| SEQ ID NO:30 (Combined) | HCDR2 | NIIPMTGQTYYAQKFQG |
| SEQ ID NO:31 (Combined) | HCDR3 | AAYHPLVFDN |
| SEQ ID NO:32 (Kabat) | HCDR1 | SYAIS |
| SEQ ID NO:33 (Kabat) | HCDR2 | NIIPMTGQTYYAQKFQG |
| SEQ ID NO:34 (Kabat) | HCDR3 | AAYHPLVFDN |
| SEQ ID NO:35 (Chothia) | HCDR1 | GGTFKSY |
| SEQ ID NO:36 (Chothia) | HCDR2 | IPMTGQ |
| SEQ ID NO:37 (Chothia) | HCDR3 | AAYHPLVFDN |
| SEQ ID NO:38 (IMGT) | HCDR1 | GGTFKSYA |
| SEQ ID NO:39 (IMGT) | HCDR2 | IIPMTGQT |
| SEQ ID NO:40 (IMGT) | HCDR3 | ARAAYHPLVFDN |
| SEQ ID NO:41 | VH | EVQLVQSGAEVKKPGSSVKVSCKASGGTFKSYAISWV RQAPGQGLEWMGNIIPMTGQTYYAQKFQGRVTITADE STSTAYMELSSLRSEDTAVYYCARAAYHPLVFDNWGQ GTLVTVSS |
| SEQ ID NO:90 | Heavy Chain mAb1 | EVQLVQSGAEVKKPGSSVKVSCKASGGTFKSYAISWV RQAPGQGLEWMGNIIPMTGQTYYAQKFQGRVTITADE STSTAYMELSSLRSEDTAVYYCARAAYHPLVFDNWGQ GTLVTVSSASTKGPSVFPLAPSSKSTSGGTAALGCLVK DYFPEPVTVSWNSGALTSGVHTFPAVLQSSGLYSLSS VVTVPSSSLGTQTYICNVNHKPSNTKVDKRVEPKSCD KTHTCPPCPAPEAAGGPSVFLFPPKPKDTLYITREPEV TCVVVDVSHEDPEVKFNWYVDGVEVHNAKTKPREEQ YNSTYRVVSVLTVLHQDWLNGKEYKCKVSNKALPAPI EKTISKAKGQPREPQVCTLPPSREEMTKNQVSLSCAV KGFYPSDIAVEWESNGQPENNYKTTPPVLDSDGSFFL VSKLTVDKSRWQQGNVFSCSVMHEALHNHYTQKSLS LSPGK |
| SEQ ID NO:43 (Combined) | HCDR1 | GFSLSAYSVN |
| SEQ ID NO:44 (Combined) | HCDR2 | MIWGDGKIVYNSALKS |
| SEQ ID NO:45 (Combined) | HCDR3 | DGYYPYAMDN |
| SEQ ID NO:46 (Kabat) | HCDR1 | AYSVN |
| SEQ ID NO:47 (Kabat) | HCDR2 | MIWGDGKIVYNSALKS |
| SEQ ID NO:48 (Kabat) | HCDR3 | DGYYPYAMDN |
| SEQ ID NO:49 (Chothia) | HCDR1 | GFSLSAY |
| SEQ ID NO:50 (Chothia) | HCDR2 | WGDGK |
| SEQ ID NO:51 (Chothia) | HCDR3 | DGYYPYAMDN |
| SEQ ID NO:52 (IMGT) | HCDR1 | GFSLSAYS |
| SEQ ID NO:53 (IMGT) | HCDR2 | IWGDGKI |
| SEQ ID NO:54 (IMGT) | HCDR3 | AGDGYYPYAMDN |
| SEQ ID NO:55 | VH | |
| SEQ ID NO:91 | Heavy Chain mAb2 | QVTLRESGPALVKPTQTLTLTCTVSGFSLSAYSVNWIR QPPGKALEWLAMIWGDGKIVYNSALKSRLTISKDTSKN QVVLTMTNMDPVDTATYYCAGDGYYPYAMDNWGQG SLVTVSSASTKGPSVFPLAPSSKSTSGGTAALGCLVKD YFPEPVTVSWNSGALTSGVHTFPAVLQSSGLYSLSSV VTVPSSSLGTQTYICNVNHKPSNTKVDKRVEPKSCDK THTCPPCPAPEAAGGPSVFLFPPKPKDTLYITREPEVT CVVVDVSHEDPEVKFNWYVDGVEVHNAKTKPREEQY NSTYRVVSVLTVLHQDWLNGKEYKCKVSNKALPAPIE KTISKAKGQPREPQVYTLPPCREEMTKNQVSLWCLVK |
| | | GFYPSDIAVEWESNGQPENNYKTTPPVLDSDGSFFLY SKLTVDKSRWQQGNVFSCSVMHEALHNHYTQKSLSL SPGK |

In some embodiments, a bispecific antibody comprises an amino acid sequence having 1, 2, or 3 substitutions, deletions, or insertions relative to a sequence of Table 1 within the scope of the claims.

The formats of the exemplary IL-13/IL-18 bispecific antibodies are illustrated in Table 2. All bbmAbs combine anti-IL13 and anti-IL-18 binding domains and are based on human IgG1 format, with all containing YTE half-life extension mutations while two contain LALA silencing mutations in addition. In more detail, bbmAb2, bbmAb1 and bbmAb3 contain YTE half-life extension mutations in Fc. bbmAb4 and bbmAb5 contain both LALA silencing and YTE half-life extension mutations. bbmAb2, bbmAb1, bbmAb4 and bbmAb5 combine the variable domains of mAb1 and MAb2, while bbmAb3 combines the variable domains of mAb1 and mAb3.

bbmAb2, bbmAb4 and bbmAb3 carry the KiH knob heterodimerization mutation in the anti-IL-18 heavy chain Fc, while the KiH hole mutations are in the anti-IL-13 heavy chain Fc. bbmAb2, bbmAb4 and bbmAb3 carry the KiH knob mutation in the anti-IL-18 heavy chain Fc and the KiH hole mutations in the anti-IL-13 heavy chain Fc.

**Table 2. The formats of the exemplary IL-13/IL-18 bispecific antibodies**

| **Candidate** | **Silencing / HLE** | **Anti- IL18 origin** | **Anti- IL13 origin** | **KiH heterodimerization Format** |
|---|---|---|---|---|
| bbmAb2 | Wt Fc / YTE | mAb1 (variable domain) | mAb2 (variable domain) | Anti-IL18 knob, anti-IL13 hole |
| bbmAb1 | | | | Anti-IL18 hole, Anti- IL13 knob |
| bbmAb4 | LALA / YTE | | | Anti-IL18 knob, anti-IL13 hole |
| bbmAb5 | | | | Anti-IL18 hole, Anti- IL13 knob |
| bbmAb3 | Wt Fc / YTE | | mAb3 (variable domain) | Anti-IL18 knob, anti-IL13 hole |

### VI. Modification of the bispecific antibodies of the present disclosure

Disclosed herein are also variants of the molecules described herein having various modifications in binding domains, variable domains and/or constant regions, as well as fusions and conjugates of the disclosed molecules. For example, the Fc region of the disclosed bispecific antibody may be wild-type, or it may be modified to achieve various outcomes. Preferred modifications to the Fc include the "LS" mutation (M428L, N434S, (EU numbering)) and the "YTE" mutation (M252Y, S254T, T256E (EU Numbering)) for half-life extension, the "DAPA" mutation (D265A,P329A (EU Numbering)) for effector silencing, and knob-in-hole mutations (e.g., knob S354C, T366W; hole Y349C, T366S, L368A, Y407V (EU Numbering)) that facilitate proper chain pairing.

### A. Bispecific antibodies with variable region modifications

Each of the VH and VL domains of the bispecific antibodies of the present disclosure comprises hypervariable regions CDR1, CDR2, and CDR3 sequences. In certain embodiments, one or more of these CDR sequences may have conservative modifications of the amino acid sequences, and wherein the modified molecules retain or have enhanced binding properties as compared to the parent antibodies.

In addition, it has been found that in certain instances it is beneficial to mutate residues within the framework regions to maintain or enhance the antigen binding ability of the antibody (see e.g., US 5,530,101; US 5,585,089; US 5,693,762 and US 6,180,370 to Queen et al). The antibodies of the present disclosure can be modified by introducing such mutations to its variable region frameworks in order to improve the binding properties.

Another type of variable region modification is to mutate amino acid residues within the VH and/or VL CDR1, CDR2 and/or CDR3 domains to thereby improve one or more binding properties (e.g., affinity) of the antibody of interest, known as "affinity maturation." Site-directed mutagenesis or PCR-mediated mutagenesis can be performed to introduce the mutation(s) and the effect on antibody binding, or other functional property of interest, can be evaluated in in vitro or in vivo assays as described herein and provided in the Examples. Conservative modifications (as discussed above) can be introduced. The mutations may be amino acid substitutions, additions or deletions. Moreover, typically no more than one, two, three, four or five residues, preferably one or two, within a CDR region are altered.

Amino acid sequence variants of the bispecific antibodies can be prepared by introducing appropriate nucleotide changes into the encoding DNAs, or by synthesis of the desired variants. Such variants include, for example, deletions from, or insertions or substitutions of, residues within the amino acid sequences of present molecules. Any combination of deletion, insertion, and substitution is made to arrive at the final construct, provided that the final construct possesses the desired antigen-binding characteristics. The amino acid changes also may alter post-translational processes of the molecules, such as changing the number or position of glycosylation sites.

Disclosed herein are variants of the bispecific antibodies having amino acid conservative modifications in variable regions and/or constant regions.

### B. Bispecific antibodies with enhanced heterodimerization

Inadequate heterodimerization of two antibody heavy chain domains can be an obstacle for increasing the yield of desired bispecific antibodies and represents challenges for purification. A variety of approaches available can be used in to enhance dimerization of the two heavy chain domains of bispecific antibody molecules, as disclosed in EP 1870459A1; US 5,582,996; US 5,731,168; US 5,910,573; US 5,932,448; US 6,833,441; US 7,183,076; US 2006204493A1; and WO 2009/089004A1.

The present disclosure provides methods of enhancing dimerization (hetero-dimerization) of two interacting heterologous polypeptides and/or reducing dimerization (homodimerization) of two identical polypeptides. Typically, each of the two interacting polypeptides comprises an Fc region having a CH2 and a CH3 domain of an antibody. The CH3 domains are derived from the constant region of an antibody of subclass of IgG (IgG1, IgG2, IgG3 and IgG4) class, most preferably IgG1.

Typically, the polypeptides of the disclosure comprise other antibody fragments in addition to CH3 domains, such as, CH1 domains, CH2 domains, hinge domain, VH domain(s), VL domain(s), CDR(s), and/or antigen-binding fragments described herein, such Fab. These antibody fragments are derived from various types of antibodies described herein, for example, polyclonal antibody, monoclonal antibodies, chimeric antibodies, humanized antibodies, human antibodies, bispecific or multi-specific antibodies, camelised antibodies, anti-idiotypic (anti-Id) antibodies and antibody conjugates. Heterodimerization of the two different heavy chains at CH3 domains give rise to the desired antibody molecule, while homodimerization of identical heavy chains will reduce yield of the desired antibody or molecule. In an exemplary embodiment, the two or more hetero-polypeptide chains comprise two chains comprising CH3 domains and forming the molecules of any of the bispecific antibody of the present disclosure. In an embodiment, the two hetero-polypeptide chains comprising CH3 domains comprise modifications that favour heterodimeric association of the polypeptides, relative to unmodified chains. Various examples of modification strategies are provided below.

### Knob-in-Hole (KIH) (also known as "key-in-hole")

Bispecific antibodies of the present disclosure may comprise one or more, e.g., a plurality, of mutations to one or more of the constant domains, e.g., to the CH3 domains. In one example, the bispecific antibody of the present disclosure comprises two polypeptides that each comprise a heavy chain Fc or a constant domain of an antibody, e.g., a CH2 or CH3 domain. In an example, the two heavy chain constant domains, e.g., the CH2 or CH3 domains of the bispecific antibody comprise one or more mutations that allow for a heterodimeric association between the two chains. In one aspect, the one or more mutations are disposed on the CH2 domain of the two heavy chains of the bispecific antibody. In one aspect, the one or more mutations are disposed on the CH3 domains of at least two polypeptides of the bispecific antibody. In one aspect, the one or more mutations to a first polypeptide of bispecific antibody comprising a heavy chain constant domain creates a "knob" and the one or more mutations to a second polypeptide of bispecific antibody comprising a heavy chain constant domain creates a "hole," such that heterodimerization of the polypeptides of the bispecific antibody comprising a heavy chain constant domain causes the "knob" to interface (e.g., interact, e.g., a CH2 domain of a first polypeptide interacting with a CH2 domain of a second polypeptide, or a CH3 domain of a first polypeptide interacting with a CH3 domain of a second polypeptide) with the "hole." As the term is used herein, a "knob" refers to at least one amino acid side chain which projects from the interface of a first polypeptide of the bispecific antibody comprising a heavy chain constant domain and is therefore positionable in a compensatory "hole" in the interface with a second polypeptide of the bispecific antibody comprising a heavy chain constant domain so as to stabilize the heteromultimer, and thereby favour heteromultimer formation over homomultimer formation, for example. The knob may exist in the original interface or may be introduced synthetically (e.g., by altering nucleic acid encoding the interface). The preferred import residues for the formation of a knob are generally naturally occurring amino acid residues and are preferably selected from arginine (R), phenylalanine (F), tyrosine (Y) and tryptophan (W). Most preferred are tryptophan and tyrosine. In the preferred embodiment, the original residue for the formation of the protuberance has a small side chain volume, such as alanine, asparagine, aspartic acid, glycine, serine, threonine or valine.

A "hole" refers to at least one amino acid side chain which is recessed from the interface of a second polypeptide of the bispecific antibody comprising a heavy chain constant domain and therefore accommodates a corresponding knob on the adjacent interfacing surface of a first polypeptide of the bispecific antibody comprising a heavy chain constant domain. The hole may exist in the original interface or may be introduced synthetically (e.g., by altering nucleic acid encoding the interface). The preferred import residues for the formation of a hole are usually naturally occurring amino acid residues and are preferably selected from alanine (A), serine (S), threonine (T) and valine (V). Most preferred are serine, alanine or threonine. In the preferred embodiment, the original residue for the formation of the hole has a large side chain volume, such as tyrosine, arginine, phenylalanine or tryptophan.

In one embodiment, a first CH3 domain is mutated at residue 366, 405 or 407 according to the EU numbering scheme of Kabat et al. (pp. 688-696 in Sequences of proteins of immunological interest, 5th ed., Vol. 1 (1991; NIH, Bethesda, Md.)) to create either a "knob" or a hole" (as described above), and the second CH3 domain that heterodimerizes with the first CH3 domain is mutated at: residue 407 if residue 366 is mutated in the first CH3 domain, residue 394 if residue 405 is mutated in the first CH3 domain, or residue 366 if residue 407 is mutated in the first CH3 domain (EU numbering), to create a "hole" or "knob" complementary to the "knob" or "hole" of the first CH3 domain.

In another embodiment, a first CH3 domain is mutated at residue 366 (EU numbering) to create either a "knob" or a hole" (as described above), and the second CH3 domain that heterodimerizes with the first CH3 domain is mutated at residues 366, 368 and/or 407 (EU numbering), to create a "hole" or "knob" complementary to the "knob" or "hole" of the first CH3 domain. In one embodiment, the mutation to the first CH3 domain introduces a tyrosine (Y) residue at position 366. In an embodiment, the mutation to the first CH3 is T366Y. In one embodiment, the mutation to the first CH3 domain introduces a tryptophan (W) residue at position 366. In an embodiment, the mutation to the first CH3 is T366W. In embodiments, the mutation to the second CH3 domain that heterodimerizes with the first CH3 domain mutated at position 366 (e.g., has a tyrosine (Y) or tryptophan (W) introduced at position 366, e.g., comprises the mutation T366Y or T366W), comprises a mutation at position 366, a mutation at position 368 and a mutation at position 407 (EU numbering). In embodiments, the mutation at position 366 introduces a serine (S) residue, the mutation at position 368 introduces an alanine (A), and the mutation at position 407 introduces a valine (V). In embodiments, the mutations comprise T366S, L368A and Y407V. In one embodiment the first CH3 domain of the bispecific antibody comprises the mutation T366Y, and the second CH3 domain that heterodimerizes with the first CH3 domain comprises the mutations T366S, L368A and Y407V, or vice versa. In one embodiment the first CH3 domain of the bispecific antibody comprises the mutation T366W, and the second CH3 domain that heterodimerizes with the first CH3 domain comprises the mutations T366S, L368A and Y407V, or vice versa.

Additional knob-in-hole mutation pairs suitable for use in any of the bispecific antibodies of the present disclosure are further described in, for example, WO1996/027011, and Merchant et al., (1998) Nat. Biotechnol., 16: 677-681.

In any of the embodiments described herein, the CH3 domains may be additionally mutated to introduce a pair of cysteine residues. Without being bound by theory, it is believed that the introduction of a pair of cysteine residues capable of forming a disulfide bond provide stability to the heterodimerized antibody. In embodiments, the first CH3 domain comprises a cysteine at position 354 (EU numbering), and the second CH3 domain that heterodimerizes with the first CH3 domain comprises a cysteine at position 349 (EU numbering) In embodiments, the first CH3 domain of the bispecific antibody comprises a cysteine at position 354 (e.g., comprises the mutation S354C) and a tyrosine (Y) at position 366 (e.g., comprises the mutation T366Y), and the second CH3 domain that heterodimerizes with the first CH3 domain comprises a cysteine at position 349 (e.g., comprises the mutation Y349C), a serine at position 366 (e.g., comprises the mutation T366S), an alanine at position 368 (e.g., comprises the mutation L368A), and a valine at position 407 (e.g., comprises the mutation Y407V). In embodiments, the first CH3 domain of the bispecific antibody comprises a cysteine at position 354 (e.g., comprises the mutation S354C) and a tryptophan (W) at position 366 (e.g., comprises the mutation T366W), and the second CH3 domain that heterodimerizes with the first CH3 domain comprises a cysteine at position 349 (e.g., comprises the mutation Y349C), a serine at position 366 (e.g., comprises the mutation T366S), an alanine at position 368 (e.g., comprises the mutation L368A), and a valine at position 407 (e.g., comprises the mutation Y407V).

### IgG Heterodimerization

In one aspect, heterodimerization of the polypeptide chains (e.g., of the half antibodies) of the bispecific antibody is increased by introducing one or more mutations in a CH3 domain which is derived from the IgG1 antibody class. In an embodiment, the mutations comprise a K409R mutation to one CH3 domain paired with F405L mutation in the second CH3 domain (EU numbering scheme). Additional mutations may also, or alternatively, be at positions 366, 368, 370, 399, 405, 407, and 409 (EU numbering). Preferably, heterodimerization of polypeptides comprising such mutations is achieved under reducing conditions, e.g., 10-100 mM 2-MEA (e.g., 25, 50, or 100 mM 2-MEA) for 1-10, e.g., 1.5-5, e.g., 5, hours at 25-37 °C, e.g., 25C or 37 °C.

The amino acid replacements described herein are introduced into the CH3 domains using techniques which are known in the art. Normally the DNA encoding the heavy chain(s) is genetically engineered using the techniques described in Mutagenesis: a Practical Approach. Oligonucleotide-mediated mutagenesis is a preferred method for preparing substitution variants of the DNA encoding the two hybrid heavy chains. This technique is known in the art as described by Adelman et al., (1983) DNA, 2:183.

A suitable IgG heterodimerization strategy is described in, for example, WO2008/119353, WO2011/131746, and WO2013/060867.

In any of the embodiments described herein, the CH3 domains may be additionally mutated to introduce a pair of cysteine residues. Without being bound by theory, it is believed that the introduction of a pair of cysteine residues capable of forming a disulfide bond provide stability to the heterodimerized bispecific antibody. In embodiments, the first CH3 domain comprises a cysteine at position 354 (EU numbering), and the second CH3 domain that heterodimerizes with the first CH3 domain comprises a cysteine at position 349 (EU numbering)

### Polar Bridge

In one aspect, heterodimerization of the polypeptide chains (e.g., of the half antibodies) of the bispecific antibody is increased by introducing mutations based on the "polar-bridging" rational, which is to make residues at the binding interface of the two polypeptide chains to interact with residues of similar (or complimentary) physical property in the heterodimer configuration, while with residues of different physical property in the homodimer configuration. In particular, these mutations are designed so that, in the heterodimer formation, polar residues interact with polar residues, while hydrophobic residues interact with hydrophobic residues. In contrast, in the homodimer formation, residues are mutated so that polar residues interact with hydrophobic residues. The favourable interactions in the heterodimer configuration and the unfavourable interactions in the homodimer configuration work together to make it more likely for CH3 domains to form heterodimers than to form homodimers.

In an exemplary embodiment, the above mutations are generated at one or more positions of residues 364, 368, 399, 405, 409, and 411 of CH3 domain (EU numbering).

In one aspect, one or more mutations selected from a group consisting of: Ser364Leu, Thr366Val, Leu368Gln, Asp399Lys, Phe405Ser, Lys409Phe and Thr411Lys are introduced into one of the two CH3 domains. For example, Ser364Leu: original residue of serine at position 364 is replaced by leucine; Thr366Val: original residue of threonine at position 366 is replaced by valine; Leu368Gln: original residue of leucine at position 368 is replaced by glutamine; Asp399Lys: original residue aspartic acid at position 399 is replaced by lysine; Phe405Ser: original residue phenylalanine at position 405 is replaced by serine; Lys409Phe: original residue lysine at position 409 is replaced by phenylalanine; Thr411Lys: original residue of threonine at position 411 is replaced by lysine.

In another aspect, the other CH3 can be introduced with one or more mutations selected from a group consisting of: Tyr407Phe, Lys409Gln and Thr411Asp (for example, Tyr407Phe: original residue tyrosine at position 407 is replaced by phenyalanine; Lys409Glu: original residue lysine at position 409 is replaced by glutamic acid; Thr411Asp: original residue of threonine at position 411 is replaced by aspartic acid).

In a further aspect, one CH3 domain has one or more mutations selected from a group consisting of: Ser364Leu, Thr366Val, Leu368Gln, Asp399Lys, Phe405Ser, Lys409Phe and Thr411Lys, while the other CH3 domain has one or more mutations selected from a group consisting of: Tyr407Phe, Lys409Gln and Thr411Asp.

In one exemplary embodiment, the original residue of threonine at position 366 of one CH3 domain is replaced by valine, while the original residue of tyrosine at position 407 of the other CH3 domain is replaced by phenylalanine.

In another exemplary embodiment, the original residue of serine at position 364 of one CH3 domain is replaced by leucine, while the original residue of leucine at position 368 of the same CH3 domain is replaced by glutamine.

In yet another exemplary embodiment, the original residue of phenylalanine at position 405 of one CH3 domain is replaced by serine and the original residue of lysine at position 409 of this CH3 domain is replaced by phenylalanine, while the original residue of lysine at position 409 of the other CH3 domain is replaced by glutamine.

In yet another exemplary embodiment, the original residue of aspartic acid at position 399 of one CH3 domain is replaced by lysine, and the original residue of threonine at position 411 of the same CH3 domain is replaced by lysine, while the original residue of threonine at position 411 of the other CH3 domain is replaced by aspartic acid.

The amino acid replacements described herein can be introduced into the CH3 domains using techniques which are known in the art. Normally the DNA encoding the heavy chain(s) is genetically engineered using the techniques described in Mutagenesis: a Practical Approach. Oligonucleotide-mediated mutagenesis is a preferred method for preparing substitution variants of the DNA encoding the two hybrid heavy chains. This technique is known in the art as described by Adelman et al., (1983) DNA, 2:183.

The polar bridge strategy is described in, for example, WO2006/106905, WO2009/089004 and Gunasekaran K et al.,(2010) J Biol Chem., 285: 19637-19646.

In any of the embodiments described herein, the CH3 domains may be additionally mutated to introduce a pair of cysteine residues. Without being bound by theory, it is believed that the introduction of a pair of cysteine residues capable of forming a disulfide bond provide stability to the heterodimerized bispecific antibody. In embodiments, the first CH3 domain comprises a cysteine at position 354 (EU numbering), and the second CH3 domain that heterodimerizes with the first CH3 domain comprises a cysteine at position 349 (EU numbering).

### C. Bispecific antibody with an extended in vivo half-life.

The present bispecific antibody can be further modified to have an extended half-life *in vivo.*

A variety of strategies can be used to extend the half-life of the bispecific antibody of the present disclosure. For example, by chemical linkage to polyethylene glycol (PEG), reCODE PEG, antibody scaffold, polysialic acid (PSA), hydroxyethyl starch (HES), albumin-binding ligands, and carbohydrate shields; by genetic fusion to proteins binding to serum proteins, such as albumin, IgG, FcRn, and transferring; by coupling (genetically or chemically) to other binding moieties that bind to serum proteins, such as nanobodies, Fabs, DARPins, avimers, affibodies, and anticalins; by genetic fusion to rPEG, albumin, domain of albumin, albumin-binding proteins, and Fc; or by incorporation into nanocarriers, slow release formulations, or medical devices.

A bispecific antibody of the present disclosure having an increased half-life *in vivo* can also be generated introducing one or more amino acid modifications (i.e., substitutions, insertions or deletions) into an IgG constant domain, or FcRn binding fragment thereof (preferably a Fc region or fragment thereof). See, e.g., WO 1998/23289, WO 1997/34631 and US 6,277,375. Preferred modifications to the Fc of the disclosed bispecific antibody include the "LS" mutation (M428L, N434S, (EU numbering)) and the "YTE" mutation (M252Y, S254T, T256E (EU Numbering)) for half-life extension.

Further, the bispecific antibody can be conjugated or fused to one or more human serum albumin (HSA) polypeptides, or a portion thereof. The use of albumin in order to make the molecules more stable in vivo or have a longer half-life in vivo. The techniques are known in the art, see, e.g., WO 1993/15199, WO 1993/15200, and WO 2001/77137; and EP 413622. The use of N-terminal fragments of HSA for fusions to polypeptides has also been proposed (e.g. EP 399666). Accordingly, genetically or chemically fusing or conjugating the molecules to albumin can stabilize or extend the shelf-life, and/or retain the molecule's activity for extended periods of time in solution, in vitro and/or in vivo. Additional methods pertaining to HSA fusions can be found, for example, in WO 2001/077137 and WO 2003/06007. In a specific embodiment, the expression of the fusion protein is performed in mammalian cell lines, for example, CHO cell lines.

### D. Fc silencing

In embodiments of the present disclosure that incorporate one or more constant domains, e.g., heavy chain constant regions, it may be beneficial to include one or mutations to silence, e.g., ADCC and/or CDC effector function within hFc. Activation of the immune cell occurs preferentially in the presence of crosslinking to the target cell. However, human Fc may bind to high and low affinity FcR gamma receptors. Therefore, crosslinking of receptors (e.g., CD3) on the immune cell and subsequent agonism may occur upon binding in the absence of tumor targeting. Additionally, crosslinking of Fc via gamma receptors may induce antibody dependent cellular cytotoxicity (ADCC). Human Fc when complexed at the cell surface can also bind complement proteins and induce complement dependent cytotoxicity (CDC). Mutations to residues in Fc which reduce or abrogate these interactions may thus limit these effects and focus the impact of the molecules described herein upon the tumor target cell.

In embodiments, one or more, e.g., all, of the heavy chain constant region domains of the bispecific antibody thereof comprise the DAPA mutation (e.g., D265A and P329A in EU numbering). See e.g., Shields RL et al., (2001) J Biol Chem., 276(9): 6591-604; US 2015/0320880 A1.

In embodiments, one or more, e.g., all, of the heavy chain constant region domains of the bispecific antibody comprise the LALA mutation (e.g., L234A and L235A in EU numbering). E.g., Hezareh M et al., (2001) Journal of Virology, 75(24): 12161-12168; Shields RL et al., (2001) supra.

In embodiments, one or more, e.g., all, of the heavy chain constant region domains of the bispecific antibody comprise an N279A mutation (according to EU numbering) (see, e.g., Tao MH & Morrison SL (1989) J Immunol. 143(8): 2595-601; Shields RL et al., (2001) supra.

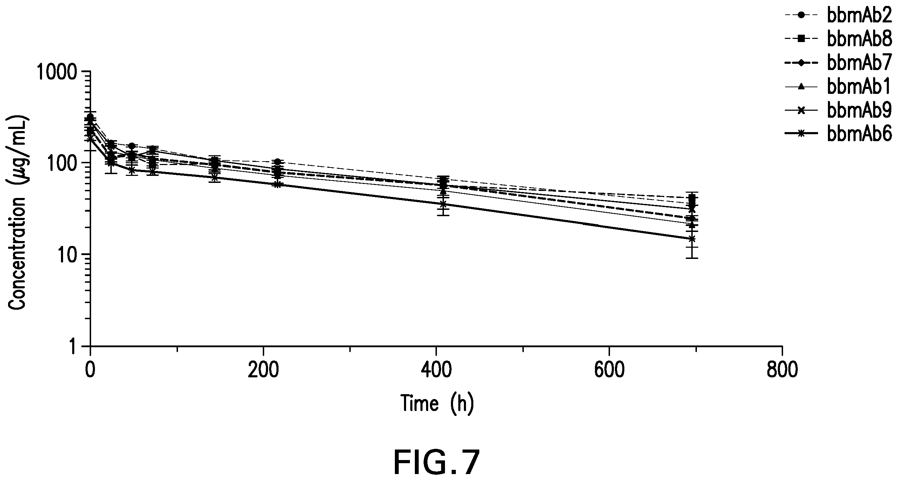

Additional Fc mutations for providing silenced effector function are described in WO2014/145806 (e.g., in Figure 7). One example from WO2014/145806 of a silent IgG1 antibody comprises a E233P, L234V, L235A, and S267K mutation, and a deletion of G236 (G236del). Another example from WO2014/145806 of a silent IgG1 antibody comprises a E233P, L234V, and L235A mutation, and a deletion of G236 (G236del). Another example from WO2014/145806 of a silent IgG1 antibody comprises a S267K mutation.

### E. Conjugates

The present disclosure includes a bispecific antibody recombinantly fused or chemically conjugated (including both covalent and non-covalent conjugations) to a heterologous protein or polypeptide (or fragment thereof, preferably to a polypeptide of at least 10, at least 20, at least 30, at least 40, at least 50, at least 60, at least 70, at least 80, at least 90 or at least 100 amino acids) to generate fusion proteins. Methods for fusing or conjugating proteins, polypeptides, or peptides to an antibody or an antibody fragment are known in the art. See, e.g., US 5,336,603, US 5,622,929, US 5,359,046, US 5,349,053, US 5,447,851, and US 5,112,946; EP 307434 and EP 367166; WO 1996/04388 and WO 1991/06570; Ashkenazi et al., (1991) PNAS. USA 88:10535-10539; Zheng et al., (1995) J. Immunol. 154: 5590-5600; and Vil et al., (1992) PNAS. USA 89:11337- 11341.

Additional fusion proteins may be generated through the techniques of gene-shuffling, motif-shuffling, exon-shuffling, and/or codon-shuffling (collectively referred to as "DNA shuffling"). DNA shuffling may be employed to alter the activities of the bispecific antibody. See, generally, US 5,605,793, US 5,811,238, US 5,830,721, US 5,834,252, and US 5,837,458; Patten et al., (1997) Curr. Opinion Biotechnol. 8:724-33; Harayama (1998) Trends Biotechnol. 16(2):76-82; Hansson et al., (1999) J. Mol. Biol. 287: 265-76; and Lorenzo & Blasco (1998) Biotechniques, 24(2):308- 313. The bispecific antibody may be altered by being subjected to random mutagenesis by error-prone PCR, random nucleotide insertion or other methods prior to recombination. A polynucleotide encoding a fragment of the present molecule may be recombined with one or more components, motifs, sections, parts, domains, fragments, etc. of one or more heterologous molecules.

Moreover, the bispecific antibody can be fused to marker sequences, such as a peptide to facilitate purification. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., (1989) PNAS. USA 86:821-824, for instance, hexa-histidine provides for convenient purification of the fusion protein. Other peptide tags useful for purification include, but are not limited to, the hemagglutinin ("HA") tag, which corresponds to an epitope derived from the influenza hemagglutinin protein (Wilson et al., (1984) Cell 37:767), and the "flag" tag.

In other embodiments, the bispecific antibody is conjugated to a diagnostic or detectable agent. Such molecules can be useful for monitoring or prognosing the onset, development, progression and/or severity of a disease or disorder as part of a clinical testing procedure, such as determining the efficacy of a particular therapy. Such diagnosis and detection can accomplished by coupling the molecules to detectable substances including, but not limited to, various enzymes, such as, but not limited to, horseradish peroxidase, alkaline phosphatase, beta-galactosidase, or acetylcholinesterase; prosthetic groups, such as, but not limited to, streptavidin/biotin and avidin/biotin; fluorescent materials, such as, but not limited to, umbelliferone, fluorescein, fluorescein isothiocyanate, rhodamine, dichlorotriazinylamine fluorescein, dansyl chloride or phycoerythrin; luminescent materials, such as, but not limited to, luminol; bioluminescent materials, such as but not limited to, luciferase, luciferin, and aequorin; radioactive materials, such as, but not limited to, iodine (131I, 125I, 123I, and 121I,), carbon (14C), sulfur (35S), tritium (3H), indium (115In, 113In, 112In, and 111In,), technetium (99Tc), thallium (201Ti), gallium (68Ga, 67Ga), palladium (103Pd), molybdenum (99Mo), xenon (133Xe), fluorine (18F), 153Sm, 177Lu, 159Gd, 149Pm, 140La, 175Yb, 166Ho, 90Y, 47Sc, 186Re, 188Re, 142 Pr, 105Rh, 97Ru, 68Ge, 57Co, 65Zn, 85Sr, 32P, 153Gd, 169Yb, 51Cr, 54Mn, 75Se, 113Sn, and 117Tin; and positron emitting metals using various positron emission tomographies, and nonradioactive paramagnetic metal ions.

Disclosed herein are uses of bispecific antibody conjugated to a therapeutic moiety. The molecules of the present disclosure may be conjugated to a therapeutic moiety such as a cytotoxin, e.g., a cytostatic or cytocidal agent, a therapeutic agent or a radioactive metal ion, e.g., alpha-emitters. A cytotoxin or cytotoxic agent includes any agent that is detrimental to cells.

Further, the bispecific antibody may be conjugated to a therapeutic moiety or drug moiety that modifies a given biological response. Therapeutic moieties or drug moieties are not to be construed as limited to classical chemical therapeutic agents. For example, the drug moiety may be a protein, peptide, or polypeptide possessing a desired biological activity. Such proteins may include, for example, a toxin such as abrin, ricin A, pseudomonas exotoxin, cholera toxin, or diphtheria toxin; a protein such as tumor necrosis factor, α-interferon, β-interferon, nerve growth factor, platelet derived growth factor, tissue plasminogen activator, an apoptotic agent, an anti-angiogenic agent; or, a biological response modifier such as, for example, a lymphokine.

For further discussion of types of cytotoxins, linkers and methods for conjugating therapeutic agents to the molecules, see also Saito et al., (2003) Adv. Drug Deliv. Rev. 55:199-215; Trail et al., (2003) Cancer Immunol. Immunother. 52: 328-337; Payne (2003) Cancer Cell 3: 207-212; Allen (2002) Nat. Rev. Cancer, 2:750-763; Pastan and Kreitman (2002) Curr. Opin. Investig. Drugs, 3: 1089-1091; Senter & Springer (2001) Adv. Drug Deliv. Rev. 53: 247-264.

The bispecific antibody also can be conjugated to a radioactive isotope to generate cytotoxic radiopharmaceuticals, also referred to as radioimmunoconjugates. Examples of radioactive isotopes that can be conjugated to molecules for use diagnostically or therapeutically include, but are not limited to, iodinel31, indium111, yttrium90, and lutetium177. Method for preparing radioimmunconjugates are established in the art. See, e.g., Denardo et al., (1998) Clin Cancer Res. 4(10): 2483-90; Peterson et al., (1999) Bioconjug. Chem. 10(4):553-7; and Zimmerman et al., (1999) Nucl. Med. Biol. 26(8): 943-50.

Techniques for conjugating therapeutic moieties to antibodies are known, see, e.g., Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies 84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., (1982) Immunol. Rev. 62:119-58.

The bispecific antibody may also be attached to solid supports, which are particularly useful for immunoassays or purification of the target antigen. Such solid supports include, but are not limited to, glass, cellulose, polyacrylamide, nylon, polystyrene, polyvinyl chloride or polypropylene.

### VII. Methods of producing the antibodies of the present invention

Where polypeptides of the bispecific antibody of the present disclosure are cross-linked, these functional linkages can be accomplished using methods known in the art. A variety of coupling or cross-linking agents can be used for covalent conjugation. Examples of cross-linking agents include protein A, carbodiimide, N-succinimidyl-S-acetyl-thioacetate (SATA), 5,5'-dithiobis(2-nitrobenzoic acid) (DTNB), o-phenylenedimaleimide (oPDM), N-succinimidyl-3-(2-pyridyldithio)propionate (SPDP), and sulfosuccinimidyl 4-(N-maleimidomethyl) cyclohexane-I-carboxylate (sulfo-SMCC) (see e.g., Karpovsky et al., (1984) J. Exp. Med. 160: 1686; Liu et al. (1985) PNAS. USA 82:8648). Other methods include those described in Paulus (1985) Behring Ins. Mitt. No. 78: 118-132; Brennan et al., (1985) Science 229: 81-83), and Glennie et al., (1987) J. Immunol. 139: 2367-2375). Conjugating agents are SATA and sulfo-SMCC, both available from Pierce Chemical Co. (Rockford, IL).

Alternatively, the present bispecific antibody can be generated recombinantly by introducing DNA constructs encoding the desired molecules into expression vectors and expressing and assembling the desired molecules in the same host cells.

### A. Preparing polypeptide chains

Polypeptides and antibodies and fragments thereof (e.g., half antibodies) can be produced by a variety of techniques, including conventional monoclonal antibody methodology e.g., the standard somatic cell hybridization technique of Kohler and Milstein, (1975) Nature 256: 495. Many techniques for producing monoclonal antibody can be employed e.g., viral or oncogenic transformation of B lymphocytes.

An animal system for preparing hybridomas is the murine system. Hybridoma production in the mouse is a well-established procedure. Immunization protocols and techniques for isolation of immunized splenocytes for fusion are known in the art. Fusion partners (e.g., murine myeloma cells) and fusion procedures are also known.

Chimeric or humanized antibodies used in the present disclosure can be prepared based on the sequence of a murine monoclonal antibody prepared as described above. DNA encoding the heavy and light chain immunoglobulins can be obtained from the murine hybridoma of interest and engineered to contain non-murine (e.g., human) immunoglobulin sequences using standard molecular biology techniques. For example, to create a chimeric antibody, the murine variable regions can be linked to human constant regions using methods known in the art (see e.g., US 4,816,567 to Cabilly et al.). To create a humanized antibody, the murine CDR regions can be inserted into a human framework using methods known in the art. See e.g., US 5,225,539 to Winter, and US 5,530,101; US 5,585,089; US 5,693,762 and US 6,180,370 to Queen et al.

In a certain embodiment, the antibody molecules of the disclosure are human monoclonal antibodies. Such human monoclonal antibodies can be generated using transgenic or transchromosomic mice carrying parts of the human immune system rather than the mouse system. These transgenic and transchromosomic mice include mice referred to herein as HUmAb mice and KM mice, respectively, and are collectively referred to herein as "human Ig mice."

The HUmAb mouse (Medarex, Inc.) contains human immunoglobulin gene miniloci that encode un-rearranged human heavy (µ and γ) and κ light chain immunoglobulin sequences, together with targeted mutations that inactivate the endogenous µ and κ chain loci (see e.g., Lonberg, et al., (1994) Nature 368(6474): 856-859). Accordingly, the mice exhibit reduced expression of mouse IgM or κ, and in response to immunization, the introduced human heavy and light chain transgenes undergo class switching and somatic mutation to generate high affinity human IgGκ monoclonal (Lonberg et al., (1994) supra; reviewed in Lonberg, (1994) Handbook of Experimental Pharmacology 113:49-101; Lonberg and Huszar, (1995) Intern. Rev. Immunol.13: 65-93, and Harding and Lonberg, (1995) Ann. N. Y. Acad. Sci. 764:536-546). The preparation and use of HUmAb mice, and the genomic modifications carried by such mice, is further described in Taylor et al., (1992) Nucleic Acids Research 20:6287-6295; Chen et al., (1993) International Immunology 5: 647-656; Tuaillon et al., (1993) PNAS USA 94:3720-3724; Choi et al., (1993) Nature Genetics 4:117-123; Chen et al., (1993) EMBO J. 12:821-830; Tuaillon et al., (1994) J. Immunol. 152:2912-2920; Taylor et al., (1994) International Immunology 579-591; and Fishwild et al., (1996) Nature Biotechnology 14: 845-851. See further, US 5,545,806; US 5,569,825; US 5,625,126; US 5,633,425; US 5,789,650; US 5,877,397; US 5,661,016; US 5,814,318; US 5,874,299; and US 5,770,429; all to Lonberg and Kay; US 5,545,807 to Surani et al.; WO 1992/103918, WO 1993/12227, WO 1994/25585, WO 1997113852, WO 1998/24884 and WO 1999/45962, all to Lonberg and Kay; and WO 2001/14424 to Korman et al.

In another embodiment, human antibodies used in the present disclosure can be raised using a mouse that carries human immunoglobulin sequences on transgenes and transchomosomes such as a mouse that carries a human heavy chain transgene and a human light chain transchromosome. Such mice, referred to herein as "KM mice", are described in detail in WO 2002/43478 to Ishida et al.

Still further, alternative transgenic animal systems expressing human immunoglobulin genes are available in the art and can be used to raise human antibodies used in the present disclosure. For example, an alternative transgenic system referred to as the Xenomouse (Abgenix, Inc.) can be used. Such mice are described in, e.g., US 5,939,598; US 6,075,181; US 6,114,598; US 6,150,584 and US 6,162,963 to Kucherlapati et al.

Moreover, alternative transchromosomic animal systems expressing human immunoglobulin genes are available in the art and can be used to raise the human antibodies used in the disclosure. For example, mice carrying both a human heavy chain transchromosome and a human light chain tranchromosome, referred to as "TC mice" can be used; such mice are described in Tomizuka et al., (2000) PNAS USA 97:722-727. Furthermore, cows carrying human heavy and light chain transchromosomes have been described in the art (Kuroiwa et al., (2002) Nature Biotechnology 20:889-894) and can be used to raise human antibodies used herein.

Human monoclonal antibodies can also be prepared using phage display methods for screening libraries of human immunoglobulin genes. Such phage display methods for isolating human antibodies are established in the art or described in the examples below. See for example: US 5,223,409; US 5,403,484; and US 5,571,698 to Ladner et al.; US 5,427,908 and US 5,580,717 to Dower et al.; US 5,969,108 and US 6,172,197 to McCafferty et al.; and US 5,885,793; US 6,521,404; US 6,544,731; US 6,555,313; US 6,582,915 and US 6,593,081 to Griffiths et al.

Human monoclonal antibodies used in the disclosure can also be prepared using SCID mice into which human immune cells have been reconstituted such that a human antibody response can be generated upon immunization. Such mice are described in, for example, US 5,476,996 and US 5,698,767 to Wilson et al.

Methods of making bispecific antibodies are known in the art and discussed herein.

### B. Methods of producing recombinant molecules

In one embodiment, disclosed herein is a method of producing the one or more main polypeptide chains of the bispecific antibody recombinantly, comprising: 1) producing one or more DNA constructs comprising a nucleic acid molecule encoding each of the polypeptide chains of the bi-specific binding molecule; 2) introducing said DNA construct(s) into one or more expression vectors; 3) co-transfecting said expression vector(s) in one or more host cells; and 4) expressing and assembling the molecule in a host cell or in solution.

In this respect, the disclosure provides isolated nucleic acids, e.g., one or more polynucleotides, encoding the bispecific antibody described herein, a bi-specific binding molecule that includes an IL-13 binding domain and an IL-18 binding domain, e.g., as described herein. In embodiments, the isolated nucleic acid is disposed on a single continuous polynucleotide. In other embodiments, the isolated polynucleotide is disposed on two or more continuous nucleic acid sequences.

In aspects, the isolated nucleic acid includes a sequence encoding an IL-13 binding domain and a sequence encoding an IL-18 binding domain. In aspects, the sequence encoding the IL-13 binding domain and the sequence encoding the IL-18 binding domain are disposed on separate polynucleotides, which is also referred to as a "set of nucleic acid molecules."

In aspects, the sequence encoding the IL-13 binding domain and the sequence encoding the IL-18 binding domain are disposed on a single polynucleotide.

In an exemplary embodiment, a DNA sequence encoding the light chain of an antibody and a DNA sequence encoding the heavy chain of the first half antibody are placed in separate expression vectors. The expression vectors are then co-transfected into a host cell at a ratio giving rise to optimal assembly. The encoded heavy chains and light chains are expressed in the host cell and assemble into functional molecules.

In another exemplary embodiment, a DNA sequence encoding a light chain of an antibody and a DNA sequence encoding the heavy chain of the first half antibody are placed in one expression vector. The expression vector may then be transfected into a host cell. The encoded heavy chains and light chains are expressed in the host cell and assemble into functional molecules.

Provided herein are cloning and expression vectors comprising one or more nucleic acid molecules or a set of nucleic acid molecules that encode a bispecific antibody as described herein, wherein the vector is suitable for the recombinant production of a bi-specific binding molecule. Provided herein are processes for the production of a bispecific antibody as described herein, comprising culturing a host cell as disclosed herein under conditions sufficient to express the bispecific antibody, and thereafter purifying and recovering the bispecific antibody from the host cell culture.

Desired mutations on the variable region or the constant region of the molecule described herein, such as, for enhancing hetero-dimerization, can be introduced at this stage as described herein.

The DNA sequences can be produced by *de novo* solid-phase DNA synthesis or by PCR mutagenesis of an existing sequence (e.g., sequences as described in the Examples below) encoding heavy or light chains of the present molecules. Direct chemical synthesis of nucleic acids can be accomplished by methods known in the art, such as the phosphotriester method of Narang et al., (1979) Meth. Enzymol. 68: 90; the phosphodiester method of Brown et al., (1979) Meth. Enzymol. 68:1 09; the diethylphosphoramidite method of Beaucage et al., (1981) Tetra. Lett., 22: 1859; and the solid support method of US 4,458,066. Introducing mutations to a polynucleotide sequence by PCR can be performed as described in, e.g., PCR Technology: Principles and Applications for DNA Amplification, H.A. Erlich (Ed.), Freeman Press, NY, NY, 1992; PCR Protocols: A Guide to Methods and Applications, Innis et al. (Ed.), Academic Press, San Diego, CA, 1990; Mattila et al., (1991) Nucleic Acids Res. 19: 967; and Eckert et al., (1991) PCR Methods and Applications 1: 17.

Also provided in the disclosure are expression vectors and host cells for producing the molecules described above. The term "vector" means any molecule or entity (e.g. nucleic acid, plasmid, bacteriophage or virus) that is suitable for transformation or transfection of a host cell and contains nucleic acid sequences that direct and/or control (in conjunction with the host cell) expression of one or more heterologous coding regions operatively linked thereto. Various expression vectors can be employed to express the polynucleotides encoding chains or binding domains of the molecule. Both viral-based and nonviral expression vectors can be used to produce the antibodies in a mammalian host cell. Nonviral vectors and systems include plasmids, episomal vectors, typically with an expression cassette for expressing a protein or RNA, and human artificial chromosomes (see, e.g., Harrington et al., (1997) Nat Genet 15: 345). For example, nonviral vectors useful for expression of the polynucleotides and polypeptides in mammalian (e.g., human) cells include pThioHis A, B & C, pcDNA3.1/His, pEBVHis A, B & C, (Invitrogen, San Diego, CA), MPSV vectors, and numerous other vectors known in the art for expressing other proteins. Useful viral vectors include vectors based on retroviruses, adenoviruses, adeno associated viruses, herpes viruses, vectors based on SV40, papilloma virus, HBP Epstein Barr virus, vaccinia virus vectors and Semliki Forest virus (SFV). See, Brent et al., (1995) supra; Smith, Annu. Rev. Microbiol. 49: 807; and Rosenfeld et al., (1992) Cell 68: 143.

The choice of expression vector depends on the intended host cells in which the vector is to be expressed. Typically, the expression vectors contain a promoter and other regulatory sequences (e.g., enhancers) that are operably linked to the polynucleotides encoding an antibody chain or fragment. In some embodiments, an inducible promoter is employed to prevent expression of inserted sequences except under inducing conditions. Inducible promoters include, e.g., arabinose, lacZ, metallothionein promoter or a heat shock promoter. Cultures of transformed organisms can be expanded under noninducing conditions without biasing the population for coding sequences whose expression products are better tolerated by the host cells. In addition to promoters, other regulatory elements may also be required or desired for efficient expression of the heavy chains and light chains of the bispecific antibody. These elements typically include an ATG initiation codon and adjacent ribosome binding site or other sequences. In addition, the efficiency of expression may be enhanced by the inclusion of enhancers appropriate to the cell system in use (see, e.g., Scharf et al., (1994) Results Probl. Cell Differ. 20: 125; and Bittner et al., (1987) Meth. Enzymol., 153 :516). For example, the SV40 enhancer or CMV enhancer may be used to increase expression in mammalian host cells.

The expression vectors may also provide a secretion signal sequence position to form a fusion protein with polypeptides encoded by inserting the above-described sequences of heavy chain and/or light chain or fragments thereof. More often, the inserted antibody or antibody-like molecule sequences are linked to a signal sequences before inclusion in the vector. Vectors to be used to receive sequences encoding light and heavy chain variable domains sometimes also encode constant regions or parts thereof. Such vectors allow expression of the variable regions as fusion proteins with the constant regions thereby leading to production of intact antibody or fragments thereof. Typically, such constant regions are human.

The host cells for harbouring and expressing the present molecules can be either prokaryotic or eukaryotic. *E. coli* is one prokaryotic host useful for cloning and expressing the polynucleotides of the present disclosure. Other microbial hosts suitable for use include bacilli, such as Bacillus subtilis, and other enterobacteriaceae, such as Salmonella, Serratia, and various Pseudomonas species. In these prokaryotic hosts, one can also make expression vectors, which typically contain expression control sequences compatible with the host cell (e.g., an origin of replication). In addition, any number of a variety of known promoters will be present, such as the lactose promoter system, a tryptophan (trp) promoter system, a beta-lactamase promoter system, or a promoter system from phage lambda. The promoters typically control expression, optionally with an operator sequence, and have ribosome binding site sequences and the like, for initiating and completing transcription and translation. Other microbes, such as yeast, can also be employed to express the antibody of the disclosure. Insect cells in combination with baculovirus vectors can also be used.

In some preferred embodiments, mammalian host cells are used to express and produce the bispecific antibody of the present disclosure. For example, they can be either a hybridoma cell line expressing endogenous immunoglobulin genes (e.g., the 1D6.C9 myeloma hybridoma clone) or a mammalian cell line harbouring an exogenous expression vector (e.g., the SP2/0 myeloma cells). These include any normal mortal or normal or abnormal immortal animal or human cell. For example, a number of suitable host cell lines capable of secreting intact immunoglobulins have been developed including the CHO cell lines, various Cos cell lines, HeLa cells, myeloma cell lines, transformed B-cells and hybridomas. The use of mammalian tissue cell culture to express polypeptides is discussed generally in, e.g., Winnacker, FROM GENES TO CLONES, VCH Publishers, N.Y. 1987. Expression vectors for mammalian host cells can include expression control sequences, such as an origin of replication, a promoter, and an enhancer (see, e.g., Queen et al., (1986) Immunol. Rev. 89:49-68), and necessary processing information sites, such as ribosome binding sites, RNA splice sites, polyadenylation sites, and transcriptional terminator sequences. These expression vectors usually contain promoters derived from mammalian genes or from mammalian viruses. Suitable promoters may be constitutive, cell type-specific, stage-specific, and/or modulatable or regulatable. Useful promoters include, but are not limited to, the metallothionein promoter, the constitutive adenovirus major late promoter, the dexamethasone-inducible MMTV promoter, the SV40 promoter, the MRP pollll promoter, the constitutive MPSV promoter, the tetracycline-inducible CMV promoter (such as the human immediate-early CMV promoter), the constitutive CMV promoter, and promoter-enhancer combinations known in the art.

Methods for introducing expression vectors containing the polynucleotide sequences of interest vary depending on the type of cellular host. For example, calcium chloride transfection is commonly utilized for prokaryotic cells, whereas calcium phosphate treatment or electroporation may be used for other cellular hosts. (See generally Sambrook, et al., supra). Other methods include, e.g., electroporation, calcium phosphate treatment, liposome-mediated transformation, injection and microinjection, ballistic methods, virosomes, immunoliposomes, polycation:nucleic acid conjugates, naked DNA, artificial virions, fusion to the herpes virus structural protein VP22 (Elliot and O'Hare, (1997) Cell 88:223), agent-enhanced uptake of DNA, and *ex vivo* transduction. For long-term, high-yield production of recombinant proteins, stable expression will often be desired. For example, cell lines which stably express antibody chains or binding fragments can be prepared using expression vectors of the disclosure which contain viral origins of replication or endogenous expression elements and a selectable marker gene. Following the introduction of the vector, cells may be allowed to grow for 1-2 days in an enriched media before they are switched to selective media. The purpose of the selectable marker is to confer resistance to selection, and its presence allows growth of cells which successfully express the introduced sequences in selective media. Resistant, stably transfected cells can be proliferated using tissue culture techniques appropriate to the cell type.

The present antibodies or fragments thereof are typically recovered from the culture medium as a secreted polypeptide, although it also may be recovered from host cell lysate when directly produced without a secretory signal. If the molecule is membrane-bound, it can be released from the membrane using a suitable detergent solution (e.g., Triton-X 100).

When the molecule is produced in a recombinant cell other than one of human origin, it is completely free of proteins or polypeptides of human origin. However, it is necessary to purify the molecule from recombinant cell proteins or polypeptides to obtain preparations that are substantially homogeneous as to heteromultimer. As a first step, the culture medium or lysate is normally centrifuged to remove particulate cell debris. The produced molecules can be conveniently purified by hydroxylapatite chromatography, gel electrophoresis, dialysis, or affinity chromatography, with affinity chromatography being the preferred purification technique. Other techniques for protein purification such as fractionation on an ion-exchange column, ethanol precipitation, reverse phase HPLC, chromatography on silica, chromatography on heparin Sepharose, chromatography on an anion or cation exchange resin (such as a polyaspartic acid column), chromatofocusing, SDS-PAGE, and ammonium sulfate precipitation are also available. (mRNA).

### VIII. Use of the antibodies of the present invention

### A. Diagnostic and general therapeutic use

The antibodies of present disclosure have many diagnostic and therapeutic applications. For instance, they can be used for enzyme immunoassay, with arms binding a specific epitope on an enzyme and other portions of the molecule binding an immobilizing matrix. The enzyme immunoassay using antibody-like molecules is discussed by Nolan et al. (Nolan et al., (1990) Biochem. Biophys. Acta. 1040: 1-11). The bispecific antibodies can also be for use in diagnosis of various diseases, e.g., autoimmune diseases (Songsivilai et al., (1990) Clin. Exp. Immunol. 79: 315). In particular, one antigen binding domain of the molecule can bind a IL-13 or IL-18 in a tissue sample (*in vitro, ex vivo, in vivo*) and the other binding site can bind a detectable marker described herein, for example, a chelator which tightly binds a radionuclide (Le Doussal et al., (1992) Int. J. Cancer Suppl. 7: 58-62 ; Le Doussal et al., (1993) J. Nucl. Med. 34: 1662-1671; Stickney et al., (1995) Cancer Res. 51:6650-6655).

The antibodies of present disclosure can be for use in *in vitro* and *in vivo* diagnostic and therapeutic utilities. For example, these antibodies can be administered to cells in culture, e.g., *in vitro* or *in vivo,* or in a subject, e.g., *in vivo,* to treat, prevent or diagnose a variety of disorders.

In one aspect, the molecules of the disclosure are useful for detecting the presence of IL-13 and/or IL-18 in a biological sample. The term "detecting" as used herein encompasses quantitative or qualitative detection. In certain aspects, a biological sample comprises a cell or tissue. In certain aspects, such tissues include normal and/or cancerous tissues that express IL-13 and/or IL-18 at higher levels relative to other tissues.

In one aspect, the present disclosure provides a method of detecting the presence of IL-13 and/or IL-18 in a biological sample. In certain aspects, the method comprises contacting the biological sample with a bispecific antibody of the disclosure under conditions permissive for binding of the antibody to the antigen, and detecting whether a complex is formed between the antibody and the antigen. The biological sample can include, without limitation, urine or blood samples.

Also included, for reference purposes, is a method of diagnosing a disorder associated with expression of IL-13 and/or IL-18. In certain aspects, the method comprises contacting a test cell with a bispecific antibody of the disclosure; determining the level of expression (either quantitatively or qualitatively) of IL-13 and/or IL-18 in the test cell by detecting binding of the a bispecific molecule of the disclosure; and comparing the level of expression of IL-13 and/or IL-18 in the test cell with the level of expression of IL-13 and/or IL-18 in a control cell (e.g., a normal cell of the same tissue origin as the test cell or a non-virus infected cell), wherein a higher level of presence of IL-13 and/or IL-18 in the test cell as compared to the control cell indicates the presence of a disorder associated with IL-13 and/or IL-18. In certain aspects, the test cell is obtained from an individual suspected of having a pathological disorder mediated by IL-13 and IL-18.

In certain cases, a method of diagnosis or detection, such as those described above, comprises detecting binding of a bi-specific molecule of the disclosure, e.g., using a "FACS" assay.

Certain other methods can be used to detect binding of a bispecific antibody of the disclosure. Such methods include, but are not limited to, antigen-binding assays that are known in the art, such as Western blots, radioimmunoassays, ELISA (enzyme linked immunosorbent assay), "sandwich" immunoassays, immunoprecipitation assays, fluorescent immunoassays, protein A immunoassays, and immunohistochemistry (IHC).

In certain aspects, the bispecific antibody of the disclosure is labelled. Labels include, but are not limited to, labels or moieties that are detected directly (such as fluorescent, chromophoric, electron-dense, chemiluminescent, and radioactive labels), as well as moieties, such as enzymes or ligands, that are detected indirectly, e.g., through an enzymatic reaction or molecular interaction.

### B. Pharmaceutical Compositions and Dosing Aspects

Provided herein are pharmaceutical compositions comprising a bispecific antibody of the present disclosure, which are for use in the methods of the present disclosure in the treatment of atopic dermatitis or related condition, the composition further comprising one or more pharmaceutically acceptable carriers and/or diluents.

The phrase "pharmaceutically acceptable" means approved by a regulatory agency of a federal or a state government, or listed in the U.S. Pharmacopeia or other generally recognized pharmacopeia for use in animals, and more particularly, in humans.

The term "pharmaceutical composition" refers to a mixture of at least one active ingredient (e.g., an antibody or fragment of the disclosure) and at least one pharmaceutically-acceptable excipient, diluent or carrier.

Pharmaceutical compositions of therapeutic and diagnostic agents can be prepared by mixing with physiologically acceptable carriers, excipients, or stabilizers in the form of, e.g., lyophilized powders, slurries, aqueous solutions, lotions, or suspensions (see, e.g., Hardman, et al. (2001) Goodman and Gilman's The Pharmacological Basis of Therapeutics, McGraw-Hill, New York, N.Y.; Gennaro (2000) Remington: The Science and Practice of Pharmacy, Lippincott, Williams, and Wilkins, New York, N.Y.; Avis, et al. (eds.) (1993) Pharmaceutical Dosage Forms: eral Medications, Marcel Dekker, NY; Lieberman, et al. (eds.) (1990) Pharmaceutical Dosage Forms: Tablets, Marcel Dekker, NY; Lieberman, et al. (eds.) (1990) Pharmaceutical Dosage Forms: Disperse Systems, Marcel Dekker, NY; Weiner and Kotkoskie (2000) Excipient Toxicity and Safety, Ma Selecting an administration regimen for a therapeutic depends on several factors, including the serum or tissue turnover rate of the entity, the level of symptoms, the immunogenicity of the entity, and the accessibility of the target cells in the biological matrix. In certain embodiments, an administration regimen maximizes the amount of therapeutic delivered to the patient consistent with an acceptable level of side effects. Accordingly, the amount of biologic delivered depends in part on the particular entity and the severity of the condition being treated. Guidance in selecting appropriate doses of antibodies, cytokines, and small molecules are available (see, e.g., Wawrzynczak (1996) Antibody Therapy, Bios Scientific Pub. Ltd, Oxfordshire, UK; Kresina (ed.) (1991) Monoclonal Antibodies, Cytokines and Arthritis, Marcel Dekker, New York, N.Y.; Bach (ed.) (1993) Monoclonal Antibodies and Peptide Therapy in Autoimmune Diseases, Marcel Dekker, New York, N.Y.; Baert, et al. (2003) New Engl. J. Med. 348:601-608; Milgrom, et al. (1999) New Engl. J. Med. 341:1966-1973; Slamon, et al. (2001) New Engl. J. Med. 344:783-792; Beniaminovitz, et al. (2000) New Engl. J. Med. 342:613-619; Ghosh, et al. (2003) New Engl. J. Med. 348:24-32; Lipsky, et al. (2000) New Engl. J. Med. 343:1594-1602).rcel Dekker, Inc., New York, N.Y.).

Determination of the appropriate dose is made by the clinician, e.g., using parameters or factors known or suspected in the art to affect treatment or predicted to affect treatment. Generally, the dose begins with an amount somewhat less than the optimum dose and it is increased by small increments thereafter until the desired or optimum effect is achieved relative to any negative side effects. Important diagnostic measures include those of symptoms of, e.g., the inflammation or level of inflammatory cytokines produced.

Actual dosage levels of the active ingredients in the pharmaceutical compositions of the present disclosure may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present disclosure employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors known in the medical arts.

Compositions comprising the bispecific antibody of the present disclosure can be provided by continuous infusion, or by doses at intervals of, e.g., one day, one week, or 1-7 times per week. Doses may be provided intravenously, subcutaneously, topically, orally, nasally, rectally, intramuscular, intracerebrally, or by inhalation.

The desired dose of the bispecific antibody of present the disclosure is about the same as for an antibody or polypeptide, on a moles/kg body weight basis. The doses administered to a subject may number at least 1, 2, 3, 4, 5, 6, 7, 8, 9, 10, 11, or 12, or more.

For the bispecific antibody of the present disclosure, the dosage administered to a patient may be about 0.0001 mg/kg to about 100 mg/kg of the patient's body weight, e.g., about 1 mg/kg - about 5 mg/kg, about 5 mg/kg to about 10 mg/kg of the patient's body weight. Unit dose of the bispecific antibody of the present disclosure may be about 0.1 mg to 100 mg, e.g., about 1 mg to 5 mg, about 5 mg - about 10 mg, about 10 mg - about 25 mg, about 25 mg - about 50 mg, about 50 mg - about 100 mg, about 100 mg - about 150 mg.

Where a series of doses are administered, these may, for example, be administered approximately every day, approximately every week, approximately every 2 weeks, approximately every 3 weeks, approximately every 4 weeks (monthly), approximately every 2 months, approximately every 3 months (quarterly), approximately every 6 months. The doses may, for example, continue to be administered until disease progression, adverse event, or other time as determined by the physician. For example, from about two, three, or four, up to about 17 or more fixed doses may be administered.

An effective amount for a particular patient may vary depending on factors such as the condition being treated, the overall health of the patient, the method route and dose of administration and the severity of side effects (see, e.g., Maynard, et al. (1996) A Handbook of SOPs for Good Clinical Practice, Interpharm Press, Boca Raton, Fla.; Dent (2001) Good Laboratory and Good Clinical Practice, Urch Publ., London, UK).

Where necessary, the bispecific antibody of the present disclosure may be incorporated into a composition that includes a solubilizing agent and a local anaesthetic such as lidocaine to ease pain at the site of the injection. In addition, pulmonary administration can also be employed, e.g., by use of an inhaler or nebulizer, and formulation with an aerosolizing agent. See, e.g., US 6,019,968, US 5,985,320, US 5,985,309, US 5,934,272, US 5,874,064, US 5,855,913, US 5,290,540, and US 4,880,078; and WO 1992/19244, WO 1997/32572, WO 1997/44013, WO 1998/31346, and WO 1999/66903.

The bispecific antibody of the present disclosure can also be administered via one or more routes of administration using one or more of a variety of methods known in the art. As will be appreciated by the skilled artisan, the route and/or mode of administration will vary depending upon the desired results. Selected routes of administration for the antibodies include intravenous, intramuscular, intradermal, intraperitoneal, subcutaneous, spinal or other parenteral routes of administration, for example by injection or infusion. Parenteral administration can represent modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. Alternatively, a composition of the present disclosure can be administered via a non-parenteral route, such as a topical, epidermal or mucosal route of administration, for example, intranasally, orally, vaginally, rectally, sublingually or topically.

In one aspect, the bispecific antibody of the present disclosure is administered by infusion. In one aspect, the bispecific antibody of present disclosure is administered subcutaneously. In one aspect, the bispecific antibody of present disclosure is administered intravenously.

The bispecific antibody of the present disclosure may be administered via any of the above routes using, e.g., an injection device, an injection pen, a vial and syringe, pre-filled syringe, autoinjector, an infusion pump, a patch pump, an infusion bag and needle, etc. If the bispecific antibody of the present disclosure is administered in a controlled release or sustained release system, a pump may be used to achieve controlled or sustained release (see Langer, supra; Sefton, 1987, CRC Crit. Ref Biomed. Eng. 14:20; Buchwald et al., 1980, Surgery 88:507; Saudek et al., 1989, N. Engl. J. Med. 321:574). Polymeric materials can be used to achieve controlled or sustained release of the therapies of the disclosure (see e.g., Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York (1984); Ranger and Peppas, 1983, J., Macromol. Sci. Rev. Macromol. Chem. 23:61; see also Levy et al., 1985, Science 228:190; During et al., 1989, Ann. Neurol. 25:351; Howard et al., 1989, J. Neurosurg. 7 1:105); US 5,679,377; US 5,916,597; US 5,912,015; US 5,989,463; US 5,128,326; WO 1999/15154; and WO 1999/20253. Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N-vinyl pyrrolidone), poly(vinyl alcohol), polyacrylamide, poly(ethylene glycol), polylactides (PLA), poly(lactide-co-glycolides) (PLGA), and polyorthoesters. In one embodiment, the polymer used in a sustained release formulation is inert, free of leachable impurities, stable on storage, sterile, and biodegradable. A controlled or sustained release system can be placed in proximity of the prophylactic or therapeutic target, thus requiring only a fraction of the systemic dose (see, e.g., Goodson, in Medical Applications of Controlled Release, supra, vol. 2, pp. 115-138 (1984)).

Controlled release systems are discussed in the review by Langer (1990, Science 249:1527-1533). Any technique known to one of skill in the art can be used to produce sustained release formulations comprising one or more bispecific antibody of the present disclosure. See, e.g., US 4,526,938, WO 1991/05548, WO 1996/20698, Ning et al., 1996, "Intratumoral Radioimmunotheraphy of a Human Colon Cancer Xenograft Using a Sustained-Release Gel," Radiotherapy & Oncology 39:179-189, Song et al., 1995, "Antibody Mediated Lung Targeting of Long-Circulating Emulsions," PDA Journal of Pharmaceutical Science & Technology 50:372-397, Cleek et al., 1997, "Biodegradable Polymeric Carriers for a bFGF Antibody for Cardiovascular Application," Pro. Int'l. Symp. Control. Rel. Bioact. Mater. 24:853-854, and Lam et al., 1997, "Microencapsulation of Recombinant Humanized Monoclonal Antibody for Local Delivery," Proc. Int'l. Symp. Control Rel. Bioact. Mater. 24:759-760.

If the bispecific antibody of the present disclosure is administered topically, they can be formulated in the form of an ointment, cream, transdermal patch, lotion, gel, shampoo, spray, aerosol, solution, emulsion, or other forms known to one of skill in the art. See, e.g., Remington's Pharmaceutical Sciences and Introduction to Pharmaceutical Dosage Forms, 19th ed., Mack Pub. Co., Easton, Pa. (1995). For non-sprayable topical dosage forms, viscous to semi-solid or solid forms comprising a carrier or one or more excipients compatible with topical application and having a dynamic viscosity, in some instances, greater than water are typically employed. Suitable formulations include, without limitation, solutions, suspensions, emulsions, creams, ointments, powders, liniments, salves, and the like, which are, if desired, sterilized or mixed with auxiliary agents (e.g., preservatives, stabilizers, wetting agents, buffers, or salts) for influencing various properties, such as, for example, osmotic pressure. Other suitable topical dosage forms include sprayable aerosol preparations wherein the active ingredient, in some instances, in combination with a solid or liquid inert carrier, is packaged in a mixture with a pressurized volatile (e.g., a gaseous propellant, such as Freon) or in a squeeze bottle. Moisturizers or humectants can also be added to pharmaceutical compositions and dosage forms if desired. Examples of such additional ingredients are known in the art.

If the compositions comprising the bispecific antibody of present disclosure are administered intranasally, they can be formulated in an aerosol form, spray, mist or in the form of drops. In particular, prophylactic or therapeutic agents for use according to the present disclosure can be conveniently delivered in the form of an aerosol spray presentation from pressurized packs or a nebulizer, with the use of a suitable propellant (e.g., dichlorodifluoromethane, trichlorofluoromethane, dichlorotetrafluoroethane, carbon dioxide or other suitable gas). In the case of a pressurized aerosol the dosage unit may be determined by providing a valve to deliver a metered amount. Capsules and cartridges (composed of, e.g., gelatin) for use in an inhaler or insufflator may be formulated containing a powder mix of the compound and a suitable powder base such as lactose or starch.

The bispecific antibody of present disclosure can also be cyclically administered to a patient.

In certain embodiments, the bispecific antibody of the present disclosure can be formulated to ensure proper distribution *in vivo.* For example, the blood-brain barrier (BBB) excludes many highly hydrophilic compounds. To ensure that the therapeutic compounds of the disclosure cross the BBB (if desired), they can be formulated, for example, in liposomes. For methods of manufacturing liposomes, see, e.g., US 4,522,811; US 5,374,548; and US 5,399,331. The liposomes may comprise one or more moieties which are selectively transported into specific cells or organs, thus enhance targeted drug delivery (see, e.g., Ranade VV (1989) J. Clin. Pharmacol. 29:685). Exemplary targeting moieties include folate or biotin (see, e.g., US 5,416,016 to Low et al); mannosides (Umezawa et al., (1988) Biochem. Biophys. Res. Commun. 153:1038); antibodies (P. G. Bloeman et al. (1995) FEBS Lett. 357:140; M. Owais et al. (1995) Antimicrob. Agents Chemother. 39:180); surfactant protein A receptor (Briscoe et al. (1995) Am. J. Physiol. 1233:134); p 120 (Schreier et al (1994) J. Biol. Chem. 269:9090); see also K. Keinanen; M. L. Laukkanen (1994) FEBS Lett. 346:123; J. J. Killion; I. J. Fidler (1994) Immunomethods 4:273.

Provided herein are also protocols for the co-administration or treatment of patients using a pharmaceutical composition comprising a bispecific antibody of present disclosure for use in combination with other therapies or therapeutic agent(s). Methods for co-administration or treatment with an additional therapeutic agent, e.g., a cytokine, steroid, chemotherapeutic agent, antibiotic, or radiation, are known in the art (see, e.g., Hardman, et al. (eds.) (2001) Goodman and Gilman's The Pharmacological Basis of Therapeutics, 10.sup.th ed., McGraw-Hill, New York, N.Y.; Poole and Peterson (eds.) (2001) Pharmacotherapeutics for Advanced Practice: A Practical Approach, Lippincott, Williams & Wilkins, Phila., Pa.; Chabner and Longo (eds.) (2001) Cancer Chemotherapy and Biotherapy, Lippincott, Williams & Wilkins, Phila., Pa.). An effective amount of therapeutic may decrease the symptoms by at least 10%; by at least 20%; at least about 30%; at least 40%, or at least 50%.

In some embodiments, a pharmaceutical composition of the disclosure further comprises one or more additional therapeutic agents.

In addition to the above therapeutic regimens, the patient may be subjected to surgery and other forms of physical therapy.

### C. Therapeutic Application to Pathological Disorders Mediated by IL-13 and IL-18

The bispecific antibodies of the present disclosure can be for use in treating various human diseases, for example, pathological disorders mediated by IL-13 and IL-18, e.g., autoimmune diseases, and inflammatory diseases or conditions that involve IL-13 and/or IL-18 dysregulation (e.g., inappropriate expression, expression levels, signaling, etc.). In one embodiment, the bispecific antibody of present disclosure is for use in the treatment of atopic dermatitis.

In one aspect, bispecific antibody of the present disclosure is for use in treating, reducing the likelihood of or ameliorating a pathological disorder mediated by IL-13 and IL-18. The phrase "pathological disorder mediated by IL-13 and IL-18" encompasses all diseases and medical conditions in which IL-13 and IL-18, whether directly or indirectly, in the disease or medical condition, including the causation, development, progress, persistence or pathology of the disease or condition. Accordingly these terms include conditions associated with or characterized by aberrant IL-13 and IL-18 levels and/or diseases or conditions that can be treated by reducing or suppressing IL-13 and IL-18-induced activity in target cells or tissues. Pathological disorders mediated by IL-13 and IL-18 include autoimmune diseases and/or inflammatory conditions and disorders having an IL-13 and IL-18 component.

In some embodiments, the pathological disorder is associated with inappropriate IL-13 and IL-18 expression. In some embodiments, the pathological disorder is associated with inappropriate IL-13 and IL-18 signalling.

The bispecific antibody of present disclosure, whilst not being limited to, can be used in the treatment, prevention, or amelioration of autoimmune diseases and/or inflammatory conditions and disorders, in particular inflammatory conditions with an etiology including an autoimmune component. In one case, for reference purposes, the disclosure provides methods for treating an autoimmune disease. In one case, for reference purposes, the disclosure provides methods for treating an inflammatory disease or condition. In one aspect, the subject of treatment is a human.

Provided herein are the bispecific antibody of present disclosure and pharmaceutical compositions as described herein, for use in the treatment and/or prevention of a pathological disorder mediated by IL-13 and IL-18. Provided herein, for reference purposes, are uses of a bispecific antibody or a pharmaceutical composition as described herein in the manufacture of a medicament for use in the treatment of a pathological disorder mediated by IL-13 and IL-18. Provided herein, for reference purposes, are methods of treating and/or preventing a pathological disorder mediated by IL-13 and IL-18, comprising administering a therapeutically effective amount of a bispecific antibody or a pharmaceutical composition as described herein, to a subject in need thereof. In some embodiments, the pathological disorder mediated by IL-13 and IL-18 is an autoimmune disease or an inflammatory disorder or condition. In some embodiments, the autoimmune disease or inflammatory disorder or condition is atopic dermatitis.

Provided herein are the bispecific antibody of present disclosure and pharmaceutical compositions as described herein, for use in the treatment and/or prevention of moderate-to-severe AD.

The term "Atopic dermatitis" (AD) or "eczema", as used herein, means an inflammatory skin disease characterized by intense pruritus (e.g., severe itch) and by scaly and dry eczematous lesions. The term "Atopic dermatitis" or "eczema" includes, but is not limited to, AD (eczema) caused by or associated with epidermal barrier dysfunction, allergy (e.g., skin allergy, allergy to certain foods, pollen, mold, dust mite, animals, etc.), radiation exposure, and/or asthma. The present disclosure encompasses methods to treat patients with mild, moderate-to-severe or severe AD. As used herein, "moderate-to-severe AD", is characterized by intensely pruritic, widespread skin lesions that are often complicated by persistent bacterial, viral or fungal infections. Moderate-to-severe AD also includes chronic AD in patients. In many cases, the chronic lesions include thickened plaques of skin, lichenification and fibrous papules. Patients affected by moderate-to-severe AD also, in general, have more than 10% or more that 20% of the body's skin affected, or 10% of skin area in addition to involvement of the eyes, hands and body folds. Patients affected by moderate-to-severe AD also, in general, have (i) an Investigator's Global Assessment (IGA) score of 3 or 4, (ii) an Eczema Area and Severity Index (EASI) score of at least 10, preferably at least 12, and (iii ) itch. Moderate-to-severe AD is also considered to be present in patients who require frequent treatment with topical corticosteroids. A patient may also be said to have moderate-to-severe AD when the patient is resistant or refractory to treatment by either a topical corticosteroid or a calcineurin inhibitor or any other commonly used therapeutic agent known in the art.

Suitably, the uses of the present disclosure comprise administering the bispecific antibody of present disclosure, at a dose sufficient to achieve a therapeutically effective serum level. Suitably, the therapeutically effective serum level of the bispecific antibody is maintained during the treatment course.

As used herein, the terms "therapeutically effective serum level" refers to a serum level of a therapy in a subject (the bispecific antibody) which is sufficient to reduce and/or ameliorate the severity and/or duration of a given condition, disorder, or disease and/or a symptom related thereto. In some aspects, "therapeutically effective serum level" as used herein also refers to the amount of an bispecific antibody in serum of a subject which achieves a specified result, for example, improvements in AD-associated parameters, e.g., a decrease in Investigator's Global Assessment (IGA) score; a decrease from baseline in Dermatology Life Quality Index (DLQI ); a decrease from baseline in a patient global impression of severity (PGIS ); improvement decrease from baseline in a patient global impression of change (PGIC); a decrease in Body Surface Area Involvement of Atopic Dermatitis (BSA) score; a decrease in Eczema Area and Severity Index (EASI) score; a decrease in SCORAD score; and/or a decrease in Pruritus Numeric Rating Scale (NRS) score.

In some aspects, "therapeutically effective serum level" as used herein also refers to the amount of the bispecific antibody in serum of a subject which achieves a specified result, for example, decrease of the expression level of one or more AD-associated biomarker, in particular one or more AD-associated biomarker selected from the list consisting of CCL17/TARC, IgE (e.g., serum IgE), CCL26/eotaxin-3, CCL22/MDC, hsCRP, CD40, IL-13, IL-24, IL-22, IL-18 (e.g., serum IL-18, serum free IL-18 (bioactive)), and IL-18BP (e.g., serum IL-18BP), as compared to the level before treatment with the bispecific antibody.

Suitably, the uses of the present disclosure comprise administering the bispecific antibody once a week, once every two weeks, once every three weeks, once every four weeks, once every eight weeks, or once every 12 weeks. According to certain exemplary embodiments, the uses of the present disclosure comprise administering the bispecific antibody once every 4 weeks.

### D. Combination Therapies

A bispecific antibody thereof of the present disclosure may be for use in combination with other agents and therapies that have use in treating various diseases, disorders and conditions (herein "additional therapeutic agent").

Administered "in combination", in reference to an additional therapeutic agent, means that two (or more) different treatments are delivered to the subject during the course of the subject's affliction with the disorder, e.g., the two or more treatments are delivered after the subject has been diagnosed with the disorder and before the disorder has been cured or eliminated or treatment has ceased for other reasons. In some embodiments, the delivery of one treatment is still occurring when the delivery of the second begins, so that there is overlap in terms of administration. This is sometimes referred to herein as "simultaneous" or "concurrent delivery". In other embodiments, the delivery of one treatment ends before the delivery of the other treatment begins. In some embodiments of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some embodiments, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered.

The term "concurrently" is not limited to the administration of therapies (e.g., prophylactic or therapeutic agents) at exactly the same time, but rather it is meant that a pharmaceutical composition comprising a bispecific antibody of present disclosure is administered to a subject in a sequence and within a time interval such that the molecules of the disclosure can act together with the additional therapeutic agent(s) to provide an increased benefit than if they were administered otherwise. For example, each therapy may be administered to a subject at the same time or sequentially in any order at different points in time; however, if not administered at the same time, they should be administered sufficiently close in time so as to provide the desired therapeutic or prophylactic effect. Each therapy can be administered to a subject separately, in any appropriate form and by any suitable route.

Additional therapeutic agents (e.g., additional prophylactic or therapeutic agents), which can be administered in combination with the molecules disclosed herein may be administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours apart from the molecules or fragments thereof of the disclosure. In other embodiments, two or more additional therapeutic agents are administered to a patient within the same patient visit.

A bispecific antibody of the present disclosure and the additional therapeutic agent(s) can be administered simultaneously, in the same or in separate pharmaceutical composition as the disclosed, or sequentially. For sequential administration, the bispecific antibody of the present disclosure can be administered first, and the additional agent can be administered second, or the order of administration can be reversed. The additional therapeutic agent(s) may be administered to a subject by the same or different routes of administration compared to the disclosed bi-specific binding molecules.

The bispecific antibody of the present disclosure, and/or additional therapeutic agent(s), can be administered during periods of active disorder, or during a period of remission or less active disease. The bispecific antibody of the present disclosure can be administered before the other treatment, concurrently with the treatment, post-treatment, or during remission of the disorder.

The additional therapeutic agent(s) of the combination therapies of the present disclosure can also be cyclically administered. Combination cycling therapy involves the administration of a first therapy (e.g., a first prophylactic or therapeutic agent) for a period of time, followed by the administration of a second therapy (e.g., a second prophylactic or therapeutic agent) for a period of time and repeating this sequential administration, i.e., the cycle, in order to reduce the development of resistance to one of the therapies (e.g., agents) to avoid or reduce the side effects of one of the therapies (e.g., agents), and/or to improve, the efficacy of the therapies.

When administered in combination, the bispecific antibody of the present disclosure and the additional therapeutic agent (e.g., second or third agent), or all, can be administered in an amount or dose that is higher, lower or the same than the amount or dosage of each agent used individually, e.g., as a monotherapy. In certain embodiments, the administered amount or dosage of the bispecific antibody molecule as described herein, the additional agent (e.g., second or third agent), or all, is lower (e.g., at least 20%, at least 30%, at least 40%, or at least 50%) than the amount or dosage of each agent used individually, e.g., as a monotherapy. In other embodiments, the amount or dosage of the bispecific antibody of the present disclosure, the additional agent (e.g., second or third agent), or all, that results in a desired effect (e.g., treatment of an autoimmune disease or an inflammatory disease or condition) is lower (e.g., at least 20%, at least 30%, at least 40%, or at least 50% lower) than the amount or dosage of each agent used individually, e.g., as a monotherapy, required to achieve the same therapeutic effect.

Preferably, the additional therapeutic agent (e.g., second or third agent) is an AD agent, e.g., small molecule, biological therapy, or an agent employing AD modality, e.g., phototherapy, including topical therapy, systemic therapy, phototherapy, and combinations thereof. "AD agent" includes topical therapies in the form of creams, ointments, lotions, gels or sprays (e.g., low-medium potency corticosteroids [Group IV-VII according to WHO guidelines, see Bolognia JL, Jorizzo JL, Schaffer JV. Glucocorticosteroids. Dermatology. 3rd ed. 2012. Ch 125, 2075-88; Ference JD, Last AR. Choosing topical corticosteroids. Am Fam Physician. 2009 Jan 15;79(2):135-40]); over the counter (OTC) emollients, and medical devices or so called barrier creams (such as atopiclair); and lubricants for the treatment of itch and/or pain, e.g. anti-itch lotions containing menthol, pramoxine or anti-histamines; local anesthetics, systemic agents (e.g., biological agents, e.g., IL-4R inhibitors, such as dupilumab; IL-13Ra1 inhibitors, such as ASLAN-004; IL-13Ra2 inhibitors; IL-31 inhibitors, such as nemolizumab; TNF alpha inhibitors, such as adalimumab, infliximab, certolizumab and etanercept, alefacept; IL-1a inhibitors, such as bermekimab (MABp1); IL-23 inhibitors, such as briakinumab, ustekinumab, guselkumab, risankizumab, tildrakizumab; IL-17 inhibitors, such as brodalumab, ixekizumab; CD11a inhibitors, such as efalizumab; IL-22 inhibitors, such as fezalimumab, IL-22 binding proteins; IL-5 inhibitors, such as mepolizumab, benralizumab; a synthetic form of IL-2, such as aldesleukin; recombinant IL-2 approaches targeting the interleukin-2 receptor complex, such as LY3471851; OSMR inhibitors, such as KPL-716; VAP-1 inhibitors; OX-40 inhibitors or OX40L inhibitors such as GBR830, KY1005; IgE inhibitors, such as omalizumab, ligelizumab; TSLP inhibitors, such as tezepelumab; IL-33 inhibitors, such as MEDI3506; IL-36 inhibitors, such as spesolimab, ANB019; B-cell modulating approaches, such as rituximab, ocrelizumab; non-biological immunomodulating treatments, e.g., cyclosporine and other calcineurin inhibitors, JAK inhibitors such as tofacitinib, upadacitinib, abrocitinib, baricitinib; TYK2 inhibitors such as deucravacitinib; methotrexate; PDE4 inhibitors such as apremilast; Siglec inhibitor such as AK-002; S1P agonists or antagonists, such as etrasimod or SCD-044; BTK inhibitors such as TAS-5315, IRAK4 antagonists and CCR4- inhibiting approaches, such as RPT-193; systemic corticosteroids, cyclophosphamide, sulphasalazine, azathioprin, mycophenolate mofetil, dapson, hydroxychloroquine); retinoids (e.g., alitretinoin); leukotriene inhibitors or antileukotrienes, such as montelukast, pranlukast or zafirlukast, as well as 5-LO inhibitors such as zileuton, and LTA4H inhibitors such as acebilustat, intralesional corticosteroid injections; phototherapy (e.g. UVB and UVA high dose). photochemotherapy (e.g. psoralen and UVA (PUVA)); topical calcineurin inhibitors (cyclosporine, tacrolimus, pimecrolimus) or topical PDE4 inhibitors such as crisaborole, difamilast or roflumilast; topical JAK inhibitors such as ruxolitinib, delgocitinib, or topical Vitamin D analogues and topical aryl hydrocarbon receptor (AhR) inhibitors such as benvitimod / tapinarof; topical corticosteroids of high - ultrahigh potency (Group I, II, III as per WHO definition); anti-fungal drugs with known anti-inflammatory properties, e.g., griseofulvin, itraconazole, betamethasone, dexamethasone, INCB018424, triamcinolone, apremilast, turmeric past, glucosamine sulfate, triamcinolone acetonide, sesame oil, betamethasone dipropionate, clobetasol propionate, probiotics (e.g., bifidobacterium animalis subst. lactis HN019, lactobacilli reuteri), omega-3, prednisone, prednisolone, platelet rich plasma, orabase paste, lycopene, topical chamomile, green tea, CO2 laser treatment, allergen specific immunotherapies, polybiotics, photobiomodulation, metronidazole, doxycycline, minocycline, cedar honey, purslane, curcuminoids, alefacept, hexaminolevulinate, hydroxychloroquine, adcortyl, efalizumab, fluocinolone, co-enzyme Q10 mucoadhesive tablets, chamaemelum nobile, sirolimus, tacrolimus, qingxuan decoction, NSAID topical rinse, NSAIDs, quercetin, NAVS naphthalan, valchlor, bupivacaine, oatmeal baths. Suitably, topical AD therapy is an atopic dermatitis prescription therapy including but not limited to topical steroid, e.g., corticosteroid, tacrolimus, cyclophosphamide, azathioprine, methotrexate, mycophenolate mofetil, apremilast, calcineurin inhibitor, e.g., topical calcineurin inhibitor, phosphodiesterase 4 (PDE4) inhibitor, e.g., topical PDE4 inhibitor, e.g. Crisaborole, adrenocorticotropic hormone analogs, dupilumab, etanercept, adalimumab, infliximab, omalizumab, secukinumab.

### E. Kits

The disclosure also encompasses kits for treating a patient having a pathological disorder mediated by IL-13 and IL-18, e.g., an autoimmune disease or an inflammatory disorder or condition. Such kits comprise a therapeutically effective amount of a bispecific antibody of the present disclosure. Additionally, such kits comprise means for administering the bispecific antibody of the present disclosure (e.g., an autoinjector, a syringe and vial, a prefilled syringe, a prefilled pen) and instructions for use. These kits may contain additional therapeutic agents (described infra) for treating having a pathological disorder mediated by IL-13 and IL-18, e.g., an autoimmune disease or an inflammatory disorder or condition. Such kits also comprise instructions for administration of the bispecific antibody of the present disclosure to treat the patient. Such instructions may provide the dose, route of administration, regimen, and total treatment duration for use with the enclosed bispecific antibody of the present disclosure.

The phrase "means for administering" is used to indicate any available implement for systemically administering a drug to a patient, including, but not limited to, a pre-filled syringe, a vial and syringe, an injection pen, an auto-injector, an IV drip and bag, an infusion pump, a patch, an infusion bag and needle, etc. With such items, a patient may self-administer the drug (i.e., administer the drug without the assistance of a physician) or a medical practitioner may administer the drug.

### Examples

The following examples are illustrative and are not to be construed as limiting the scope of the appended claims.

### Example 1: Generation of the IL-13/IL-18 bispecific antibodies in CHO cell line 1. Expression vector construction

The vectors used in the examples consist of following elements: hCMV promoter/enhancer driving expression of the individual genes needed for assembly of the antibody construct, polyadenylation signal (polyA), folic acid receptor (FoIR, FAR), DHFR, puromycin and/or hygromycin gene as selection markers, *E.Coli* origin of replication and the beta-lactamase gene for ampicillin resistance to enable amplification in bacteria. Different plasmid setups were evaluated and more details are provided within the figures.

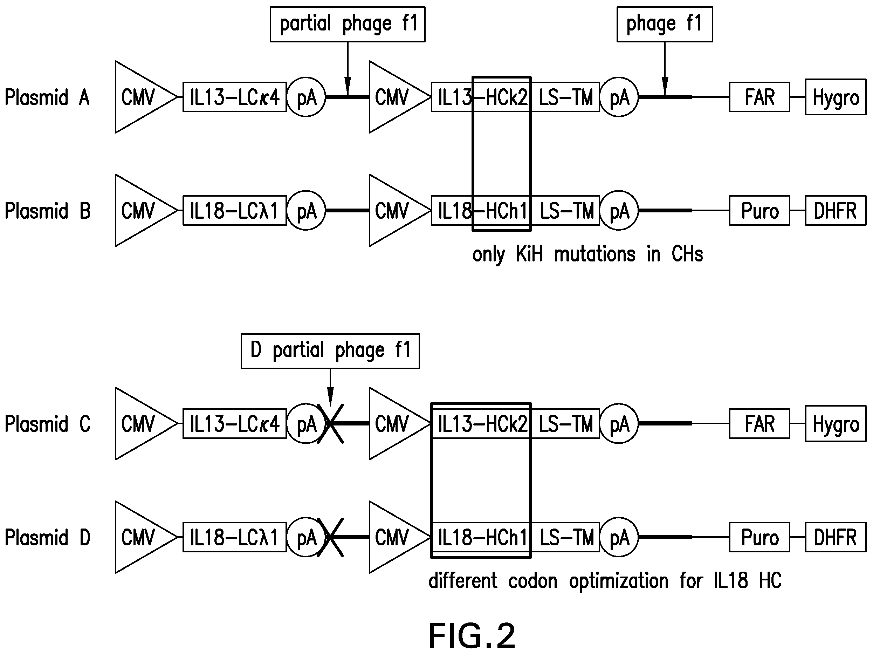

Figure 2 is a schematic representation of NVS standard plasmid A-D. Plasmids A and C are encoding for expression of anti-IL13 kappa LC and anti-L13 knob HC; plasmid B and D are encoding for expression of anti-IL18 lambda LC and anti-IL18 hole HC. Expression of each individual protein chain is driven by a separate CMV promoter. Linearized plasmids A and B or C and D were simultaneously co-transfected in CHO-C8TD parental cells. Cells were selected in a first selection round using selection markers DHFR and FAR. For a second selection, additional selection markers hygromycin and puromycin can be used. Plasmids carry information of the leaky stop transmembrane technology (LS-TM) to enable staining and enrichment of high producing clones during FACS assisted single cell sorting. Plasmids C and D were designed with a lower sequence homology to minimize the risk of homologous recombination by deleting a repetitive partial phage f1 region between expression cassettes encoding for the LC and HC. Plasmid D carries a different codon optimization and signal peptide for IL18 hole HC whereas on plasmid A and B the DNA sequences of the IL18 knob CH and IL13 hole CH differ only by the bases that encode for the KiH mutations.

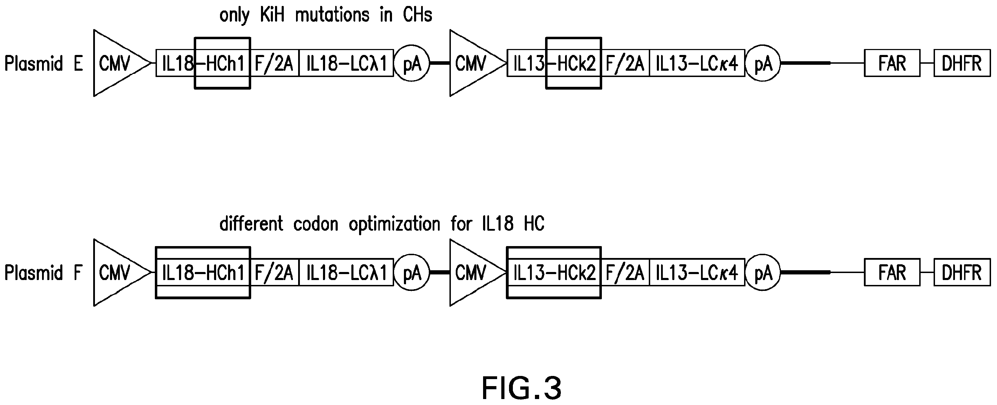

Figure 3 is a schematic representation of NVS Furin-2A peptide (F2A) plasmids E and F. F2A technology enables combined expression of more than one protein chain from one promoter. On plasmids E and F, the first expression cassette is encoding for anti-IL18 lambda LC and anti-IL18 hole HC and the second expression cassette is encoding for anti-IL13 kappa LC and anti-L13 knob HC. Linearized plasmid E or F was transfected in CHO-C8TD parental cells. Cells were selected using selection markers DHFR and FAR. Plasmids F was designed with a lower sequence homology to minimize the risk of homologous recombination by using a different codon optimization for IL18 hole HC and IL13 knob HC whereas on plasmid E the DNA sequences of the IL18 knob CH and IL13 hole CH differ only by the bases that encode for the KiH mutations.

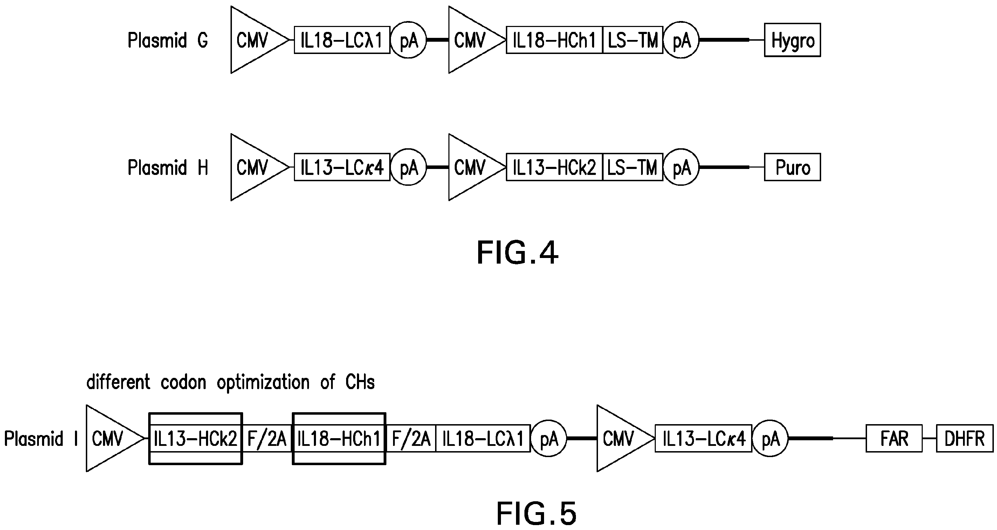

Figure 4 is a schematic representation of adapted NVS standard plasmid G and H. Plasmid G and/or H are used for super transfection of pools expressing plasmids E or F to increase integrated number of plasmids. Plasmid G is encoding for expression of anti-IL13 kappa LC and anti-L13 knob HC; plasmid H is encoding for expression of anti-IL18 lambda LC and anti-IL18 hole HC. Expression of each individual protein chain is driven by a separate CMV promoter. Different approaches of transfection and selection were done, i.e. (I) plasmids G and H were simultaneously co-transfected in CHO-C8TD parental cells and cells selected using selection markers hygromycin and puromycin or (II) plasmid G was transfected in a first round and selected using hygromycin and/or plasmid H transfected in a second round and selected using puromycin or (III) plasmid H transfected in a first round and selected using puromycin and/or plasmid G was transfected in a second round and selected using hygromycin. Plasmids G and H carry information of the leaky stop transmembrane technology (LS-TM) to enable staining and enrichment of high producing clones during FACS assisted single cell sorting. Partial phage f1 region between expression cassettes encoding for the LC and HC was deleted on plasmids G and H and codon optimization of LCs and HCs are different to each other and to plasmids E and F to lower sequence homology to minimize the risk of homologous recombination.

Figure 5 is a schematic representation of NVS Furin-2A peptide (F2A) plasmid I with a different combination of protein chains in the expression cassettes compared to plasmids E and F. On plasmid I the first expression cassette is encoding for anti-IL18 lambda LC, anti-IL18 hole HC and anti-IL13 knob HC and the second expression cassette is encoding for anti-IL13 kappa LC. Linearized plasmid I was transfected in CHO-C8TD parental cells. Cells were selected using selection markers DHFR and FAR. Partial phage f1 region between the two expression cassettes was deleted and different codon optimizations of CHs were used to lower sequence homology to minimize the risk of homologous recombination.

### 2. Cell lines, cultivation, transfection and selection

A parental CHO cell line was used as host cell lines for the production of the antibody constructs. Host cell lines were derived from the CHO-K1 cell line. A single vial from the CHO line was used to prepare the recombinant cell lines. CHO cell lines were cultivated in shake flasks in a non-humidified shaker cabinet at 150 rpm, 10% CO2 at 36.5°C in suspension in proprietary, chemically defined culture media. Cell viabilities and growth rates were monitored by means of an automated system (ViCell, Beckman Coulter). Cells were passaged 2-3 times per week into fresh medium and were maintained in logarithmic growth phase.

Swal linearized expression plasmids encoding the antibody constructs were transfected by electroporation (Amaxa Nucleofection system, Lonza, Germany). The transfection reaction was performed in chemically defined cultivation medium, according to the manufactures instructions. The parental CHO cells used for transfection were in exponential growth phase with cell viabilities higher than 95%. Transfections were performed with 5x 10⁶ cells per transfection. Immediately, after transfection cells were transferred into shake flasks, containing chemically defined cultivation medium. Cell pools were incubated for 48 hours at 36.5°C and 10% CO2 before starting the selection process.

A selection procedure was carried out using the selection markers encoded by the individual expression vectors. First transfection and selection round was performed using folic acid receptor and DHFR. Both proteins are participating in the same molecular pathway; the FolR is transporting folic acid as well as the folate analogue MTX into the cell, the DHFR is converting it into vital precursors for purine and methionine synthesis. Combining them as selective principle, a particular strong selective regime can be taken to enrich for recombinant cells expressing both recombinant protein.

48 h after transfection and growth under low folate conditions, additional selective pressure was applied by adding 10 nM MTX to the chemically defined cultivation medium. After pool recovery cells were frozen in culture medium, supplemented with 7.5% DMSO and material produced for further analysis as described below.

Depending on the plasmid design used for the first transfection and selection round different possibilities for progression emerge. Either pools were directly used for single cell cloning as described below or underwent a second selection or super-transfection before single cell cloning.

For second selection, recovered pools were kept under low folate conditions with 10 nM MTX and additional selective pressure was applied by adding 0.5 ug/ml puromycin and 0.8 mg/ml hygromycin to the chemically defined cultivation medium. After pool recovery cells were frozen in culture medium, supplemented with 7.5% DMSO and material produced for further analysis as described below.

For super transfection Swal linearized expression plasmids encoding the antibody constructs were transfected by electroporation (Amaxa Nucleofection system, Lonza, Germany). The transfection reaction was performed in chemically defined cultivation medium, according to the manufactures instructions. The CHO pools that have recovered after transfection from selection were used for a second or third transfection. Transfections were performed as described above and selection started after 48 hours at 36.5°C and 10% CO2 by adding 0.5 ug/ml puromycin or 0.8 mg/ml hygromycin or both to the chemically defined cultivation medium. After pool recovery cells were frozen in culture medium, supplemented with 7.5% DMSO and material produced for further analysis as described below.

### 3. Single Cell Cloning

After selection, single cell cloning was performed either using the Cytena cell printer device or by flow cytometry to derive cell lines of monoclonal origin.

The Cytena cell printer contains a single-use dispensing cartridge containing a microfluidic chip, into which the cell suspension is loaded. From this cartridge, droplets are ejected through a nozzle into a 96-well plate. During this process, images of the nozzle region are recorded by a microscopic system. An automated image analysis algorithm detects the cells on the image and classifies them along morphological criteria like size and roundness. Based on the image analysis of the droplet-forming region at the nozzle outlet, droplets containing single cells are directed into separate wells of the 96-well plate, whereas droplets that do not contain single cells (void droplets or droplets with multiple cells) are directed into the waste.

Prior to flow cytometry, cells were stained with FITC-labeled in-house produced BD Ab, directed against the Fc part of cell-surface attached ABC123, to facilitate the selection of high producer clones.

Single cell cloning was performed with a Sony Cell Sorter instrument equipped with a 96-well plate holder, using a 100 µm disposable sorting chip. To assure that only single cells are sorted, settings were adjusted to single cell mode, 3-droplet sorting. With these settings, cell-containing droplets are only sorted if the previous and subsequent droplets are both empty. Cell concentration and flow rate were optimized at the expense of yield to increase the probability that each droplet contains not more than one single cell. Multiple gates were set to select single live cells with high fluorescence.

After single cell sorting using Cytena cell printer or Sony Cell Sorter into a separate well of a 96-well plate, a high resolution microscopy image of each well was acquired to document monoclonality and verify the single cell cloning procedure.

After single cell cloning, clones were expanded and characterized with regards to productivity and bioprocess suitability, as well as transgene integration and expression. Primary seed lot (PSL) vials were prepared by freezing cells from top performing clones in medium supplemented with 7.5 % dimethyl sulfoxide (DMSO) and PSLs of final selected clones were used for MCB manufacturing.

### 4. Upstream processing

Subsequent to selection, material was produced in shake flask fed batch cultures. Fed batch cultures were inoculated with a defined cell seeding density, addition of proprietary feed solutions started on day 3 and cultivation temperature shift to 33°C on day 5. During the cultivation in-process controls were performed to monitor the concentration of the antibody construct. The individual culture was cultivated over a period of 14 days. At the end of the cultivation process cells were separated from the culture supernatant by centrifugation followed by sterile filtration before further downstream processing and analytical characterization. Volumetric productivities of selected pools were determined by Protein A HPLC in cell culture supernatants to determine all kind of product and related impurities carrying a Fc part or by RP-LC.

### Example 2. LC-MS screening and purity assessment of the IL-13/IL-18 bispecific antibodies

100 ug purified bispecific mAbs were diluted in 20 mM Tris-HCl pH 7.5 to 1 mg/ml and de-glycosylated for 4 h at 37°C using 2 µl PNGaseF enzyme (New England Biolabs). Deglycosylated samples were subjected to LC-MS system using Waters ACQUITY UPLC Class equipped with a PLRP-S RP column (3 µm, 2.1 x 150 mm, 300 Å, Agilent) and a TripleTOF 6600 with Dual spray ion source (Sciex) mass spectrometer. Eluents were A: 0.1 % TFA in water and B: 70% isopropanol, 20% acetonitrile, 10 % water and 0.09 % TFA . The column was set to 60°C. Flowrate was 0.2 ml/min. Proteins were eluted with a 40 min gradient as follows: 0-4 min 35% B, 4-28 min 35-50 % B, 28-29 min 50-80% B, 29-34 min 80%B, 34-35 min 80-35%, 35-40 min 35 % B. UV chromatograms were recorded at 214 nm and MS data acquisition was performed in positive ES(+). Data were acquired with the Analyst software TF 1.7 (ABSciex) and analysed using the BioPharmaView (version 3.0, ABSciex) and PeakView (version 2.2, ABSciex) software. ). Identification and relative quantification of bbmAb species and misspaired variants is based on the match to the theoretically expected mass and the relative mass signal intensity of the de-convoluted mass spectrum, and the results are shown in Table 3.

A wide range of correct heavy chain heterodimerization, heavy chain homodimerization and half molecules were detected. The desired degree of heterodimerzation was high at > 95% and ideally close to 100 %. In order to identify a candidate with > 95 % heterodimerization, bbmAb1, bbmAb2, bbmAb5, bbmAb4 and bbmAb3 were generated. Only bbmAb1, bbmAb2 and bbmAb5 showed > 95 % heterodimerization, while bbmAb4 and bbmAb3 showed < 95 % heterodimerization and did not qualify for therapeutic development.

**Table 3. Mispairing detected by LC-MS analysis**

| **Candidate** | **Mispairing by intact LC-MS analysis** | | | | |
|---|---|---|---|---|---|
| | Isoforms with expected mass (%) | Mispaired HkHhLhLh (%) | Mispaired HkHhLkLk (%) | Homodimers HkHkLkLh HkHkLkLk HkHkLhLh (%) | Homodimers HhHhLhLh HhHhLkLk HhHhLkLh (%) |
| **bbmAb1** | **>99** | <1 | <1 | <1 | <1 |
| **bbmAb2** | **>99** | <1 | <1 | <1 | <1 |
| **bbmAb5** | **>99** | <1 | <1 | <1 | <1 |
| **bbmAb4** | **94** | <1 | <1 | **6** | <1 |
| **bbmAb3** | **72** | <1 | <1 | **28** | <1 |

### Example 3. Thermal stability assessment of the CH2 and Fab domains of the IL-13/IL-18 bispecific antibodies

An antibody's stability greatly influences its performance (i.e. its specificity and affinity). Thus, stability is a major issue for researchers and manufacturers, especially with the increasing use of antibodies in therapeutics, diagnostics and rapid analytical platforms. The key parameters are the thermal stability and melting temperature (Tₘ) of the CH2 and Fab domains. The protein melting temperature (Tₘ) is defined as the temperature at which the protein denatures. Tₘ values and unfolded fraction can predict aggregation rates (Robinson et al., 2018).

The thermal transition midpoint was determined by differential scanning fluorimetry using CFX96 Teal-Time PCR detection system (BioRad). Purified samples were diluted to 0.3 mg/mL in a final volume of 43 µL in 20 mM His/His-HCl, pH 6.0 and mixed with 7 µL SYPRO orange dilution (1.4 µL SYPRO orange stock solution diluted in 1 mL water). The thermocycler start temperature was set to 20 °C, the end temperature to 95°C and the ramping rate of 0.5 °C. Melting curves and thermal melting temperatures were acquired using Bio Rad CFX Manager Software 3.1.

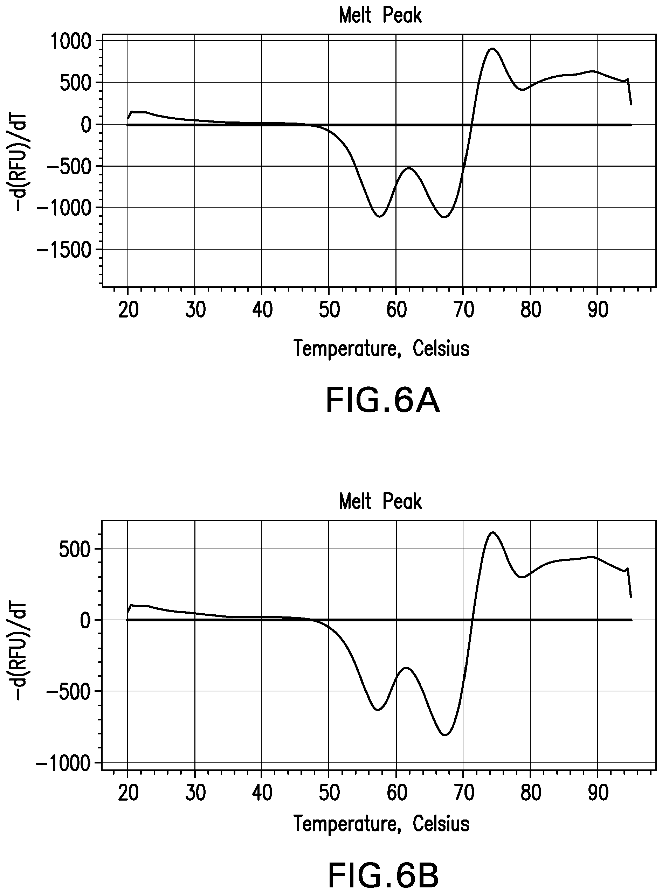

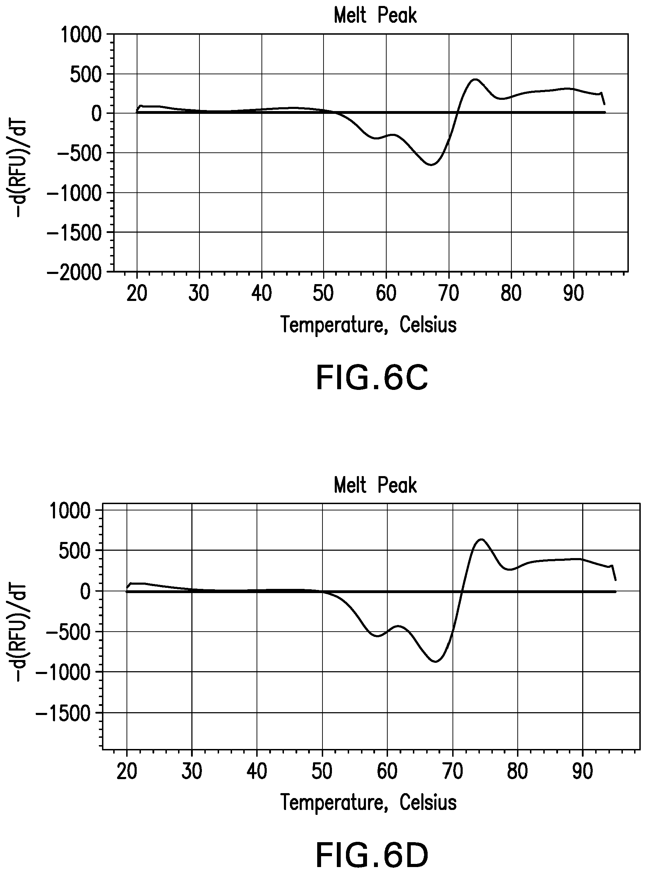

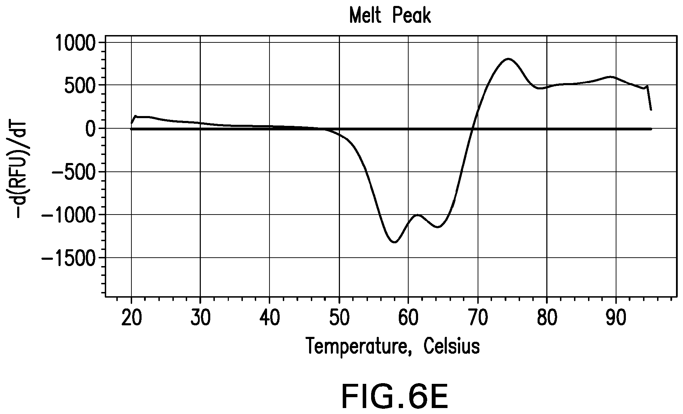

The melting temperatures of the CH2 and Fab domains of the engineered antibodies are shown listed in **Table 4,** and the melting curves of bbmAb1, bbmAb2, bbmAb5 and bbmAb4 are shown in **Figure 6****.**

The Tₘ of CH2 is reflecting the unfolding of the CH2 domain (Johnson, 2012). As shown in **Table 4,** all candidates exhibited similar Tₘ in the Ch2 domain.

Higher melting temperatures were observed in the anti-13 Fab domains of bbmAb1, bbmAb2, bbmAb5 and bbmAb4 (67-68 °C) compared to the anti-13 Fab domain of bbmAb3 (64 °C). Since the CDR sequences in the anti-13 Fab domain of bbmAb3 differ from other candidates, the result indicates that specific CDR sequences in the anti-13 Fab domains of bbmAb1, bbmAb2, bbmAb5 and bbmAb4 can result in a more stable molecule with improved thermal stability.

**Table 4. Melting temperature (Tₘ) of CH2 and Fab domains**

| **Candidate** | **Tₘ CH2 (°C, DSF)** | **Tₘ anti-13 Fab (°C, DSF)** | **Tₘ anti-18 Fab (°C, DSF)** |
|---|---|---|---|
| bbmAb1 | 58 | 68 | 79 |
| bbmAb2 | 58 | 68 | 79 |
| bbmAb5 | 59 | 67 | 79 |
| bbmAb4 | 59 | 68 | 79 |
| bbmAb3 | 58 | 64 | 79 |

### Example 4. Affinities to recombinant human and cynomolgus IL-13 and IL-18 measured by SET

The determination of the equilibrium dissociation constant (KD) was achieved by solution equilibrium titration (SET) measurements described as follows.

22 serial 2n dilutions of the antigens (highest conc.: hsIL-18, 20 nM; cylL-18, 40 nM; hsIL-13, 20 nM) were prepared in sample buffer (PBS containing 0.5 % Bovine serum albumin (BSA) and 0.02 % Tween-20) and a constant concentration of antibody was added (for hslL-13 readout 4 pM, for hslL-18 readout either 10 or 4 pM, and for cylL-18 readout 5 pM).

A volume of 60 µl/well of each antigen-antibody mix was distributed in duplicates to a 384-well polypropylene microtiter plate (MTP). Sample buffer served as negative control and a sample containing only antibody as positive control (maximal electrochemiluminescence signal without antigen, Bₘₐₓ). The plate was sealed and incubated overnight (at least 16 h) at room temperature (RT) on a shaker.

The antigens and antibodies used are listed in **Table 5.**

**Table 5: antigens and antibodies used in the SET measurements**

| **ID** | **Full name** | **Source #Cat-No. or Database-ID** | **Buffer** |
|---|---|---|---|
| hsIL-13 | Recombinant Human IL-13 (115aa) | PeproTech #200-13-10UG | Sodium Phosphate pH 7.0 |
| hslL-13 biotin | Biotinylated Human IL-13 Protein, His,Avitag^{™} | AcroBiosystems # IL3-H82E5, 25 µg | PBS pH 7.4 |
| hsIL-18 | hsIL-18 (aa37-193) | In house, BTP30685 Pool 2 | 25 mM NaHepes pH 7, 2 mM DTT, 1 mM EDTA, |
| | | | adjusted to 2 mM TCEP prior to use |
| cylL-18 | cynolL-18 (aa37-193) | In house, BTP25839 | PBS, adjusted to 2 mM TCEP prior to use |
| hslL-18 biotin | Human IL-18 Protein from SinoBio, in-house biotinylated | SinoBio #10119HNCE | PBS pH 7.4, 2 mM TCEP |
| Sulfo-α-hsIgG | SULFO-tag labeled anti-hslgG | MSD #R32AJ-5 | |
| bbmAb1 | bbmAb1-20299/20300-AFM185-UFT001-W2 | In-house | 10 mM His/His-HCl, pH 5.0 |
| bbmAb2 | bbmAb2-19302APE124-STI006-F1 | In-house | 10 mM His/His-HCl, pH 5.0 |
| bbmAb5 | bbmAb5-20311-iBET-DES Filtrate | In-house | ? |
| 21212400.2 | bbmAb4-20217APE133-UFT001-F1 | In-house | 10 mM L-His, pH 5.0 |

hsIL-18 and cylL-18 readout: After blocking with 50 µl/well blocking buffer (PBS containing 5 % BSA) for at least 1 hour (h) at room temperature (RT) or followed by a washing step (TBST, TBS containing 0.05 % Tween 20), the Streptavidin Multi-Array^{®} 384-Well Plate (MSD L21SA-5) was coated with 30 µl/well biotinylated human IL-18 (0.2 µg/ml, PBS) and incubated for at least 1h at RT on a shaker.

hsIL-13 readout: After blocking with 50 µl/well blocking buffer (PBS containing 5 % BSA) for at least 1 hour (h) at room temperature (RT) followed by a washing step (TBST, TBS containing 0.05 % Tween 20), the Streptavidin Multi-Array^{®} 384-Well Plate (MSD L21SA-5) was coated with 30 µl/well biotinylated human IL-13 (0.2 µg/ml, PBS) and incubated for at least 1h at RT on a shaker.

A volume of 30 µl/well of the equilibrated antigen-antibody mix was transferred from the polypropylene MTP to the coated MSD plate and incubated for 20 min at RT. After an additional wash step, 30 µl sulfo-tag labeled anti-hslgG detection antibody (0.5 µg/ml) diluted in sample buffer were added to each well and incubated for 30 min at RT on a shaker. The MSD plate was washed and 35µl/well MSD read buffer were added and incubated for 5 min at RT. Electrochemiluminescence (ECL) signals were generated and measured by the MSD Sector Imager 6000.

The SET Data were exported to Xlfit, an MS Excel add-in software. Average ECL-signals were calculated from duplicate measurements within each assay. Data were baseline adjusted by subtracting the lowest value from all data points and plotted against the corresponding antigen concentration to generate titration curves. *K_{D}* values were determined by fitting the plot with the following:
1:1 binding model for the knob in hole bispecific Ab $y = {B}_{max} - \left(\frac{{B}_{max}}{2 \left[Fab\right]}\left(\left[Fab\right]+x+{K}_{D}-\sqrt{{\left(\left[Fab\right]+x+{K}_{D}\right)}^{2} - 4 x \left[Fab\right]}\right)\right)$ wherein
y: blank subtracted ECL signal
Bₘₐₓ: maximal ECL signal at zero antigen concentration
[Fab]: applied bispecific antibody concentration
*K_{D}*: Dissociation equilibrium constant
x: applied antigen concentration
The *K_{D}* values obtained are shown in **Table 6.**

**Table 6: Affinities to recombinant human and cynomolgus IL-13 and IL-18 measured by SET (individual target binding determination)**

| | | | | *K_{D}* (pM) |
|---|---|---|---|---|
| Antibody | hulL-13 | cylL-13 | hulL-18 | cyIL-18 |
| bbmAb1 | <1 | n.d.* | 1.8±0.8 | 57.8±15.9 |
| bbmAb2 | n.d.* | n.d.* | 1.4±0.4 | 54.0±18.5 |
| bbmAb5 | <1 | n.d. | 1.4±0.3 | n.d. |
| bbmAb4 | n.d. | n.d. | 1.2±0.8 | n.d. |

| | | | | |
|---|---|---|---|---|
| n.d., not determined, *, determined by SPR (see below), *K_{D}* calculated from 3 or 4 replicates | | | | |

### Example 5. Affinities to recombinant human and cynomolgus IL-13 and IL-18 measured by SPR

The determination of kinetic binding parameters was achieved by surface plasmon resonance (SPR) measurements using the optical biosensor Biacore^{™} T200 (http://www. cytivalifesciences.com).

This technology allows for the label-free determination of the kinetic rate constants for binding (*kₐ*, association rate constant) and dissociation (*k_{d}*, dissociation rate constant) of a ligand to a receptor. The equilibrium dissociation constant *K_{D}* is calculated from the kinetic rate constants.

The surface of a C1 sensor chip (Cytiva #BR100535) was prepared for indirect binding of the antibodies by immobilization of NeutrAvidin^{™} (Thermo Scientific #31000) 80 µg/mL in immobilization buffer (10mM Sodium acetate pH 5.0) on the chip surface via amine coupling, followed by saturation of the NeutrAvidin^{™} with biotinylated Protein G (Sigma #P8045), 5 µg/mL in HBS-EP buffer.

Antibody was diluted into blank buffer HBS-EP (0.01M HEPES pH 7.4, 0.15 M NaCl, 3 mM EDTA, 0.05% v/v Surfactant P20; Cytiva #BR100669) to a final concentration of 10 µg/mL. Affinity measurements for the determination of kinetic constants of bbmAb2 or bbmAb1 were performed for recombinant hulL-13 (2-fold increasing concentrations from 0.25 to 8.0 nM) and recombinant hulL-18 (2-fold increasing concentrations from 0.125 to 32 nM. The surface was regenerated with 10 mM glycine, pH 1.5, 0.5% Tween 20 between cycles.

The antigens and antibodies used are listed in **Table 7.**

**Table 7: antigens and antibodies used in the SET measurements**

| **ID** | **Full name** | **Source #Cat-No. or Database-ID** | **Buffer** |
|---|---|---|---|
| hsIL-13 | Recombinant Human IL-13 (115aa) | PeproTech #200-13-10UG | Sodium Phosphate pH 7.0 |
| hsIL-18 | hsIL-18 (aa37-193) | In house, BTP30685 Pool 2 | 25 mM NaHepes pH 7, 2 mM DTT, 1 mM EDTA |
| cylL-18 | cynolL-18 (aa37-193) | In house, BTP25839 | PBS, adjusted to 2 mM TCEP prior to use |
| bbmAb1 | bbmAb1-19301APE124-CEN005-F1 | In-house | 10 mM His/His-HCl, pH 5.0 |
| bbmAb2 | bbmAb2-19302APE124-STI006-F1 | In-house | 10 mM His/His-HCl, pH 5.0 |

The kinetic traces were evaluated with the Biacore^{™} T200 Control Software v2.0.1. The full set of these traces of increasing concentrations is called a run. The set of traces of a run is fitted with the 1:1 binding model (Rₘₐₓ set global) provided by Biacore T200 Evaluation Software v3.0. A zero concentration sample (blank) was included in each run to allow for double referencing.

The *K_{D}* values obtained are shown in **Table 8.**

**Table 8: Kinetic rate constants and affinities to recombinant human and cynomolgus IL-13 measured by SPR**

| **Antibody** | **Antigen** | ***kₐ* (1/Ms)** | ***k_{d}* (1/s)** | ***K_{D}* (pM)** | **Replicates (n)** |
|---|---|---|---|---|---|
| bbmAb1 | hulL-13 | 1.39 ± 0.37E+6 | 4.57 ± 0.49E-5 | 35.1 ± 13.4 | 3 |
| | cylL-13 | 6.49 ± 1.15E+6 | 8.13 ± 0.98E-5 | 12.9 ± 3.8 | 2 |
| bbmAb2 | hulL-13 | 1.33 ± 0.33E+6 | 2.33 ± 0.66E-5 | 19.2 ± 10.6 | 3 |
| | cylL-13 | 6.34 ± 0.59E+6 | 5.90 ± 0.71E-5 | 9.4 ± 2.0 | 2 |

### Example 6. Affinities to human Fc receptors measured by surface plasmon resonance (SPR) spectroscopy

In order to characterize the binding of the engineered IL-13/IL-18 bispecific antibodies against human Fc receptors, a direct binding assay was performed using surface plasmon resonance (SPR) spectroscopy. SPR is a technology generally applied to affinity and kinetic analysis of protein-protein, protein-peptide, protein-DNA, and protein-small molecule interactions, as it allows the analysis of interactions between analytes in solution and a ligand attached to a sensor chip surface, providing a continuous readout of complex formation and dissociation.

### 1. Measurements on the binding of Fcγ receptors in vitro

The affinity determination of the human Fcy receptors binding to the Fc fragment of bbmAb2 (IL18 knob YTE IL13 hole YTE 1+1 bsAb) was carried out on a Biacore T200 instrument. The affinity determination of the human Fcy receptors binding to the Fc fragments of bbmAb5 (bispecific KiH LALA YTE) and bbmAb1 (IL18 hole YTE (mAb1) IL13 (mAb2) knob YTE 1+1 bsAb) were carried out on a Biacore 8K instrument. The samples were diluted in 10 mM sodium acetate pH 4.5 at 5 µg/ml and immobilized at a density of approximately 750 resonance units on a CM5 sensor chip applying a standard amine coupling procedure on the Biacore T200 instrument. On the Biacore 8K instrument, a similar procedure was applied but the immobilization was at a density of approximately 1310 resonance units. On Biacore T200, flow cell 1 was blank immobilized to serve as a reference. On Biacore 8K, there are in total 8 channels and flow cell 1 of each channel was left blank to serve as a reference surface. The kinetic binding data were collected by subsequent injections of 1:2 dilution series of the human Fcy receptors (CD64/FcyRI, CD32a/FcgγRIIA_{R131}, CD32b/FcyRIIB, CD16a/FcγRIIIA_{V176} and FcγRIIIA_{F176}, CD16b/FcγRIIIB) on all flow cells at a flow rate of 30 µl/min in Biacore T200, or 50 µl/min in Biacore 8K and at a temperature of 25°C. The Fcy receptors were diluted in running buffer (PBS pH 7.4 with 0.005% Tween-20) at different concentration ranges depending of the strength of the interactions (on Biacore T200 we used FcyRI: 0.20 to 100 nM, FcγRIIA_{R131}, FcyRIIB, and FcyRIIIB: 7.81 to 4000 nM, FcγRIIIA_{V176}: 1.95 to 1000 nM, and FcγRIIIA_{F176}: 3.91 to 2000 nM; on Biacore 8K, the same conditions were applied except for FcyRI which was tested from 0.05 to 20nM). On Biacore T200, the chip surface was regenerated using a 10 mM glycine pH 2.0 solution at 30 µl/min for 30 s after each measuring cycle of FcyRI and FcγRIIIA_{V176}. On Biacore 8K, the surfaces were regenerated with one injection of 10mM glycine at pH 2.0 for 30 s for the hFcyRs at a flow rate of 50 µl/min. Zero concentration samples (blank runs) were measured to allow double referencing during data evaluation. Duplicate injections of each sample and buffer blank were flowed over all surfaces. Data were evaluated using the Biacore T200 evaluation software version 3.0 and Biacore 8K evaluation software (v.3.0.12.15655).

The raw data were double referenced, i.e., the response of the measuring flow cell was corrected for the response of the reference flow cell, and in a second step the response of a blank injection was subtracted. The resulting sensorgrams were fitted using either a steady-state model or a 1:1 Langmuir model to calculate the equilibrium dissociation constants (K_{D}).

### 2. Measurements on the binding of FcRn receptor in vitro

The affinity determination of the human FcRn receptor binding to the Fc fragment of bbmAb2 (IL18 (mAb1) knob YTE IL13 (mAb2) hole YTE 1+1 bsAb) was carried out on a Biacore T200 instrument. The affinity determination of the human FcRn receptor binding to the Fc fragments of bbmAb5 (bispecific KiH LALA YTE) and bbmAb1 (IL18 hole YTE (mAb1) IL13 (mAb2) knob YTE 1+1 bsAb) were carried out on a Biacore 8K instrument. The samples were diluted in 10 mM sodium acetate pH 4.5 at 5 µg/ml and immobilized at a density of approximately 750 resonance units on a CM5 sensor chip applying a standard amine coupling procedure on the Biacore T200 instrument. On the Biacore 8K instrument, a similar procedure was applied but the immobilization was at a density of approximately 1310 resonance units. On Biacore T200, flow cell 1 was blank immobilized to serve as a reference. On Biacore 8K, there are in total 8 channels and flow cell 1 of each channel was left blank to serve as a reference surface. The kinetic binding data were collected by subsequent injections of 1:2 dilution series of the human FcRn receptor on all flow cells at a flow rate of 50 µl/min and at a temperature of 25°C. The FcRn receptor was diluted in two different running buffers to check the pH-dependent binding: PBS pH 5.8 with 0.005% Tween-20 and PBS pH 7.4 with 0.005% Tween-20, to cover a concentration range from 4.88 to 2500 nM. The chip surface was regenerated using PBS pH 7.4 with 0.005% Tween-20 for 120s at a flow rate of 50 µl/min. Zero concentration samples (blank runs) were measured to allow double referencing during data evaluation. Duplicate injections of each sample and buffer blank were flowed over all surfaces. Data were evaluated using the Biacore T200 evaluation software version 3.0 and Biacore 8K evaluation software (v.3.0.12.15655).

The raw data were double referenced, i.e., the response of the measuring flow cell was corrected for the response of the reference flow cell, and in a second step the response of a blank injection was subtracted. The resulting sensorgrams were fitted using either a steady-state model or a 1:1 Langmuir model to calculate the equilibrium dissociation constants (K_{D}).

### 3. Measurements on the binding of C1q in vitro

The affinity determination of human C1q binding to bbmAb2 was carried out on a Biacore T200 instrument. The affinity determination of human C1q binding to bbmAb5 and bbmAb1 was carried out on a Biacore 8K instrument. The samples were diluted in 10 mM sodium acetate buffer pH 4.5 at 50 µg/ml and immobilized at a density of approximately 8900 resonance units on a CM5 sensor chip applying a standard amine coupling procedure on the Biacore T200 instrument. On the Biacore 8K instrument, a similar procedure was applied but the immobilization was at a density of approximately 9400 resonance units. On Biacore T200, flow cell 1 was blank immobilized to serve as a reference. On Biacore 8K, there are in total 8 channels and flow cell 1 of each channel was left blank to serve as a reference surface. The kinetic binding data were collected by subsequent injections of 1:2 dilution series of the human C1q on all flow cells at a flow rate of 30 µl/min and a temperature of 25°C. Human C1q was diluted in running buffer (HBS-EP+ pH 7.4) at a concentration range of 0.49 nM - 250 nM. The chip surface was regenerated using a 50 mM NaOH for 30 s at a flow rate of 30 µl/min including a stabilization period of 60s after each measuring cycle. Zero concentration samples (blank runs) were measured to allow double referencing during data evaluation. Duplicate injections of each sample and buffer blank were flowed over all surfaces. Data were evaluated using the Biacore T200 evaluation software version 3.0 and Biacore 8K evaluation software (v.3.0.12.15655).

The raw data were double referenced, i.e., the response of the measuring flow cell was corrected for the response of the reference flow cell, and in a second step the response of a blank injection was subtracted. The resulting sensorgrams were fitted using either a steady-state model to calculate the equilibrium dissociation constants (K_{D}).

### Results showing the binding affinities of Fcγ and FcRn receptors in vitro

The binding affinities of bbmAb1, bbmAb2 and bbmAb5 to different Fc receptors are summarized in the following **Table 9.**

**Table 9: K_{D} values for bbmAb1, bbmAb2 and bbmAb5**

| | **bbmAb2** | | **bbmAb1** | | **bbmAb5** | |
|---|---|---|---|---|---|---|
| | **K_{D} (nM) (m ± σ)** | **n** | **K_{D} (nM) (m ± σ)** | **n** | **K_{D} (nM) (m ± σ)** | **n** |
| **hFcRn pH5.8** | 223±8 | 2 | 176±1 | 2 | 180±0.5 | 2 |
| **hFcRn pH7.4** | Low level binding | 1 | Low level binding | 1 | Low level binding | 1 |
| **hFcγRI** | 3.12±0.08 | 2 | 0.54±0.11 | 2 | > 20* | 2 |
| **hFcγRIIA R131 (low affinity)** | 3629±157* | 2 | 2735±245* | 2 | No binding | 2 |
| **hFcγRIIB** | > 4000* | 2 | > 4000* | 2 | No binding | 2 |
| **hFcγRIIIA V176 (high affinity)** | 328±38 | 2 | 171±10 | 2 | > 1000* | 2 |
| **hFcγRIIIA F176 (low affinity)** | 1287±222* | 2 | 1280±10* | 2 | No binding | 2 |
| **hFcγRIIIB** | > 4000* | 2 | > 4000* | 2 | > 4000* | 2 |

| | | | | | | |
|---|---|---|---|---|---|---|
| Note: KD values marked with * are not reliable as the determined KD is close to or greater than the highest concentration applied. | | | | | | |

### Example 7. PK studies of the IL-13/IL-18 bispecific antibodies

### PK studies conducted in hFcRn transgenic mice

The mice experiments were performed in Tg276 B6.Cg-Fcgrttm1DcrTg(CAG-FCGRT)276Dcr/DcrJ hemizygous mice derived from C57BL/6 mice and purchased from The Jackson Laboratory (USA). FcRn-/- hFcRn (line 276) Tg mice carry a null mutation for the mouse gene and a transgene expressing the hFcRn α-chain under the control of the ubiquitous CAG promoter. All mice were treatment-naive males between the ages of 8 and 12 weeks at study start. For dosing, the antibodies were prepared in phosphate buffer saline (1x PBS pH 7.3) and administered as single intravenous doses of 10 mg/kg into the left lateral vein of the tail with a dose volume of 5 mL/kg. A total of 3 animal replicates were evaluated for each antibody utilizing a serial sampling approach (pre-dose, 1h, 1d, 2d, 3d, 6d, 9d, 17d and 29d) across the study duration of 29 days. Blood samples (30-50 µL) were collected into serum separator tubes and were allowed to clot at room temperature for 20-30 minutes. Samples were then processed to obtain serum by centrifugation (2000g, room temperature, 10 minutes). The resultant serum was stored at -80°C until analysis.

A quantitative sequential electrochemiluminescence immunoassay (ECLIA) was used for the measurement of total antibody in unknown mouse serum specimens, calibration standards (Cs) and quality control samples (QCs). The use of both targets for capturing and detection of the antibody enabled the assay to detect the total amount of the bispecific therapeutic antibodies using their binding sites. Therefore, the antibody was captured by biotinylated human IL-18, which was immobilized on the SA coated and blocked MSD plate. Ruthenium (II) tris-bipyridine-(4-methylsulfonate) NHS ester (MSD Sulfo-TAG^{™}) labeled cynomolgus monkey IL-13 was added and bound for detection, which was based on electrochemiluminescence (ECL) and the read-out was performed on an ECL sector imager from MSD. The obtained ECL value is proportional to the amount of drug present in the initial sample as determined through a 5PL regression analysis with a weighting factor of 1/Y2, with Y denoting the ECL value of the corresponding calibration standard.

bbmAb1 and bbmAb2 as well as the Fc-silenced variants bbmAb6, bbmAb7, bbmAb8 and bbmAb9 were investigated using the experimental approach described above in the humanized mouse model. bbmAb7 and bbmAb8 are Fc-silencing variants of bbmAb1 which comprise the L235C mutation (EU numbering). bbmAb6 is an Fc-silencing variant of bbmAb1 which comprises the L235C mutation and does not comprise the YTE mutations (EU numbering). bbmAb9 is an Fc-silencing variant of bbmAb1 which comprises the L235C/G236C mutations (EU numbering).

All above-mentioned antibodies showed good exposure typical for a half-life extended antibody with a high Cₘₐₓ after intravenous administration and the typical shape of the PK curve in the systemic circulation with a short distribution phase and a prolonged elimination phase. Half-lives of the antibodies investigated here are very similar and could be estimated from the terminal phase of the PK curve in the range of approximately 10 to 16 days (as shown in Figure 7).

### Example 7 Simultaneous Inhibition of IL-13/IL-18

Human peripheral blood mononuclear cells (pBMCs) or human keratinocytes were treated with IL-18 or IL-13, respectively and analyzed for differences in gene expression. Very little overlap in between IL-13 and IL-18 induced gene signatures was observed (data not shown) while they were both shown to be increased in a published lesional over non-lesional skin from AD patients gene expression dataset (He et al. 2020: skin biopsies from 5 AD (lesional and non-lesional) and 7 HV subjects; scRNA-seq data).

Mining a dataset where lesional and non-lesional skin biopsy samples had been collected from AD patients treated with an antibody that antagonizes IL-4 receptor alpha (anti-IL-4Rα), thus blocking both IL-13 and IL-4 signalling, revealed that IL-13 induced gene signatures were significantly downregulated by anti-IL-4Rα treatment; however, IL-18 induced gene signatures were not significantly downregulated by anti-IL-4Rα treatment (data not shown).

These results suggest that dual inhibition of both IL-13 and IL-18 could improve treatment outcomes as compared to inhibition of IL-13 or IL-18 alone

4 mm skin biopsies from 10 atopic dermatitis patients were harvested. 4 mm skin biopsies from 8 healthy volunteers were harvested as control samples. Each biopsy was cut into 4 pieces and cultured ex vivo for 24 h in 100 µl of medium either +/- α-IL-18, α-IL-13 or both (each at 150 µg/ mL). Cell culture supernatant was centrifuged at low speed to remove cells without lysing them. Changes in protein expression were evaluated using an Olink assay. Decreases in protein expression in the supernatant of lesional biopsy samples compared to control samples showed a measurable decrease in anti-IL-13 treated samples as compared to control, a decrease in anti-IL-18 treated samples as compared to control, and a further decrease in anti-IL-18/anti-IL-13 treated samples as compared to control and as compared to the singly treated samples (data not shown

### Example 8 Simultaneous Inhibition of IL-13/IL-18 with a Bispecific Antibody Demonstrates Synergistic Suppression of AD-like Transcriptome

### Methods

8 mm full thickness skin biopsies from surgical discard from 5 individual donors were obtained and cultured in IMDM medium with 1% Pen/Strep and 10% knockout serum replacement in tissue culture inserts for 12-well plates. Biopsies were injected with either control (30 µL PBS) or were activated with 30 µL of a mix of anti-CD28 and anti-CD3 antibodies in PBS at a final concentration of 500 ng each at day 0. To induce differentiation to an AD-like transcriptome, biopsies (except control) were incubated with a mix of the following cytokines, each cytokine at 50 ng/mL: IL-4, IL-13, IL-33, TSLP, IL18, and IL-31, in IMDM medium for 6 days with medium changes on day 2, 4, and 5. During the 6 day induction period, induced biopsy samples were treated with 1µM IgG1 isotype control antibody having a LALA silencing mutation (AD+isotype); 1µM anti-IL13 antibody; 1µM anti-IL18 antibody, or 1µM anti-IL13/18 bispecific antibody bbmAb1. On day 6, supernatant was collected, and the biopsy was halved for histological and transcriptome analysis (Ampliseq whole transcriptome protocol).

Ampliseq normalized values were imported into Qlucore Omics Explorer 3.8 and variables with values less than 0.5 if true in 90% of the samples were removed resulting in 16719 variables. A threshold was set to 0.25 and values were log2 transformed. A 'disease transcriptome was generated comparing "AD isotype" samples to control with q<0.1 and FC>2 on donor corrected samples, resulting in 1485 differentially expressed 'disease genes'. 507 of which were upregulated in the AD isotype samples. Gene Set Variation Analysis (GSVA) was performed in unimodal mode on this set of genes.

### Results

As shown in Figure 8, GSVA showed ineffective inhibition for all anti-IL18 treated samples. Anti-IL13 inhibition was partially effective in 2 out of 5 samples. In contrast, the anti-IL18/IL13 bispecific antibody bbmAb1 showed solid suppression of the AD-like disease transcriptome in 4 out of 5 samples, indicating synergistic effects for combined cytokine blockade.

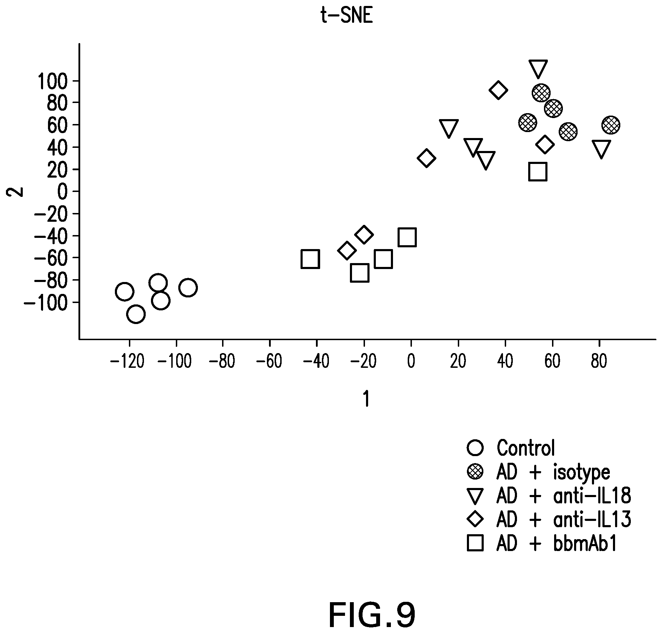

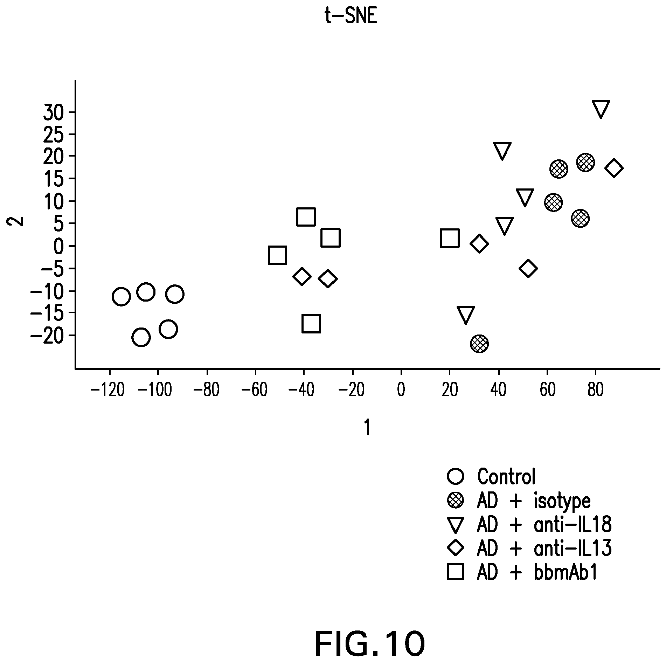

As shown in Figure 9, t-SNE analysis (perplexity 5) of the 507 upregulated genes shows a clear disease effect with the control and AD+isotype samples furthest apart on the X-axis. 4 out of 5 samples treated with the anti-IL13/18 bispecific antibody bbmAb1 clustered close to the control samples, suggesting that cells from these samples have the least AD-like transcriptome of the induced cells. In contrast only two anti-IL13 treated samples showed a similar effect and the anti-IL18 treated samples were indistinguishable from AD+isotype samples indicating no treatment effect. These results were further supported by the t-SNE analysis (perplexity 5) of the 1485 differentially expressed genes (AD+isotype versus control) illustrated in Figure 10. However, Figure 10 does suggest a modest treatment effect for blockade of IL-18 alone. Without wishing to be bound by theory, the present inventors hypothesize that the data of Figures 8-10 suggest that IL-18 blockade (e.g., using an anti-IL18 antibody) can exhibit a treatment effect on AD in a patient, but that IL13/18 co-blockade (e.g., by simultaneous or sequential administration of IL-13 and IL-18 antagonists) can be an unexpectedly superior treatment as compared to blockade of IL-13 or IL-18.

### Example 9. Simultaneous Inhibition of IL-13/IL-18 with a bispecific antibody

In a test assay, a plurality of cells are contacted with a carrier comprising a bispecific antibody disclosed herein. IL-13 and IL-18 activity are assayed and compared to a control assay in which a plurality of cells are contacted with carrier only. Both IL-13 and IL-18 activity are significantly reduced in the test assay as compared to the control.

### Example 10. Treatment of Atopic Dermatitis with an anti-IL-13/IL-18 bispecific antibody

A subject having atopic dermatitis is administered a bispecific antibody described herein. At week 16, the subject achieves a greater reduction in one or more signs and/or symptoms of atopic dermatitis as compared to a placebo treated subject.

## Claims

1. A bispecific IgG antibody, wherein the antibody comprises
a. a first part comprising a first light chain variable domain (VL1) and a first heavy chain variable domain (VH1), that binds specifically to Interleukin-18 (IL-18), wherein the VL1 domain comprises SEQ ID NO: 13, and wherein the VH1 domain comprises SEQ ID NO: 41, and
b. a second part comprising a second light chain variable domain (VL2) and a second heavy chain variable domain (VH2), that binds specifically to Interleukin-13 (IL-13), wherein the VL2 domain comprises SEQ ID NO: 27, and wherein the VH2 domain comprises SEQ ID NO: 55.

2. The bispecific antibody according to claim 1, wherein the antibody comprises a first light chain of lambda type, and a second light chain of kappa type, preferably a first light chain of lambda 1 type, and a second light chain of kappa 4 type.

3. The bispecific antibody according to claim 1 or 2, comprising a first heavy chain comprising a hetero-dimerization modification, and a second heavy chain comprising a hetero-dimerization modification which is complementary to the hetero-dimerization modification of the first heavy chain.

4. The bispecific antibody according to any one of claims 1 to 3, wherein the antibody comprises mutations which enhance the half-life of the bispecific antibody via enhanced FcRn binding, preferably wherein the mutations which enhance the half-life of the bispecific antibody are M252Y/S254T/T256E (YTE), and wherein the amino acid residues are numbered according to the EU numbering.

5. The bispecific antibody according to any one of claims 1 to 4, wherein the antibody comprises a first heavy chain comprising an amino acid sequence as set forth in SEQ ID NO:42, and a first light chain comprising an amino acid sequence as set forth in SEQ ID NO:14, and a second heavy chain comprising an amino acid sequence as set forth in SEQ ID NO:56, and a second light chain comprising an amino acid sequence as set forth in SEQ ID NO:28.

6. The bispecific antibody according to any one of claims 1 to 4, wherein the antibody comprises a first heavy chain comprising an amino acid sequence as set forth in SEQ ID NO:57, and a first light chain comprising an amino acid sequence as set forth in SEQ ID NO:14, and a second heavy chain comprising an amino acid sequence as set forth in SEQ ID NO:58, and a second light chain comprising an amino acid sequence as set forth in SEQ ID NO:28.

7. A pharmaceutical composition comprising the bispecific antibody according to any one of the preceding claims, in combination with one or more pharmaceutically acceptable excipients, diluents or carriers.

8. An isolated nucleic acid molecule encoding the bispecific antibody according to any one of claims 1 to 6.

9. A cloning or expression vector comprising one or more nucleic acid sequences according to claim 8, wherein the vector is suitable for the recombinant production of the bispecific antibody according to any one of claims 1 to 6.

10. A host cell comprising one or more cloning or expression vectors according to claim 9.

11. A process for the production of the bispecific antibody according to any one of claims 1 to 6, comprising culturing a host cell according to claim 10 under conditions sufficient to express the bispecific antibody, and thereafter purifying and recovering the bispecific antibody from the host cell culture.

12. A kit comprising the bispecific antibody according to any one of claims 1 to 6 or the pharmaceutical composition according to claim 7, wherein the kit additionally comprises instructions for use and a pharmaceutical delivery device for administering the bispecific antibody or the pharmaceutical composition to a subject in need thereof, preferably wherein the pharmaceutical delivery device for administering comprises a syringe, an autoinjector, an injection pen, a vial and syringe, an infusion pump, a patch, or an infusion bag and needle.

13. An in vitro method of simultaneously inhibiting the activities of IL-13 and IL-18, comprising contacting a plurality of mammalian cells with an effective amount of a bispecific antibody according to any one of claims 1 to 6.

14. A bispecific antibody according to any one of claims 1 to 6 for use as a medicament.

15. A bispecific antibody according to any one of claims 1 to 6 for use in the prevention or the treatment of atopic dermatitis, preferably moderate to severe atopic dermatitis.

## Patentansprüche

1. Bispezifischer IgG-Antikörper, wobei der Antikörper
a. einen eine erste variable Domäne der leichten Kette (VL1) und eine erste variable Domäne der schweren Kette (VH1) umfassenden ersten Teil umfasst, der spezifisch an Interleukin-18 (IL-18) bindet, wobei die VL1-Domäne SEQ ID NO: 13 umfasst und wobei die VH1-Domäne SEQ ID NO: 41 umfasst, und
b. einen eine zweite variable Domäne der leichten Kette (VL2) und eine zweite variable Domäne der schweren Kette (VH2) umfassenden zweiten Teil umfasst, der spezifisch an Interleukin-13 (IL-13) bindet, wobei die VL2-Domäne SEQ ID NO: 27 umfasst und wobei die VH2-Domäne SEQ ID NO: 55 umfasst.

2. Bispezifischer Antikörper nach Anspruch 1, wobei der Antikörper eine erste leichte Kette vom Lambda-Typ und eine zweite leichte Kette vom Kappa-Typ, vorzugsweise eine erste leichte Kette vom Lambda-1-Typ und eine zweite leichte Kette vom Kappa-4-Typ umfasst.

3. Bispezifischer Antikörper nach Anspruch 1 oder 2, umfassend eine erste schwere Kette, die eine Heterodimerisierungsmodifikation umfasst, und eine zweite schwere Kette, die eine Heterodimerisierungsmodifikation umfasst, die zur Heterodimerisierungsmodifikation der ersten schweren Kette komplementär ist.

4. Bispezifischer Antikörper nach einem der Ansprüche 1 bis 3, wobei der Antikörper Mutationen umfasst, die die Halbwertszeit des bispezifischen Antikörpers über verbesserte FcRn-Bindung verbessern, vorzugsweise wobei es sich bei den Mutationen, die die Halbwertszeit des bispezifischen Antikörpers verbessern, um M252Y/S254T/T256E (YTE) handelt und wobei die Aminosäurereste gemäß der EU-Nummerierung nummeriert sind.

5. Bispezifischer Antikörper nach einem der Ansprüche 1 bis 4, wobei der Antikörper eine erste schwere Kette, die eine Aminosäuresequenz gemäß SEQ ID NO:42 umfasst, und eine erste leichte Kette, die eine Aminosäuresequenz gemäß SEQ ID NO:14 umfasst, und eine zweite schwere Kette, die eine Aminosäuresequenz gemäß SEQ ID NO:56 umfasst, und eine zweite leichte Kette, die eine Aminosäuresequenz gemäß SEQ ID NO:28 umfasst, umfasst.

6. Bispezifischer Antikörper nach einem der Ansprüche 1 bis 4, wobei der Antikörper eine erste schwere Kette, die eine Aminosäuresequenz gemäß SEQ ID NO:57 umfasst, und eine erste leichte Kette, die eine Aminosäuresequenz gemäß SEQ ID NO:14 umfasst, und eine zweite schwere Kette, die eine Aminosäuresequenz gemäß SEQ ID NO:58 umfasst, und eine zweite leichte Kette, die eine Aminosäuresequenz gemäß SEQ ID NO:28 umfasst, umfasst.

7. Pharmazeutische Zusammensetzung, umfassend den bispezifischen Antikörper nach einem der vorhergehenden Ansprüche in Kombination mit einem oder mehreren pharmazeutisch unbedenklichen Exzipienten, Verdünnungsmitteln oder Trägern.

8. Isoliertes Nukleinsäuremolekül, codierend den bispezifischen Antikörper nach einem der Ansprüche 1 bis 6.

9. Klonierungs- oder Expressionsvektor, umfassend eine oder mehrere Nukleinsäuresequenzen nach Anspruch 8, wobei der Vektor für die rekombinante Produktion des bispezifischen Antikörpers nach einem der Ansprüche 1 bis 6 geeignet ist.

10. Wirtszelle, umfassend einen oder mehrere Klonierungs- oder Expressionsvektoren nach Anspruch 9.

11. Verfahren zur Produktion des bispezifischen Antikörpers nach einem der Ansprüche 1 bis 6, umfassend Kultivieren einer Wirtszelle nach Anspruch 10 unter Bedingungen, die zur Expression des bispezifischen Antikörpers hinreichend sind, und danach Aufreinigen und Gewinnen des bispezifischen Antikörpers aus der Wirtszellkultur.

12. Kit, umfassend den bispezifischen Antikörper nach einem der Ansprüche 1 bis 6 oder die pharmazeutische Zusammensetzung nach Anspruch 7, wobei das Kit zusätzlich Gebrauchsanweisungen und eine pharmazeutische Zuführungsvorrichtung zur Verabreichung des bispezifischen Antikörpers oder der pharmazeutischen Zusammensetzung an ein diesbezüglich bedürftiges Individuum umfasst, vorzugsweise wobei die pharmazeutische Zuführungsvorrichtung zur Verabreichung eine Spritze, einen Autoinjektor, einen Injektionsstift, eine Durchstechflasche und Spritze, eine Infusionspumpe, ein Pflaster, oder einen Infusionsbeutel und Nadel umfasst.

13. In-vitro-Verfahren zur gleichzeitigen Hemmung der Aktivitäten von IL-13 und IL-18, umfassend Inkontaktbringen mehrerer Säugerzellen mit einer wirksamen Menge eines bispezifischen Antikörpers nach einem der Ansprüche 1 bis 6.

14. Bispezifischer Antikörper nach einem der Ansprüche 1 bis 6 zur Verwendung als Medikament.

15. Bispezifischer Antikörper nach einem der Ansprüche 1 bis 6 zur Verwendung bei der Vorbeugung oder Behandlung von atopischer Dermatitis, bevorzugt mittelschwerer bis schwerer atopischer Dermatitis.

## Revendications

1. Anticorps IgG bispécifique, l'anticorps comprenant
a. une première partie comprenant un premier domaine variable de chaîne légère (VL1) et un premier domaine variable de chaîne lourde (VH1), qui se lie spécifiquement à l'interleukine-18 (IL-18), où le domaine VL1 comprend SEQ ID NO: 13, et où le domaine VH1 comprend SEQ ID NO: 41, et
b. une deuxième partie comprenant un deuxième domaine variable de chaîne légère (VL2) et un deuxième domaine variable de chaîne lourde (VH2), qui se lie spécifiquement à l'interleukine-13 (IL-13), où le domaine VL2 comprend SEQ ID NO: 27, et où le domaine VH2 comprend SEQ ID NO: 55.

2. Anticorps bispécifique selon la revendication 1, l'anticorps comprenant une première chaîne légère de type lambda, et une deuxième chaîne légère de type kappa, de préférence une première chaîne légère de type lambda 1, et une deuxième chaîne légère de type kappa 4.

3. Anticorps bispécifique selon la revendication 1 ou 2, comprenant une première chaîne lourde comprenant une modification d'hétéro-dimérisation, et une seconde chaîne lourde comprenant une modification d'hétéro-dimérisation qui est complémentaire de la modification d'hétéro-dimérisation de la première chaîne lourde.

4. Anticorps bispécifique selon l'une quelconque des revendications 1 à 3, l'anticorps comprenant des mutations qui augmentent la demi-vie de l'anticorps bispécifique via une liaison FcRn augmentée, de préférence où les mutations qui augmentent la demi-vie de l'anticorps bispécifique sont M252Y/S254T/T256E (YTE), et où les résidus d'acides aminés sont numérotés conformément à la numérotation de l'UE.

5. Anticorps bispécifique selon l'une quelconque des revendications 1 à 4, l'anticorps comprenant une première chaîne lourde comprenant une séquence d'acides aminés telle que décrite dans SEQ ID NO: 42, et une première chaîne légère comprenant une séquence d'acides aminés telle que décrite dans SEQ ID NO: 14, et une deuxième chaîne lourde comprenant une séquence d'acides aminés telle que décrite dans SEQ ID NO: 56, et une deuxième chaîne légère comprenant une séquence d'acides aminés telle que décrite dans SEQ ID NO: 28.

6. Anticorps bispécifique selon l'une quelconque des revendications 1 à 4, l'anticorps comprenant une première chaîne lourde comprenant une séquence d'acides aminés telle que décrite dans SEQ ID NO: 57, et une première chaîne légère comprenant une séquence d'acides aminés telle que décrite dans SEQ ID NO: 14, et une deuxième chaîne lourde comprenant une séquence d'acides aminés telle que décrite dans SEQ ID NO: 58, et une deuxième chaîne légère comprenant une séquence d'acides aminés telle que décrite dans SEQ ID NO: 28.

7. Composition pharmaceutique comprenant l'anticorps bispécifique selon l'une quelconque des revendications précédentes, en combinaison avec un ou plusieurs excipients, diluants ou véhicules pharmaceutiquement acceptables.

8. Molécule d'acide nucléique isolée codant pour l'anticorps bispécifique selon l'une quelconque des revendications 1 à 6.

9. Vecteur de clonage ou d'expression comprenant une ou plusieurs séquences d'acides nucléiques selon la revendication 8, le vecteur étant approprié pour la production recombinante de l'anticorps bispécifique selon l'une quelconque des revendications 1 à 6.

10. Cellule hôte comprenant un ou plusieurs vecteurs de clonage ou d'expression selon la revendication 9.

11. Procédé pour la production de l'anticorps bispécifique selon l'une quelconque des revendications 1 à 6, comprenant la culture d'une cellule hôte selon la revendication 10 dans des conditions suffisantes pour exprimer l'anticorps bispécifique, et ensuite la purification et la récupération de l'anticorps bispécifique à partir de la culture de cellule hôte.

12. Kit comprenant l'anticorps bispécifique selon l'une quelconque des revendications 1 à 6 ou la composition pharmaceutique selon la revendication 7, le kit comprenant en outre des instructions d'utilisation et un dispositif de d'administration pharmaceutique pour administrer l'anticorps bispécifique ou la composition pharmaceutique à un sujet nécessitant celui-ci, de préférence où le dispositif d'administration pharmaceutique pour administrer comprend une seringue, un auto-injecteur, un stylo d'injection, un flacon et une seringue, une pompe de perfusion, un patch, ou une poche de perfusion et une aiguille.

13. Procédé *in vitro* pour inhiber simultanément les activités de IL-13 et IL-18, comprenant la mise en contact d'une pluralité de cellules de mammifère avec une quantité efficace d'un anticorps bispécifique selon l'une quelconque des revendications 1 à 6.

14. Anticorps bispécifique selon l'une quelconque des revendications 1 à 6, pour une utilisation en tant que médicament.

15. Anticorps bispécifique selon l'une quelconque des revendications 1 à 6, pour une utilisation dans la prévention ou le traitement d'une dermatite atopique, de préférence une dermatite atopique modérée à sévère.
